# EUROPEAN PATENT SPECIFICATION

(11) **EP 1 515 684 B1**
(45) Date of publication and mention of the grant of the patent: **19.05.2010**
(21) Application number: 03759973.5
(22) Date of filing: 02.06.2003
(51) Int. Cl.: A61K 8/894, A61K 8/42, A61K 8/891, A61K 8/898, A61Q 1/02, A61Q 1/06

(54) **CARE AND/OR MAKE-UP COSMETIC COMPOSITION STRUCTURED WITH SILICONE POLYMERS AND ORGANOGELLING AGENTS, IN RIGID FORM**
MIT SILIKONPOLYMEREN UND ORGANISCHEN GELIERMITTELN STRUKTURIERTE PFLEGE- UND/ODER- MAKEUP-ZUSAMMENSETZUNG IN STARRER FORM
COMPOSITION COSMETIQUE DE SOIN ET/OU DE MAQUILLAGE SOUS FORME RIGIDE, STRUCTUREE PAR DES POLYMERES DE SILICONE ET DES AGENTS ORGANOGELIFIANTS

(30) Priority: 12.06.2002 FR 0207206; 27.06.2002 US 391617 P
(43) Date of publication of application: 23.03.2005
(73) Proprietor: L'Oréal, 75008 Paris (FR)
(72) Inventor: FERRARI, Véronique, F-94700 Maisons-Alfort (FR); MONDET, Jean, F-93600 Aulnay-sous-Bois (FR)
(74) Representative: Le Coupanec, Pascale A.M.P.
(86) International application number: PCT/EP2003/006463
(87) International publication number: WO 2003/105788

(56) References cited:
- EP-A- 1 116 740
- WO-A-03/013447
- WO-A-03/030854
- US-A- 6 051 216
- US-A- 6 066 314
- US-A1- 2002 048 557
- US-A1- 2002 051 758
- DATABASE WPI Derwent Publications Ltd., London, GB; AN 1992-060684 XP002243441 & JP 04 005224 A (KAO CO.)

## Description

### Technical field

The present invention relates to a care and/or treatment and/or make-up cosmetic composition for the skin, including the scalp, and/or the lips of human beings, containing a liquid fatty phase comprising at least one silicone oil, gelled with a particular polymer, provided in particular in the form of a cast make-up product, in particular as a make-up stick such as lipsticks, whose application leads to a glossy and non-migrating deposit.

A care and/or treatment cosmetic composition is a composition which comprises at least one active compound for treating wrinkles, for moisturizing the skin and the lips, for protecting the skin, the lips and superficial body growths from ultraviolet rays, for treating acne and/or for acting as self-tanning agent.

The invention relates more particularly to cosmetic and dermatological compositions such as make-up products exhibiting properties of staying power, non-transfer and stability.

### Prior state of the art

In cosmetic or dermatological products, it is common to find a structured, namely gelled and/or rigidified, liquid fatty phase; this is in particular the case in solid compositions such as deodorants, balms and lipsticks, eyeshadows, concealer products and foundations which have been cast. This structuring is obtained with the aid of waxes or fillers. Unfortunately, these waxes and fillers tend to mattify the composition, which is not always desirable in particular for a lipstick.or an eyeshadow.

The expression liquid fatty phase is understood to mean, for the purposes of the application, a fatty phase which is liquid at room temperature (25°C) and atmospheric pressure (760 mmHg), which is composed of one or more fatty substances which are liquid at room temperature, which are also called oils, which are compatible with each other and which contain a silicone oil.

The expression structured liquid fatty phase is understood to mean, for the purposes of the application, that this structured phase does not run under its own weight.

The structuring of the liquid fatty phase makes it possible in particular to limit its exudation from solid compositions and, furthermore, to limit, after deposition on the skin or the lips, the migration of this phase into wrinkles and fine lines, which is particularly sought for a lipstick or an eyeshadow. Indeed, substantial migration of the liquid fatty phase, charged with colouring matter, leads to an inaesthetic effect around the lips or the eyes, particularly accentuating the wrinkles and fine lines. This migration is often cited by women as a major defect of conventional lipsticks or eyeshadows. The expression migration is understood to mean an overflowing of the composition deposited on the skin or the lips, outside its initial outline.

The gloss is mainly linked to the nature of the liquid fatty phase. Thus, it is possible to reduce the amount of waxes and fillers in the composition in order to increase the gloss of a lipstick, but then the migration of the liquid fatty phase increases. In other words, the amounts of waxes and fillers necessary for producing a stick of suitable hardness are a barrier to the gloss of the deposit.

The document EP-A-1 068 856 [1] describes solid cosmetic compositions, with no wax, containing a liquid fatty phase structured with a polymer, in which the fatty phase is mainly a non-silicone oil.

The document WO-A-01/97758 [2] describes cosmetic compositions based on polyamide resins comprising a gelling agent chosen from esters and amides of N-acylamino acids and mixtures thereof. The composition also comprises a solvent for the polyamide resin which may be chosen from unsaturated and saturated fatty alcohols, fatty and/or aromatic carboxylic acid esters, ethoxylated and/or propoxylated alcohols and acids, silicones, mineral oils and branched-chain hydrocarbons; preferably, fatty acid esters, fatty alcohols, mineral oils, branched hydrocarbons and mixtures thereof.

The use of fatty phases based on silicone oils makes it possible currently to obtain cosmetic compositions having a long staying power when the oils are' only slightly volatile or are non-volatile, namely a good staying power in particular of the colour over time (unchanging, unfading and transfer-free compositions when the silicone oils are volatile, not forming a deposit on a support such as a glass, a cup, a fabric or a cigarette, placed in contact with the film of make-up.

Currently, the use of silicone oils in cosmetics is limited by the small number of molecules which can gel these media and thus give compositions which exist in solid form such as lipsticks or cast foundations for example. The use of cosmetic compositions whose fatty phase is predominantly siliconized leads, in most cases, to problems of compatibility with the ingredients conventionally used in cosmetics.

In the documents US-A-5 874 069 [3], US-A-5 919 441 [4], US-A-6 051 216 [5], WO-A-99/06473 [40], US-A-6 353 076 [41], WO-A-02/17870 [6], and WO-A-02/17871 [7], cosmetic compositions such as deodorant sticks or gels, comprising a silicone oily phase gelled with a polysiloxane- and polyamide-based wax, or with a polymer containing siloxane groups and groups capable of hydrogen interactions, have been prepared.

In WO-A-02/17870 [6], it is envisaged to add to the composition another gelling agent, but the quantities added should be low, for example less than 0.5% in the case of hydroxystearic acid, in order to preserve the clarity of the product.

In WO-A-02/17871 [7], it is also envisaged to use a second gelling agent with the silicone polymer in a quantity representing 0.5 to 2% by weight of the composition, and a solvent system comprising a non-silicone organic compound, a volatile silicone and optionally another silicone.

The document EP-A-1 177 784 [8] illustrates a deodorant composition comprising a liquid phase containing, for example, a volatile silicone and optionally a non-volatile silicone and/or a non-silicone hydrophobic organic liquid, structured with an organic compound with amido groups, with optionally one or more polymeric or non-polymeric secondary structuring agents in small proportions. Among the secondary structuring agents, this document mentions polymers having siloxane groups and groups exhibiting hydrogen interactions without giving examples or results on a composition using these polymers.

It should be stated that the documents [6], [7] and [8] relate to deodorants for which the problems of exudation and migration of the liquid fatty phase charged with colouring matter into wrinkles and fine lines, and of staying power and non-transfer of the composition, do not exist as in the case of the make-up cosmetic products described above. Moreover, no gloss is sought for deodorants.

In addition, the' sticks obtained by structuring the liquid fatty phase with solely one or more gelling silicone polymers do not exhibit sufficient mechanical resistance to shearing, in particular during the application of the stick to the lips and/or the skin, leading to breaking of the stick.

EP-A-1 116 740 discloses in example 20 a low-transfer color skin composition comprising:
- a polyorganosiloxane/amide polymer: P 17-50
- a volatile silicone oil: decamethylcyclopentasiloxane
- a non-volatile non-silicone oil: liquid paraffin and isopropyl myristate.

### Disclosure of the invention

The subject of the invention is precisely a care and/or make-up and/or treatment composition for the skin and/or the lips, which makes it possible to overcome the disadvantages mentioned above.

Surprisingly, the applicant has found that the use of particular polymers combined with one or more non-polymeric organogelling agents made it possible to structure, in the absence or in the presence of small quantities of wax, the silicone oilbased liquid fatty phases, in the form of a make-up or care product whose application led to a glossy, satiny or matt film according to the wishes of the user and/or the type of non-migrating product desired, and to reinforce the staying power and/or transfer-free properties of these products. In addition, their heat-stability is enhanced.

The expression stable is understood to mean a composition which does not exude at room temperature (25°C) for at least 2 months, or even up to 9 months.

The combination of these polymers with one or more organogelling agents makes it possible to obtain gels, in particular solid gels, having a good mechanical strength and an acceptable rheology in order to allow a deposit in a sufficient quantity which does not feel sticky, which has a very good staying power, which is transfer-free (in particular when volatile silicone oils are used) and which does not migrate into wrinkles and fine lines.

The effects obtained by virtue of this combination do not appear in the documents relating to deodorants which possibly envisage this combination since the problems solved by this combination do not exist in the field of deodorants.

The invention not only applies to make-up products for the lips such as lipsticks, lip pencils and lip glosses, but also to care and/or treatment products for the skin, including the scalp, and the lips, such as sun protection products in stick form for the skin, the face or the lips, or lip balms, to make-up products for the skin, both of the face and of the human body, such as foundations cast as a stick or in a dish, concealer products and temporary tattoo products, to cleansing products, in particular in stick form, and to make-up products for the eyes' such as eyeliners, in particular in pencil form, and mascaras, in particular cakes for keratinous fibres (eyelashes, eyebrows, hair).

More precisely, the subject of the invention is a care and/or make-up cosmetic composition comprising a liquid fatty phase comprising at least one silicone oil, structured with at least one gelling system comprising
a) at least one polymer (homopolymer or copolymer) having a weight-average molecular mass ranging from 500 to 500 000, containing at least one moiety comprising:
   - at least one polyorganosiloxane group consisting of 1 to 1 000 organosiloxane units in the chain of the moiety or in the form of a graft, and
   - at least two groups capable of establishing hydrogen interactions, chosen from amide.
b) at least one non-polymeric organogelling agent, the liquid fatty phase and the gelling system forming a physiologically acceptable medium and the liquid fatty phase comprising at least one volatile silicone oil and at least one volatile non-silicone oil, and a non-volatile non-silicone oil.

According to the invention, the expression "gelling system" is understood to mean a system which makes it possible to rigidify the composition by forming hydrogen bonds.

The composition of the invention may be provided in the form of a paste, a solid or a more or less viscous cream. It may be a simple or multiple, in particular an oil-in-water or a water-in-oil, emulsion a rigid or soft gel having an oily continuous phase. The simple or multiple emulsion may comprise an aqueous or oily continuous phase optionally containing dispersed lipid vesicles. In particular, it is provided in a form cast as a stick or in a dish and more especially in the form of an oily, in particular anhydrous, rigid gel and in particular of an anhydrous stick. More especially, it is provided in the form of a translucent or opaque rigid gel (according to whether it contains pigments or otherwise), the liquid fatty phase forming the continuous phase. An anhydrous composition will comprise less than 10% by weight of water, for example less than 5% by weight.

The structuring of the liquid fatty phase can be modulated according to the nature of the polymer and the non-polymeric organogelling agent used in the gelling system, and may be such that a rigid structure is obtained in the form of a baton or a stick, having good mechanical strength. When they are coloured, these batons make it possible, after application, to obtain a glossy deposit, which does not migrate and which has good staying power, in particular of the colour over time. The composition may comprise one or more structuring polymers and one or more non-polymeric organogelling agents.

Avantageously, the composition of the invention is a composition for the lips and even better a lipstick composition in particular in stick form.

### Liquid fatty phase

According to the invention, the liquid fatty phase comprises at least one silicone oil which is a volatile oil, a non-volatile non-silicone oil and a volatile non-silicone oil. An oil is a non-aqueous compound which is immiscible with water.

According to the invention, the volatile silicone oil may be chosen from linear or cyclic silicone oils having a flash point equal to or greater than 40°C and advantageously greater than the softening point of the gelling system and/or a viscosity of less than 8 cSt, such as linear or cyclic polydimethylsiloxanes (PDMS) having from 3 to 7 silicon atoms.

By way of examples of such volatile oils, there may be mentioned the compounds given in Table 1 below.

The non-volatile silicone oils may be polydimethylsiloxanes, polyalkylmethylsiloxanes, dimethicone copolyols, alkylmethicone copolyols, cetyldimethicone, silicones with alkylglyceryl ether groups, silicones with side amine groups and dilauroyltrimethylol propane siloxysilicate. The alkyl groups of these oils have in particular from 2 to 24 carbon atoms.

The non-volatile silicone oils which can be used in the invention may be in particular linear, non-volatile polydimethylsiloxanes (PDMS) which are liquid at room temperature; polydimethylsiloxanes containing alkyl, alkoxy or phenyl groups, which are pendent and/or at the silicone chain end, groups each having from 2 to 24 carbon atoms; phenylated silicones such as phenyltrimethicones, phenyldimethicones, phenyltrimethylsiloxy diphenylsiloxanes, diphenyl-dimethicones, diphenylmethyldiphenyltrisiloxanes, 2-phenylethyl trimethylsiloxysilicates, fluorinated silicones with one or more group(s) that is (are) pendent or at the chain end having from 1 to 12 carbon atoms of which all or some of the hydrogen atoms are substituted with fluorine atoms, dimethiconols and mixtures thereof.

According to the invention, the liquid fatty phase comprises at least one volatile silicone oil and at least one volatile non-silicone oil.

For the purposes of the invention, a volatile silicone or non-silicone oil has a flash point preferably of 40 to 135°C or no flash point. Volatile oils have at room temperature (25°C) and atmospheric pressure (760 mmHg) a vapour pressure ranging from 0.02 mm to 300 mmHg (2.66 Pa to 40 000 Pa) and even better ranging from 0.1 to 90 mmHg (13 Pa to 12 000 Pat. The non-volatile oils' then correspond to a vapour pressure of less than 0.02 mmHg (2.66 Pa).

The silicone oils of the invention have a' viscosity which is advantageously chosen from the ranging going from 5 to 800 000 cSt at 25°C, preferably from 10 to 500 000 cSt, and even better from 10 to 5 000 cSt.

**Table 1**

| **Compound** | **Flash point** | **Viscosity** |
|---|---|---|
| | **(°C)** | **(cSt)** |
| Octyltrimethicone | 93 | 1.2 |
| Hexyltrimethicone | 79 | 1.2 |
| Decamethylcyclopentasiloxane (cyclopentasiloxane or D5) | 72 | 4.2 |
| Octamethylcyclotetrasiloxane (cyclotetradimethylsiloxane or D4) | 55 | 2.5 |
| Dodecamethylcyclohexasiloxane (D6) | 93 | 7 |
| Decamethyltetrasiloxane (L4) - | 63 | 1.7 |
| KF 96 A from Shin Etsu | 94 | 6 |
| PDMS (polydimethylsiloxane) DC 200 (1.5 cSt) from Dow Corning | 56 | 1.5 |
| PDMS DC 200 (2 cSt) from Dow Corning | 87 | 2 |
| PDMS DC 200 (5 cSt) from Dow Corning | 134 | 5 cSt |
| PDMS DC 200 (3 cSt) from Dow Corning | 102 | 3 cSt |

The volatile silicone oil may also be chosen from the group comprising fluorinated silicone oils such as silicones with alkyl and perfluoroalkyl groups, silicones with oxyethylenated/oxypropylenated (EO/PP) side groups and with perfluorinated groups, silicones with perfluorinated side groups and with glycerolated side groups, perfluoroalkylmethylphenyl-siloxanes, these oils having a vapour pressure greater than or equal to 0.02 mmHg.

According to the invention, the liquid fatty phase contains one or more volatile and non-volatile non-silicone oils. The volatile non-silicone oils may be chosen from the group comprising hydrocarbon oils and volatile esters and ethers such as volatile hydrocarbons such as isododecane and isohexadecane, C₈-C₁₆ isoparaffins, isohexyl or isodecyl neopentanoates.

The volatile non-silicone oil may also be chosen from fluorinated oils such as perfluoropolyethers, perfluoroalkanes such as perfluorodecalin, perfluorodamantanes, monoesters, diesters and triesters of perfluoroalkyl phosphates and fluorinated ester oils.

By way of example of volatile non-silicone oils which can be used in the invention, there may be mentioned the compounds of Table 2 which follows.

**Table 2**

| **Compound** | **Flash point** |
|---|---|
| | **(°C)** |
| Isododecane | 43 |
| Isohexadecane | 102 |
| Isodecyl neopentanoate | 118 |
| Propylene glycol n-butyl ether | 60 |
| Ethyl 3-ethoxypropionate | 58 |
| Propylene glycol methyl ether | 46 |
| acetate* | |
| Isopar L (C₁₁-C₁₃ isoparaffin) | 62 |
| Isopar H (C₁₁-C₁₂ isoparaffin) | 56 |

The liquid fatty phase advantageously contains at least 30%, and even better at least 40% by weight of silicone oil(s) advantageously having a viscosity of less than 1 000 cSt and even better of less than 100 cSt because the silicone polymers used in the invention are more soluble in silicone oils of low viscosity. It may also contain other non-silicone oils or a mixture of non-silicone oils.

When the fatty phase comprises a volatile oil, it advantageously represents from 3 to 89.4%, and even better from 5 to 60%, for example from 5 to 10% of the total weight of the composition.

The liquid fatty phase may also contain other non-silicone oils, for example polar oils such as:
- hydrocarbonaceous vegetable oils with a high content of triglycerides consisting of esters of fatty acids and of glycerol in which the fatty acids may have varied chain lengths, it being possible for the latter to be linear or branched, saturated or unsaturated; these oils are in particular wheat germ, maize, sunflower, karite, castor, sweet almond, macadamia, apricot, soybean, rapeseed, cottonseed, lucerne, poppy seed, pumpkin seed, sesame, gourd, avocado, hazelnut, grapeseed or blackcurrant seed, evening primrose, millet, barley, quinoa, olive, rye, safflower, candlenut, passion flower and rose musk oils; or triglycerides of caprylic/capric acids such as those sold by the company Stearines Dubois or those sold under the names Miglyol 810, 812 and 818 by the company Dynamit Nobel;

- synthetic oils or esters of formula R₅COOR₆ in which R₅ represents the residue of a linear or branched higher fatty acid containing from 1 to 40 and even better from 7 to 19 carbon atoms and R₆ represents a branched hydrocarbon chain containing from 1 to 40 and even better from 3 to 20 carbon atoms, with R₅ + R₆ ≥ 10 such as, for example, Purcellin oil (ketostearyl octanoate), isononyl isononanoate, C₁₂ to C₁₅ alcohol benzoate, isopropyl myristate, 2-ethylhexyl palpitate, octanoates, decanoates or ricinoleates of alcohols or of polyalcohols; hydroxylated esters such as isostearyl lactate, diisostearyl malate; and esters of pentaerythritol;
- synthetic esters having from 10 to 40 carbon atoms;
- C₈ to C₂₆ fatty alcohols such as oleyl alcohol and octyldodecanol;
- fatty acids such as oleic, linoleic or linolenic acid; and
- mixtures thereof.

The liquid fatty phase may also contain apolar oils such as linear or branched hydrocarbons or fluorocarbons of synthetic or mineral origin, which are volatile or not, such as volatile oils of paraffin (such as isoparaffins, isododecane) or non-volatile oils of paraffin and its derivatives, petroleum jelly, polydecenes, hydrogenated polyisobutene such as parleam, squalane, and mixtures thereof.

Generally, the liquid fatty phase represents from 5 to 99% of the total weight of the composition and even better from 20 to 75%.

### Solid particles

According to the invention, the composition generally comprises, in addition, solid particles chosen from fillers and pigments. Generally, the mean size of the solid particles is from 10 nm to 50 µm, and even better from 50 nm to 30 µm, for example from 100 nm to 10 µm.

The fillers used in the cosmetic compositions are generally intended to absorb sweat and sebum and/or to provide mattness. According to the invention, they further make it possible to structure the liquid fatty phase containing a silicone oil and to reinforce the staying power and/or transfer-free properties of the composition and also the heat stability.

The expression pigments is understood to mean any solid particle insoluble in the composition which serves to give and/or modify a colour and/or an iridescent appearance.

These pigments may simultaneously provide the function of absorption of sweat and of sebum, and the function of coloration or of modification of appearance of the composition, that is of the make-up and/or care cosmetic product. In the invention, they also bring about the structuring of the liquid fatty phase.

These fillers or pigments may be either of a hydrophobic nature, or of a hydrophilic nature. When these fillers or pigments are hydrophilic particles, their dispersion in the composition is facilitated either by coating them with a film of hydrophobic compound or by adding a dispersant and in particular an amphiphilic silicone to the composition.

The hydrophobic pigments or fillers may consist of hydrophobic polymer or copolymer powders. By way of example of hydrophobic polymers and copolymers used as fillers, there may be mentioned:
1) fluorinated polymers such as polytetrafluoroethylene powders and tetrafluoroethylene and olefin, for example ethylene or propylene, copolymer powders; 2) silicone elastomers, for example polymethylsilsesquioxane powders (Tospearl® from Toshiba); 3) polyolefins such as polyethylene; 4) polyalkyl methacrylates, for example polymethyl methacrylate; 5) polyamides (Nylon®); 6) polystyrenes; 7) polyesters and derivatives thereof; 8) polyacrylics (Polytrap® from Dow Corning) or polymethyl methacrylate; and 9) polyurethanes, for example hexamethylene diisocyanate/trimethylol hexalactone powders.

It is also possible to use hydrophilic fillers which are surface-treated so as to be hydrophobic, such as boron nitride, starch, precipitated calcium carbonate, silica, glass, or a ceramic.

Instead of powders, it is of course possible to use fibres of a hydrophobic nature, in particular fibres of the polymers and copolymers mentioned above.

The solid particles may also consist of pigments and/or pearlescent agents which make it possible to obtain a make-up with high coverage, that is to say which does not reveal the skin, the lips or the superficial body growths. These particles make it possible, in addition, to reduce the sticky feel of the compositions.

The pigments may be white or coloured, inorganic and/or organic, coated or not. There may be mentioned, among the inorganic pigments, titanium or zinc dioxide, optionally surface-treated, zirconium or cerium oxides, and iron or chromium oxides, manganese violet, ultramarine blue, chromium hydrate and ferric blue. Among the organic pigments, there may be mentioned carbon black, pigments of the D & C type, and lacquers based on carmine, barium, strontium, calcium or aluminium. The pigments may represent from 0.1 to 50%, preferably from 0.5 to 40%, and even better from 2 to 30% of the total weight of the composition.

The pearlescent pigments (or pearlescent agents) may be chosen from white pearlescent pigments such as mica coated with titanium, or with bismuth oxychloride, coloured pearlescent pigments such as mica-titanium with iron oxides, mica-titanium with in particular ferric blue and chromium' oxide, mica-titanium with an organic pigment of the abovementioned type and pearlescent pigments based on bismuth oxychloride. The pearlescent pigments may also have goniochromatic properties and may be provided in the form of liquid crystals or multilayer platelets. They may represent from 0 to 30% of the total weight of the composition, and even better from 0.1 to 20%.

When the pigments or fillers are hydrophilic, they are coated with a film of hydrophobic compound so as to introduce them into the liquid fatty phase of the composition of the invention.

The coating may be a fluorinated coating such as a perfluoroalkyl mono- or diester of phosphoric acid, (acid or salt), a perfluoropolyether, a perfluorocarboxylic or -sulfonic acid, or a perfluoroalkyl phosphate salt of diethanolamine.

The coating may be a coating based on a fluorinated silicone, for example a coating-grafting with a silane having a perfluoroalkyl group.

The coating may also be carried out by means of silicone derivatives, for example a coating-grafting with reactive silicones initially possessing hydrogenosilane groups, a coating-grafting with a diorganosilane such as dimethylchlorosilane or with an alkylalkoxysilane, a coating-grafting with a silane having a glycydoxypropyl group, a coating with a polyglycerolated silicone, or a coating with a silicone-g-polyacrylic or silicone grafted acrylic copolymer.

It is also possible to use a coating with N-acylamino acids, for example N-lauroyllysine, coatings with fatty acids or fatty acid salts of the stearic acid type, coatings with lecithins and coatings with ester oils.

It is also possible to facilitate the dispersion of the hydrophilic particles by means of at least one amphiphilic silicone which plays the role of a surfactant between the hydrophilic particles and the hydrophobic silicone phase.

These amphiphilic silicones contain a silicone part which is compatible with the highly siliconized medium of the compositions of the invention, and a hydrophilic part which may.be, for example, the residue of a compound chosen from alcohols and polyols, having from 1 to 12 hydroxyl groups, polyoxyalkylenes containing at least two oxyalkylenated moieties and having from 0 to 20 oxypropylenated moieties and/or from 0 to 20 oxyethylenated moieties. This hydrophilic part therefore has affinity for the hydrophilic particles and promotes their dispersion in the silicone medium.

The amphiphilic silicone may be an oil with no gelling activity. Such oils may consist of:
- dimethicone copolyols, optionally containing phenyl groups,
- alkylmethicone copolyols,
- polyglycerolated silicones, that is to say silicones with alkylglyceryl ether groups,
- silicones with perfluorinated side groups and with glycerolated side groups,
- silicones with polyoxyethylenated/polyoxypropylenated side groups and with perfluorinated side groups,
- copolymers with a silicone block and with a hydrophilic block other than polyether, for example polyoxazoline or polyethyleneimine,
- graft copolymers of the silicone-grafted polysaccharide type,
- copolymers with a silicone block and with a polyoxyethylene/polyoxypropylene block.

The amphiphilic silicone used in the invention may also be an amphiphilic silicone resin which is at least partially crosslinked.

By way of example of such resins, there may be mentioned:
- crosslinked silicone resins with alkyl polyether groups, such as polyoxyethylene (POE) and polyoxyethylene/polyoxypropylene (POE/POP), described in US-A-5 412 004 [9], and
- silicone resins partially crosslinked with α,ω-dienes, possessing both hydrophilic POE/POP side chains and hydrophobic alkyl side chains such as those described in EP-A-1 048 686 [10]. The hydrophilic side chains are obtained by reaction with a POE/POP having only one vinyl end, and the alkyl side chains are formed by reaction with an α-olefin having a fatty chain.

In the amphiphilic silicone resin, the silicone part advantageously consists of polydimethylsiloxane.

### Gelling silicone polymer

The structuring or gelling polymer(s) of the composition are solid at room temperature (25°C) and atmospheric pressure (760 mmHg) and are soluble in the liquid fatty phase at a temperature of 25 to 250°C.

The expression polymer is understood to mean, for the purpose of the invention, a compound having at least 2 repeating moieties, preferably at least 3 repeating moieties and even better 10 repeating moieties.

In the composition of the invention, the silicone polymer of the gelling system generally represents from 0.5 to 80%, preferably from 2 to 60% and even better from 5 to 40% of the total weight of the composition.

Moreover, the polymer of the gelling system/silicone oil(s) mass ratio is preferably from 0.1 to 50%.

The polymers used as gelling agents in the composition of the invention are polymers of the polyorganosiloxane type such as those described in the documents US-A-5 874 069 [3], US-A-5,9199, 441 [4], US-A-6,051,216 [5] and US-A-5,981,680 [11].

According to the invention, the polymers used as gelling agent may belong to the following two families:
1) polyorganosiloxanes comprising at least two groups capable of establishing hydrogen interactions, these two groups being situated in the polymer chain; and/or
2) polyorganosiloxanes comprising at least two groups capable of establishing hydrogen interactions, these two groups being situated on the grafts or branches.

The polymers to which the invention applies are solids which may be solubilized beforehand in as solvent with hydrogen interactions, capable of breaking the hydrogen interactions of the polymers, such as C₂ to C₈ lower alcohols and in particular ethanol, n-propanol or isopropanol, before being brought into contact with the silicone oils according to the invention. It is also possible to use these hydrogen interaction "breaking" solvents as cosolvent. These solvents may then be kept in the composition or may be removed by selective evaporation which is well known to persons skilled in the art.

The polymers comprising two groups capable of establishing hydrogen interactions in the polymer chain may be polymers comprising at least one moiety corresponding to the formula: 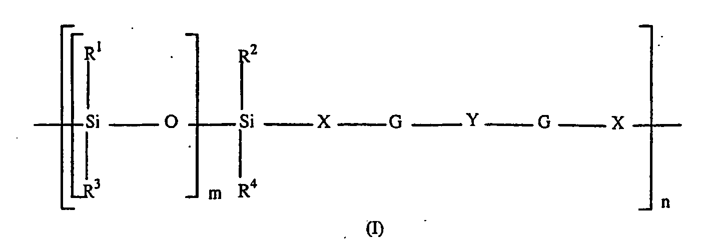 in which:
1) R¹, R², R³ and R⁴, which may be identical or different, represent a group chosen from:
   - linear, branched or cyclic, saturated or unsaturated, C₁ to C₄₀ hydrocarbon-based groups, possibly containing in their chain one or more oxygen sulphur and/or nitrogen atoms, and possibly being partially or totally substituted with fluorine atoms,
   - C₆ to C₁₀ aryl groups, optionally substituted with one or more C₁ to C₄ alkyl groups,
   - polyorganosiloxane chains possibly containing one or more oxygen, sulphur and/or nitrogen atoms;
2) the groups X, which may be identical or different, represent a linear or branched C₁ to C₃₀ alkylenediyl group, possibly containing in its chain one or more oxygen and/or nitrogen atoms;
3) Y is a saturated or unsaturated, C₁ to C₅₀ linear or branched divalent alkylene, arylene, cycloalkylene, alkylarylene or arylalkylene group, possibly comprising one or more oxygen, sulphur and/or nitrogen atoms, and/or bearing as substituent one of the following atoms or groups of atoms: fluorine, hydroxyl, C₃ to C₈ cycloalkyl, C₁ to C₄₀ alkyl, C₅ to C₁₀ aryl, phenyl optionally substituted with 1 to 3 C₁ to C₃ alkyl groups, C₁ to C₃ hydroxyalkyl and C₁ to C₆ aminoalkyl, or
4) Y represents a group corresponding to the formula: 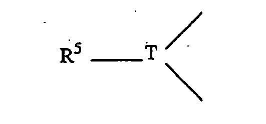 in which'
   - T represents a linear or branched, saturated or unsaturated, C₃ to C₂₄ trivalent or tetravalent hydrocarbon-based group optionally substituted with a polyorganosiloxane chain, and possibly containing one or more atoms chosen from O, N and S, or T represents a trivalent atom chosen from N, P and Al, and
   - R⁵ represents a linear or branched C₁ to C₅₀ alkyl group or a polyorganosiloxane chain, possibly comprising one or more ester, amide, urethane, thiocarbamate, urea, thiourea and/or sulphonamide groups, which may possibly be linked to another chain of the polymer;
5) the groups G, which may be identical or different, represent divalent groups chosen from:
   in which R⁶ represents a hydrogen atom or a linear or branched C₁ to C₂₀ alkyl group, on condition that at least 50% of the groups R⁶ of the polymer represent a hydrogen atom and that at least two of the groups G of the polymer are a group other than: 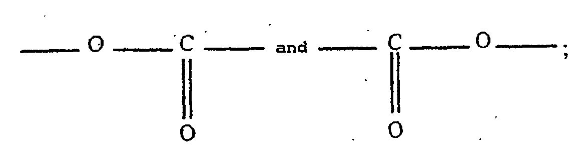
6) n is an integer ranging from 2 to 500 and preferably from 2 to 200, and m is an integer ranging from 1 to 1 000, preferably from 1 to 700 and better still from 6 to 200.

According to the invention, 80% of the groups R¹, R², R³ and R⁴ of the polymer are preferably chosen from methyl, ethyl, phenyl and 3,3,3-trifluoropropyl groups.

According to the invention, Y can represent various divalent groups, furthermore optionally comprising one or two free valencies to establish bonds with other moieties of the polymer or copolymer. Preferably, Y represents a group chosen from:
a) linear C₁ to C₂₀ and preferably C₁ to C₁₀ alkylene groups,
b) C₃₀ to C₅₆ branched alkylene groups possibly comprising rings and unconjugated unsaturations,
c) C₅-C₆ cycloalkylene groups,
d) phenylene groups optionally substituted with one or more C₁ to C₄₀ alkyl groups,
e) C₁ to C₂₀ alkylene groups' comprising from 1 to 5 amide groups,
f) C₁ to C₂₀ alkylene groups comprising one or more substituents chosen from hydroxyl, C₃ to C₈ cycloalkane, C₁ to C₃ hydroxyalkyl and C₁ to C₆ alkylamine groups,
g) polyorganosiloxane chains of formula: 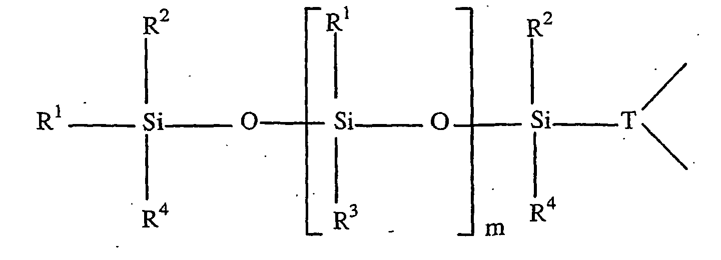 in which R¹ R² , R³ , R⁴, T and m are as defined above, and
h) polyorganosiloxane chains of formula: 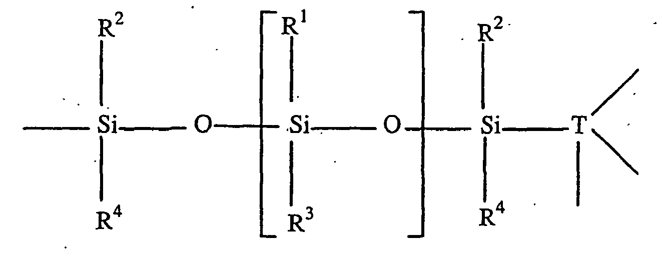 The polyorganosiloxanes of the second family may be polymers comprising at least one moiety corresponding to formula (II): 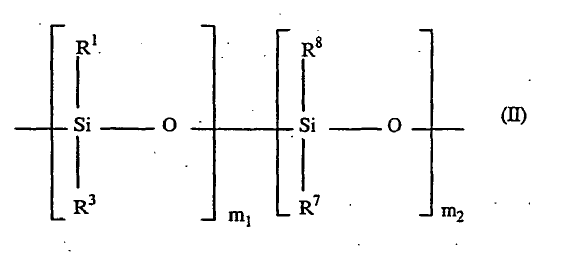 in which
   - R¹ and R³, which may be identical or different are as defined above for formula (I),
   - R⁷ represents a group as defined above for R¹ and R³, or represents the group of formula -X-G-R⁹ in which X and G are as defined above for formula (I) and R⁹ represents a hydrogen atom or a linear, branched or cyclic, saturated or unsaturated, C₁ to C₅₀ hydrocarbon-based group optionally comprising in its chain one or more atoms chosen from O, S and N, optionally substituted with one or more fluorine atoms and/or one or more hydroxyl groups, or a phenyl group optionally substituted with one or more C₁ to C₄ alkyl groups,
   - R⁸ represents the group of formula -X-G-R⁹ in which X, G and R⁹ are as defined above,
   - m₁ is an integer ranging from 1 to 998, and
   - m₂ is an integer ranging from 2 to 500.

According to the invention, the polymer used as gelling agent may be a homopolymer, that is to say a polymer comprising several identical moieties, in particular moieties of formula (I) or of formula (II).

According to the invention, it is also possible to use a polymer consisting of a copolymer comprising several different moieties of formula (I), that is to say a polymer, in which at least one of the groups R¹, R², R³, R⁴, X, G, Y, m and n is different in one of the moieties. The copolymer may also be formed from several moieties of formula (II), in which at least one of the groups R¹, R³, R⁷, R⁸, m₁ and m₂ is different in at least one of the moieties.

It is also possible to use a copolymer comprising at least one moiety of formula (I) and at least one moiety of formula (II), the moieties of formula (I) and the moieties of formula (II) possibly being identical to or different from each other.

According to one variant of the invention, it is also possible to use a copolymer furthermore comprising at least one hydrocarbon-based moiety comprising two groups capable of establishing hydrogen interactions, chosen from amide. These copolymers may be block copolymers or graft copolymers.

The groups capable of establishing hydrogen interactions are amide groups of formulae -C(O)NH- and -HN-C(O)-.

In this case, the gelling agent may be a polymer comprising at least one moiety of formula (III) or (IV): 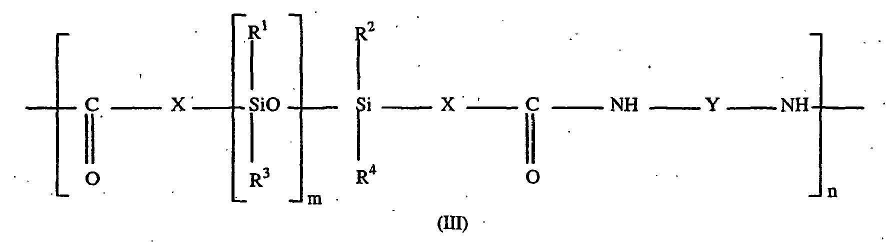 or 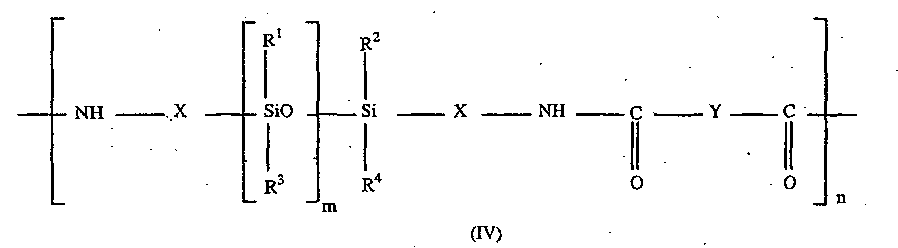 in which R¹, R², R³, R⁴, X, Y, m and n are as defined above.

Such a moiety may be obtained:
- either by a condensation reaction between a silicone containing α,ω-carboxylic acid ends and one or more diamines, according to the following reaction scheme: 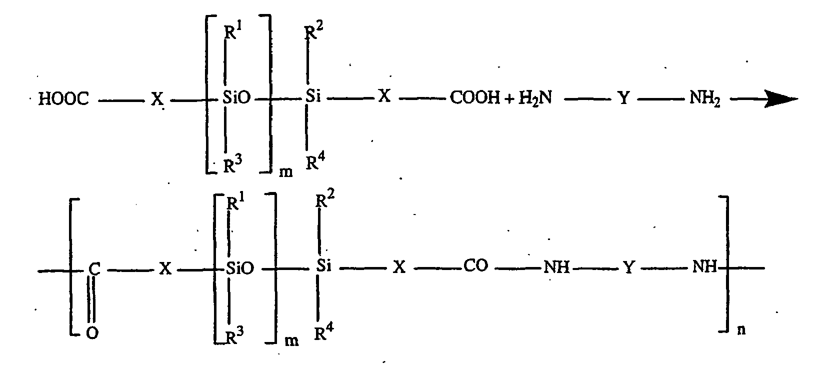
- or by reaction of two molecules of α-unsaturated carboxylic acid with a diamine according to the following reaction scheme:

   CH₂=CH-X¹-COOH+H₂N-Y-NH₂ → CH₂=CH-X¹-CO-MH-Y-NH-CO-X¹-CH=CH₂

   followed by the addition of a siloxane to the ethylenic unsaturations, according to the following scheme: 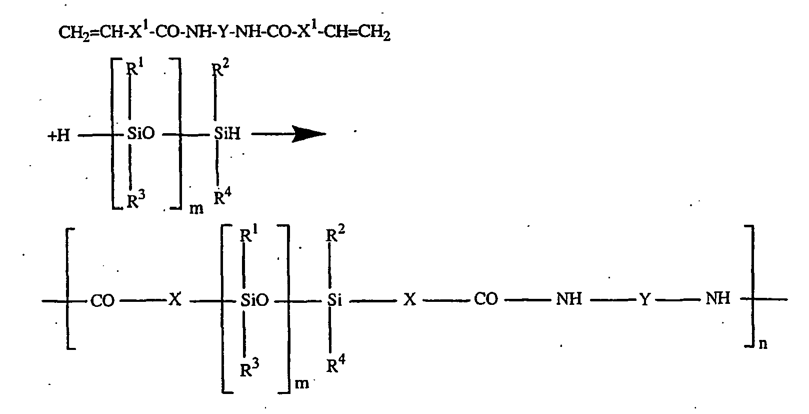 in which X¹- (CH₂) ₂- corresponds to X defined above and Y, R¹, R², R³, R⁴ and m are as defined above;
- or by reaction of a silicone containing α,ω-NH₂ ends and a diacid of formula HOOC-Y-COOH according to the following reaction scheme:

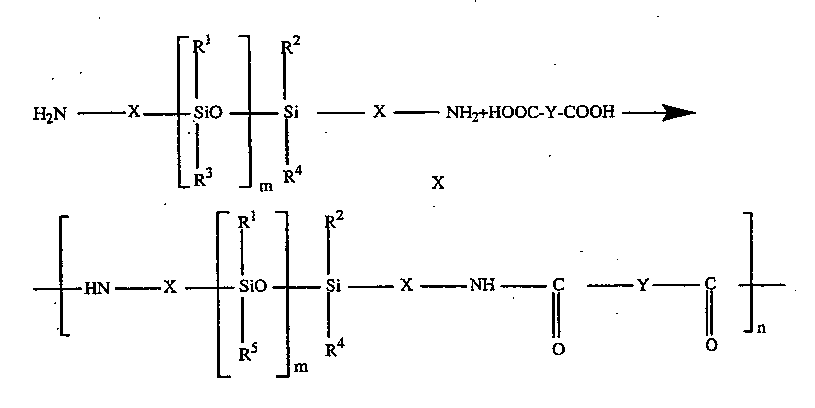

In these polyamides of formula (III) or (IV), m is preferably in the range from 1 to 700, more preferably from 15 to 500 and better still from 15 to 45, and n is in particular in the range from 1 to 500, preferably from 1 to 100 and better still from 4 to 25,
X is preferably a linear or branched alkylene chain containing from 1 to 30 carbon atoms and in particular 3 to 10 carbon atoms, and
- Y is preferably an alkylene chain that is linear or branched or that possibly comprises rings and/or unsaturations, containing from 1 to 40 carbon atoms, in particular from 1 to 20 carbon atoms and better still from 2 to 6 carbon atoms, in particular 6 carbon atoms.

In formulae (III) and (IV), the alkylene group representing X or Y can optionally contain in its alkylene portion at least one of the following elements:
1) 1 to 5 amide, urea or carbamate groups,
2) a C₅ or C₆ cycloalkyl group, and
3) a phenylene group optionally substituted with 1 to 3 identical or different C₁ to C₃ alkyl groups.

In formulae (III) and (IV), the alkylene groups may also be substituted with at least one element chosen from the group consisting of:
- a hydroxyl group
- a C₃ to C₈ cycloalkyl group,
- one to three C₁ to C₄₀ alkyl groups,
- a phenyl group optionally substituted with one to three C₁ to C₃ alkyl groups,
- a C₁ to C₃ hydroxyalkyl group, and
- a C₁ to C₆ aminoalkyl group.

In these formulae (III) and (IV), Y may also represent: 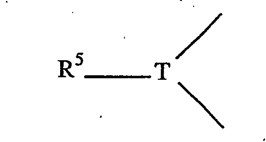 in which R⁵ represents a polyorganosiloxane chain and T represents a group of formula: 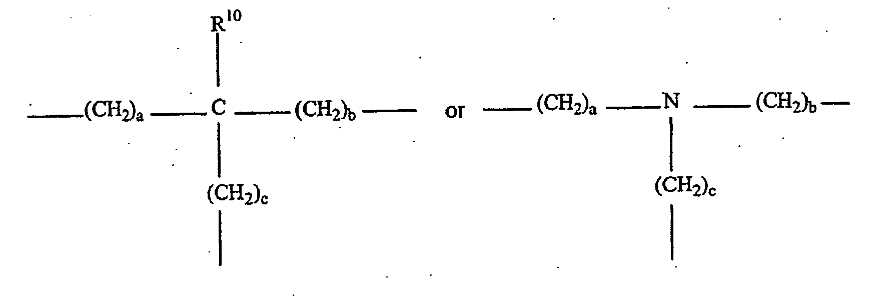 in which a, b and c are, independently, integers ranging from 1 to 10, and R¹⁰ is a hydrogen atom or a group such as those defined for R¹ R², R³ and R⁴.

In formulae (III) and (IV) , R¹, R², R³ and R⁴ preferably represent, independently, a linear or branched C₁ to C₄₀ alkyl group, preferably a CH₃, C₂H₅, n-C₃H₇ or isopropyl group, a polyorganosiloxane chain or a phenyl group optionally substituted with one to three methyl or ethyl groups.

As has been seen previously, the polymer may comprise identical or different moieties of formula (III) or (IV).

Thus, the polymer may be a polyamide containing several moieties of formula (III) or (IV) of different lengths, i.e. a polyamide corresponding to the formula : 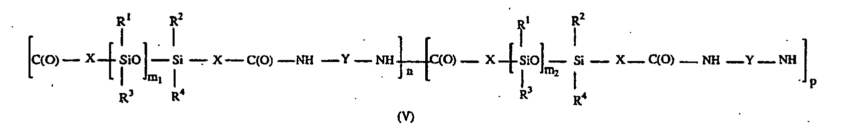 in which X, Y, n and R¹ to R⁴ have the meanings given above, m₁ and m₂, which are different, are chosen in the range from 1 to 1 000, and p is an integer ranging from 2 to 300.

In this formula, the moieties may be structured to form either a block copolymer, or a random copolymer or an alternating copolymer. In this copolymer, the moieties may be not only of different lengths, but also of different chemical structures, for example containing different groups Y. In this case, the copolymer may correspond to the formula: 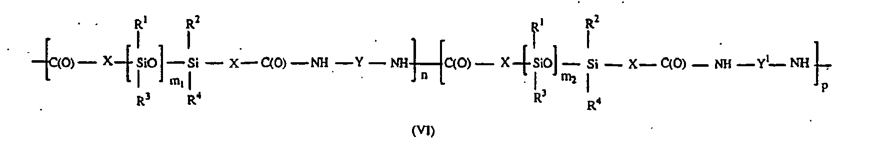 in which R¹ to R⁴, X, Y, m₁, m₂, n and p have the meanings given above and Y¹ is different from Y but chosen from the groups defined for Y. As previously, the various moieties may be structured to form either a block copolymer, or a random copolymer or an alternating copolymer.

In this first embodiment of the invention, the gelling agent may also consist of a graft copolymer. Thus, the polyamide containing silicone units may be grafted and optionally crosslinked with silicone chains containing amide groups. Such polymers' may be synthesized with trifunctional amines.

In this case, the copolymer may comprise at least one moiety of formula: 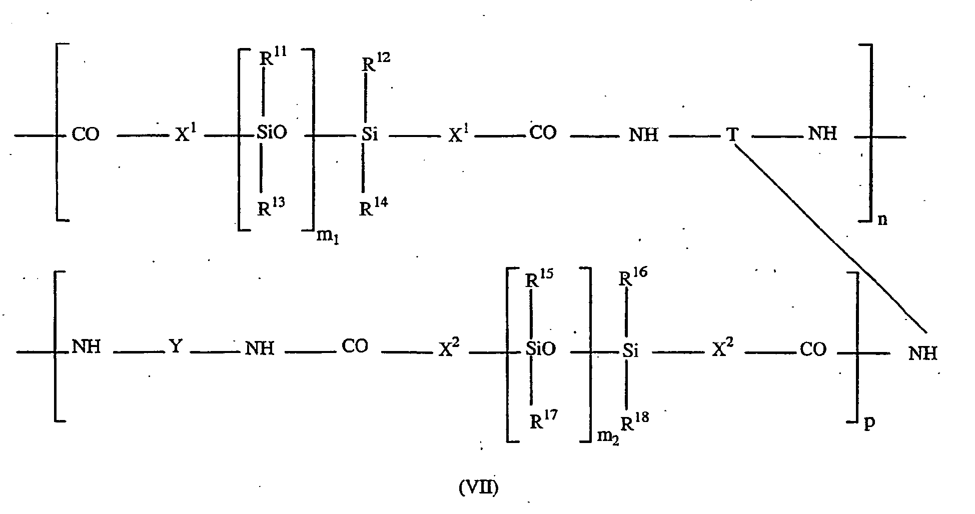 in which X¹ and X², which are identical or different, have the meaning given for X in formula (I), n is as defined in formula (I), Y and T are as defined in formula (I), R¹¹ to R¹⁸ are groups chosen from the same group as R¹ to R⁴, m₁ and m₂ are numbers in the range from 1 to 1 000, and p is an integer ranging from 2 to 500.

In formula (VII), it is preferred that:
- p is in the range from 1 to 25 and better still from 1 to 7,
- R¹¹ to R¹⁸ are methyl groups,
- T corresponds to one of the following formulae: 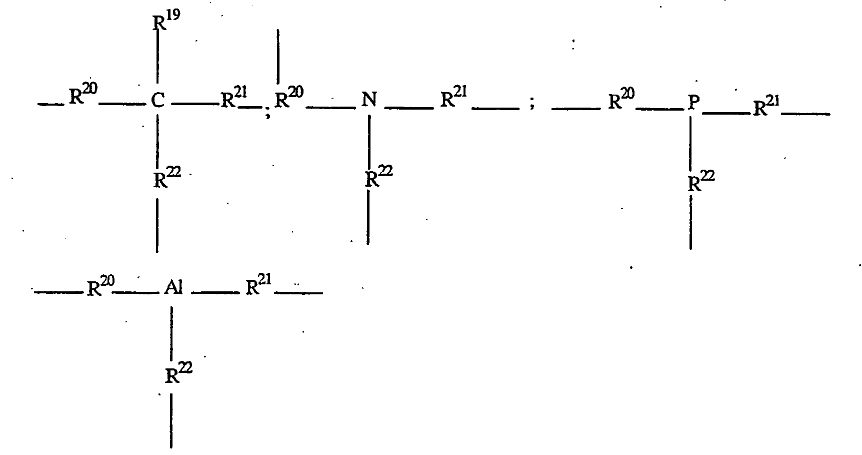 in which R¹⁹ is a hydrogen atom or a group chosen from the groups defined for R¹ to R⁴, and R²⁰, R²¹ and R²² are, independently, linear or branched alkylene groups, and more preferably corresponds to the formula: 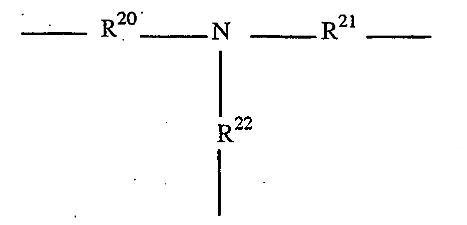 in particular with R²⁰, R²¹ and R²² representing -CH₂-CH₂-,
- m₁ and m₂ are in the range from 15 to 500 and better still from 15 to 45,
- X¹ and X² represent -(CH₂)₁₀-, and
- Y represents -CH₂-.

These polyamides containing a grafted silicone moiety of formula (VII) may be copolymerized with polyamide-silicones of formula (II) to form block copolymers, alternating copolymers or random copolymers. The weight percentage of grafted silicone moieties (VII) in the copolymer may range from 0.5% to 30% by weight.

According to the invention, as has been seen previously, the siloxane units may be in the main chain or backbone of the polymer, but they may also be present in grafted or pendent chains. In the main chain, the siloxane units may be in the form of segments as described above. In the pendent or grafted chains, the siloxane units may appear individually or in segments.

According to the invention, the preferred siloxane-based polyamides are:
- polyamides of formula (III) in which m is from 15 to 50;
- mixtures of two or more polyamides in which at least one polyamide has a value of m in the range from 15 to 50 and at least one polyamide has a value of m in the range from 30 to 50;
- polymers of formula (V) with m₁ chosen in the range from 15 to 50 and m₂ chosen in the range from 30 to 500 with the portion corresponding to m₁ representing 1% to 99% by weight of the total weight of the polyamide and the portion corresponding to m₂ representing 1% to 99% by weight of the total weight of the polyamide;
- mixtures of polyamide of formula (III) combining
   1) 80% to 99% by weight of a polyamide in which n is equal to 2 to 10 and in particular 3 to 6, and
   2) 1% to 20% of a polyamide in which n is in the range from 5 to 500 and in particular from 6 to 100;
- polyamides corresponding to formula (VI) in which at least one of the groups Y. and Y¹ contains at least one hydroxyl substituent;
- polyamides of formula (III) synthesized with at least one portion of an activated diacid (diacid chloride, dianhydride or diester) instead of the diacid;
- polyamides of formula (III) in which X represents - (CH₂)₃- or -(CH₂)₁₀; and
- polyamides of formula (III) in which the polyamides end with a monofunctional chain chosen from the group consisting of monofunctional amines, monofunctional acids, monofunctional alcohols, including fatty acids, fatty alcohols and fatty amines, such as, for example, octylamine, octanol, stearic acid and stearyl alcohol.

According to the invention, the ends of the polymer chains may end with:
- a C₁ to C₅₀ alkyl ester group by introducing a C₁ - to C₅₀ monoalcohol during the synthesis,
- a C₁ to C₅₀ alkylamide group by taking as stopping group a monoacid if the silicone is α,ω-diaminated, or a monoamine if the silicone is an α,ω-dicarboxylic acid.

According to one embodiment variant of the invention, it is possible to use a copolymer of silicone polyamide and of hydrocarbon-based polyamide, i.e. a copolymer comprising moieties of formula (III) or (IV) and hydrocarbon-based polyamide moieties. In this case, the polyamide-silicone moieties may be arranged at the ends of the hydrocarbon-based polyamide.

Polyamide-based gelling agents containing silicones may be produced by silylic amidation of polyamides based on fatty acid dimer. This approach involves the reaction of free acid sites existing on a polyamide as end sites, with oligosiloxane-monoamines and/or oligosiloxane-diamines (amidation reaction), or alternatively with oligosiloxane alcohols or oligosiloxane diols (esterification reaction). The esterification reaction requires the presence of acid catalysts, as is known in the art. It is desirable for the polyamide containing free acid sites, used for the amidation or esterification reaction, to have a relatively high number of acid end groups (for example polyamides with high acid numbers, for example from 15 to 20).

For the amidation of the free acid sites of the hydrocarbon-based polyamides, siloxane diamines with 1 to 300, more particularly 2 to 50 and better still 2, 6, 9.5, 12, 13.5, 23 or 31 siloxane groups, may be used for the reaction with hydrocarbon-based polyamides based on fatty acid dimers. Siloxane diamines containing 13.5 siloxane groups are preferred, and the best results are obtained with the siloxane diamine containing 13.5 siloxane groups and polyamides containing high numbers of carboxylic acid end groups.

The reactions may be carried out in xylene to extract the water produced from the solution by azeotropic distillation, or at higher temperatures (about 180 to 200°C) without solvent. Typically, the efficacy of the amidation and the reaction rates decrease when the siloxane diamine is longer, that is to say when the number of siloxane groups is higher. Free amine sites may be blocked after the initial amidation reaction of the diaminosiloxanes by reacting them either with a siloxane acid, or with an organic acid such as benzoic acid.

For the esterification of the free acid sites on the polyamides, this may be performed in boiling xylene with about 1% by weight, relative to the total weight of the reagents, of para-toluenesulphonic acid as catalyst.

These reactions carried out on the carboxylic acid end groups of the polyamide lead to the incorporation of silicone moieties only at the ends of the polymer chain.

It is also possible to prepare a copolymer of polyamide-silicone, using a polyamide containing free amine groups, by amidation reaction with a siloxane containing an acid group.

It is also possible to prepare a gelling agent based on a copolymer between a hydrocarbon-based polyamide and a silicone polyamide, by transamidation of a polyamide having, for example, an ethylenediamine constituent, with an oligosiloxane-α,ω-diamine, at high temperature (for example 200 to 300°C), to carry out a transamidation such that the ethylenediamine component of the original polyamide is replaced with the oligosiloxane diamine.

The copolymer of hydrocarbon-based polyamide and of polyamide-silicone may also be a graft copolymer comprising a hydrocarbon-based polyamide backbone with pendent oligosiloxane groups.

This may be obtained, for example:
- by hydrosilylation of unsaturated bonds in polyamides based on fatty acid dimers;
- by silylation of the amide groups of a polyamide; or
- by silylation of unsaturated polyamides by means of an oxidation, that is to say by oxidizing the unsaturated groups into alcohols or diols, to form hydroxyl groups that are reacted with siloxane carboxylic acids or siloxane alcohols. The olefinic sites of the unsaturated polyamides may also be epoxidized and the epoxy groups may then be reacted with siloxane amines or siloxane alcohols.

As has been seen previously, gelling agents consisting of homopolymers or copolymers of the invention may contain siloxane moieties in the main chain of the polymer and groups capable of establishing hydrogen interactions, either in the main chain of the polymer or at the ends thereof, or on side chains or branches of the main chain. This may correspond to the following five arrangements: 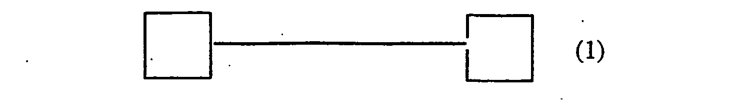 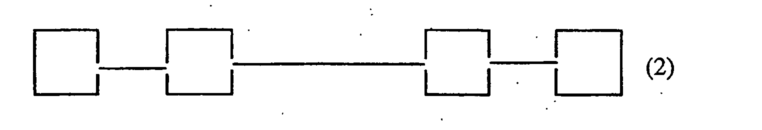 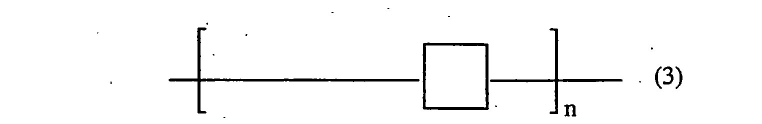 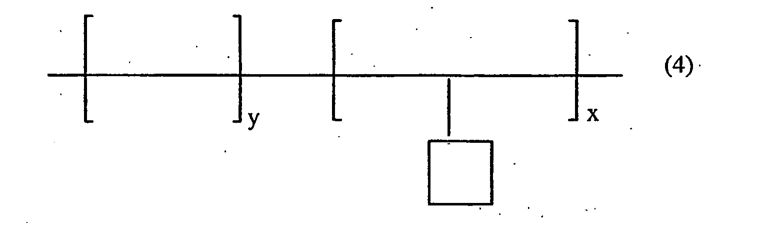 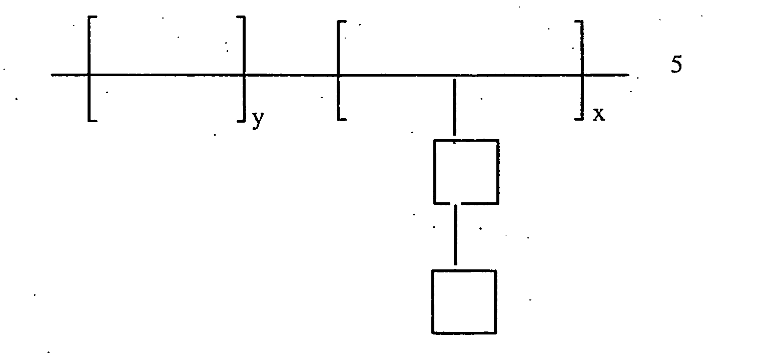 in which the continuous line is the main chain of the siloxane polymer and the squares represent the groups capable of establishing hydrogen interactions.

In case (1), the groups capable of establishing hydrogen interactions are arranged at the ends of the main chain. In case (2), two groups capable of establishing hydrogen interactions are arranged at each of the ends of the main chain.

In case (3), the groups capable of establishing hydrogen interactions are arranged within the main chain in repeating moieties.

In cases (4) and (5), these are copolymers in which the groups capable of establishing hydrogen interactions are arranged on branches of the main chain of a first series of moieties that are copolymerized with moieties not comprising groups capable of establishing hydrogen interactions. The values n, x and y are such that the polymer has the desired properties in terms of an agent for gelling fatty phases based on silicone oil.

According to the invention, the structuring of the liquid fatty phase containing at least one silicone oil, is obtained with the aid of one or more of the polymers mentioned above, in combination with one or more non-polymeric organogelling agents.

As examples of polymers that may be used, mention may be made of the silicone polyamides obtained in accordance with Examples 1 and 2 of document US-A-5 981 680.

The polymers and copolymers used in the gelling system of the composition of the invention advantageously have a softening point from 40 to 190°C. Preferably, they have a softening point ranging from 50 to 140°C and better still from 70°C to 120°C. This softening point is lower than that of the known structuring polymers, which facilitates the use of the polymers that are the subject of the invention, allows the use of volatile oils and limits the deteriorations of the liquid fatty phase.

They have good solubility in silicone oils and produce macroscopically homogeneous compositions. Preferably, they have an average molecular mass from 500 to 200 000, for example from 80 000 to 200 000, preferably from 2 000 to 30 000.

### Non-polymeric organogelling agent

The composition according to the invention contains one or more organogelling agents. This or these organogelling agent(s) make it possible to reinforce the mechanical properties of the composition, in particular the properties of resistance to shearing when it is in the form of a stick. This reinforcement results in a stick which is resistant to the shearing produced during the application of the composition to the lips or the skin, but also to the superficial body growths. Thus, it is possible to manufacture a lipstick having a stick diameter of 12.7 mm, which diameter corresponds to that customarily used in conventional lipsticks.

According to the invention, the composition comprises at least one organogelling agent. An organogelling agent is defined here as comprising a non-polymeric organic compound whose molecules may be capable of establishing, with each other, at least one physical interaction leading to self-aggregation of the molecules with formation of a three-dimensional macromolecular network which may be responsible for the gelling of the liquid fatty phase. The network can result from the formation of a network of fibrils (due to the stacking or aggregation of the organic gelling molecules), immobilizing the molecules of the liquid fatty phase. Depending on the nature of the organogelling agent, the interconnected fibrils have variable sizes which may range from a few nanometres to 1 µm or even several micrometres. These fibrils can occasionally combine to form ribbons or columns.

The term "gelling" means a thickening of the medium which may lead to a gelatinous consistency and even to a rigid, solid consistency which does not run under its own weight. The capacity to form this network of fibrils, and thus the gelling, depends on the nature (or the chemical category) of the organogelling agent, the nature of the substituents carried by its molecules for a given chemical category, and the nature of the liquid fatty phase. For example,' this gelling is reversible under the action of an external stimulus such as temperature.

The physical interactions are diverse but may exclude cocrystallization. These physical interactions are for example interactions chosen from self-complementary hydrogen interactions, π interactions between unsaturated nuclei, dipolar interactions, and coordination bonds with organometallic derivatives. The establishment of these interactions can often be promoted by the architecture of the molecule, for example by nuclei, unsaturations, and the presence of asymmetric carbon. In general, each molecule of an organogelling agent can establish several types of physical interaction with a neighbouring molecule. Thus, in one embodiment, the molecules of the organogelling agent according to the invention may comprise at least one group capable of establishing a hydrogen bond, for example at least two groups capable of establishing a hydrogen bond; at least one aromatic nucleus, for example at least two aromatic nuclei; at least one bond with ethylenic unsaturation; and/or at least one asymmetric carbon. The groups capable of forming a hydrogen bond may be chosen, for example, from the hydroxyl, carbonyl, amine, carboxylic acid, amide, benzyl, sulphonamide, carbamate, thiocarbamate, urea, thiourea, oxamido, guanidino and biguanidino groups.

The organogelling agents of the invention may be soluble in the liquid fatty phase at room temperature and at atmospheric pressure. They may be solid or liquid at room temperature and at atmospheric pressure.

Organogelling agents which may be used in the invention are, for example, those described in the document "Specialist Surfactants" published by D. Robb, 1997, pp. 209-263, chapter 8, by P. Terech [12], and the documents FR-A-2 796 276 [13] and FR-A-2 811 552 [14]. The organogelling agents described in these documents are, chosen from:
- 12-hydroxystearic acid, and salts such as the alkali metal salts (in particular the Li, Na and K salts) and the alkaline-earth metal salts (for example the magnesium salts) or esters thereof resulting from esterification with a monoalcohol or a polyol having a saturated or unsaturated, linear or cyclic chain of 1 to 6 carbon atoms;
- amides of tricarboxylic acids, for example cyclohexanetricarboxamides (see [13]), these amides corresponding, for example, to the formula (XV) given below;
- amides or esters of N-acylamino acids, for example the diamides resulting from the action of an N-acylamino acid with amines containing from 1 to 22 carbon atoms, such as those described in WO-93/23008 [15], for example N-acylglutamides in which the acyl group is a C₈ to C₂₂ alkyl chain, and the dibutylamide of N-laurylglutamic acid, such as the product sold or manufactured by the company AJINOMOTO under the name GP-1;
- dibenzylidene sorbitol or derivatives thereof, for example 1,3:2,4-di-*o-*benzylidene-D-sorbitol;
- cyclodipeptides which are cyclic condensates of two amino acids such as those described in the book "Specialist Surfactants" [12];
- the organogelling agents defined in the document WO-A-01/07007 [16] corresponding to the general formula (XVII):

   Q-O-W-(CHOH)ₛ-W¹-O-Q¹ (XVII)

   in which W and W¹, which may be identical or different, are chosen from -CH₂- and -CO-, and in which Q and Q¹, which may be identical or different, are a hydrocarbon chain chosen from saturated or unsaturated, linear or branched hydrocarbon chains containing at least 6 carbon atoms, and in which s is an integer from 2 to 4; such as the compounds in which W = W¹ = -CH₂-and s = 2 and the compounds in which W = W¹ = -CO- and s = 4;
- diamide, diurea or diurethane derivatives of amino acids such as:
   a) diamide derivatives of benzenedicarboxylic acids and of valine such as those mentioned in the article by K. Hanabusa, Chemistry Letters, pp. 767-8, 1999 [21], corresponding for example to the formulae: 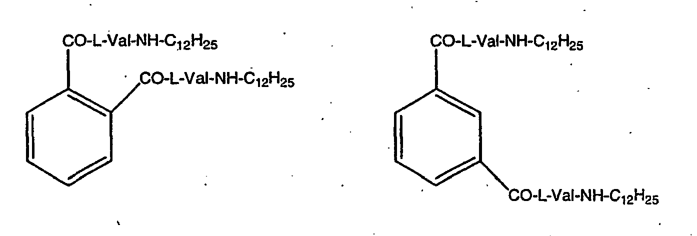 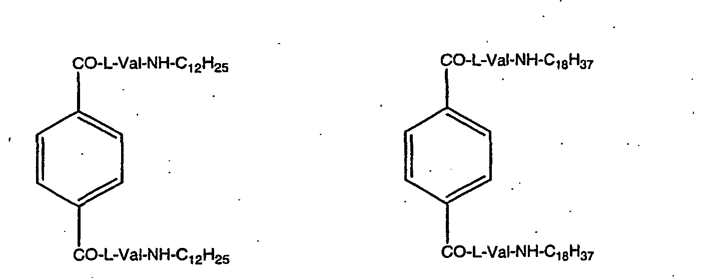 in which -L-Val- represents:

      -NH-CH [CH(CH₃)₂)] -CO- ;
- bolaamphiphilic amides derived from amido acids mentioned by K. Hanabusa, Adv. Mater., 9, No. 14, 1997, pp. 1095-1097 [24], corresponding to the formulae: 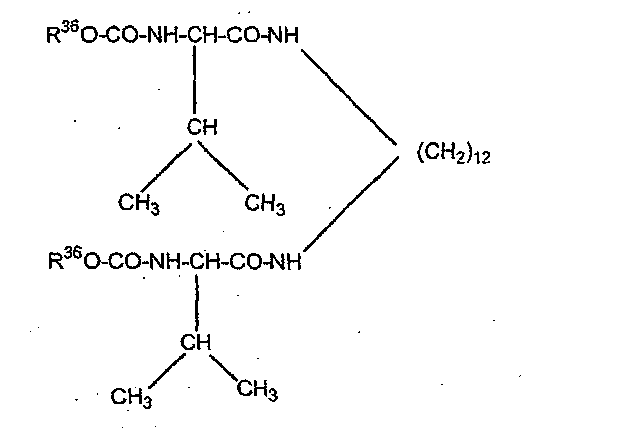 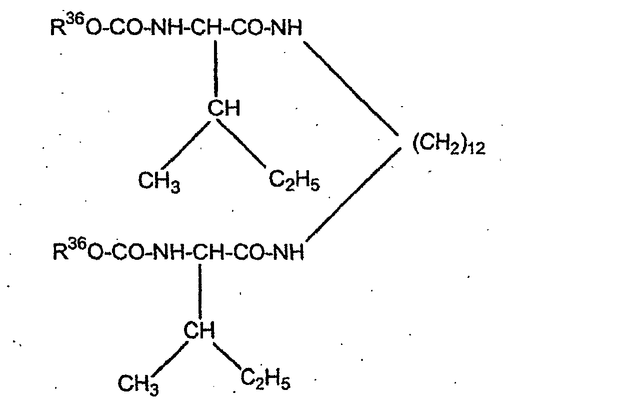 where R³⁶ = -CH₂-C₆H₅ or -CH₂-CH₃, and 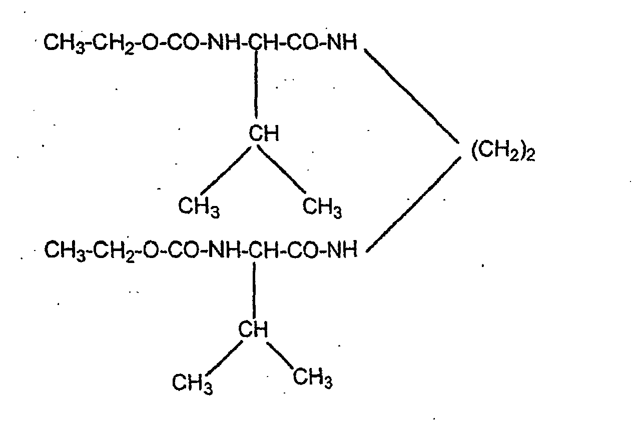
- 2-alkyl-2-ammoniumisobutyl acetate p-toluenesulphonate salts such as those described by K. Hanabusa, Colloid Polym. Sci., 276, pp. 252-59, 1998 [25], corresponding to the formula (XXII) :

   p-CH₃-C₆H₄-SO₃^{- +}H₃N-CH(R³⁷)-CO-OR³⁸ (XXII)

   in which R³⁷ represents:
   -CH₂-CH-(CH₃)₂ (leucine), -CH-(CH₃)₂ (L-valine); 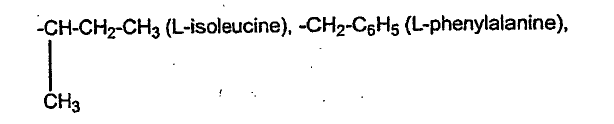 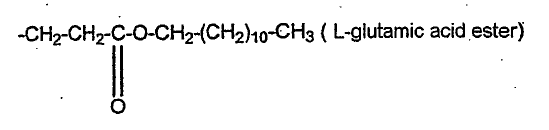 R³⁸ represents:
   [with n = 4 to 12, or]

   -CH₂-(CH₂)ₙ-CH₃ with n = 4 to 12, or 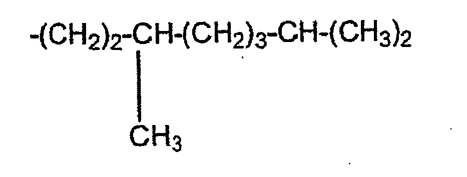
- diamides of formula (XXV) or (XXVI):

   R⁴⁴-X-CO-NH-R⁴⁵-NH-CO-X-R⁴⁴ (XXV)

   or

   R⁴⁴-CO-NH-R⁴⁵-NH-CO-R⁴⁴ (XXVI)

in which the groups R⁴⁴, which may be identical or different, represent a saturated or unsaturated, linear or branched C₈-C₆₀ hydrocarbon chain, the group (s) R⁴⁴ optionally comprising a hydroxyl group or at least one heteroatom such as N, O, S or Si, R⁴⁵ is a hydrocarbon-based group chosen from linear, branched and cyclic C₁ to C₅₀ groups and C₅ to C₈ arylene groups optionally substituted with one or more C₁-C₄ alkyl groups, and X represents -O- or -NH-.

It is also possible to use mixtures of the various organogelling agents described above.

According to one embodiment, the organogelling agent is chosen from amides of amino acids such as N-acylamino acids and mixtures thereof with cyclohexanetricarboxamides.

### Organogelling agents of formula (XV):

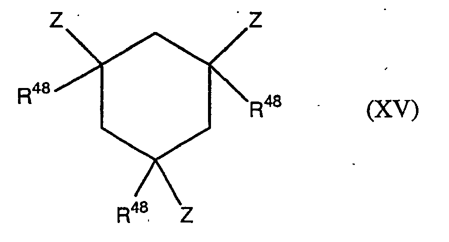

in which the groups R⁴⁸, which are identical or different, are chosen from a hydrogen atom and saturated, linear and branched hydrocarbon chains, the said hydrocarbon chains containing from 1 to 6 carbon atoms, for example from 1 to 4 carbon atoms;
- the groups Z, which are identical or different, each represent a group chosen from the following groups: -CO-S-R⁴⁹; -CO-NHR⁴⁹; -NH-COR⁴⁹ and -S-COR⁴⁹; in which the groups R⁴⁹, which may be identical or different, are chosen from:
- a hydrogen atom,
- an aryl group,
- an aralkyl group, that is to say an aryl group substituted with a hydrocarbon chain chosen from saturated, linear and branched hydrocarbon chains, in which the hydrocarbon chain contains from 1 to 22 carbon atoms, for example from 10 to 18 carbon atoms, and
- a saturated hydrocarbon chain chosen from linear, branched and cyclic hydrocarbon chains, containing from 1 to 22 carbon atoms, for example from 10 to 18 carbon atoms, optionally substituted with at least one group chosen from aryl, ester, amide and urethane groups; and/or optionally comprising at least one heteroatom chosen from O, S and N; and/or, optionally substituted with at least one fluorine atom and/or one hydroxyl radical.

R⁴⁸ is for example a hydrogen atom.

Z is for example the group -CO-NHR⁴⁹ or -NH-COR⁴⁹.

R⁴⁹ is for example an aryl group; an aralkyl group in which the linear or branched alkyl chain contains from 12 to 16 carbon atoms; or a linear or branched C₁₁-C₁₈ alkyl group.

In one embodiment, Z is a group -CO-NHR⁴⁹ in which R⁴⁹ is chosen from aryl groups substituted with a linear or branched C₁₁-C₁₆ alkyl chain, unsubstituted linear C₁₁ to C₁₈ alkyl chains and unsubstituted branched C₁₁ to C₁₈ alkyl chains.

In the compounds of formula (XV), the three substituents represented by Z may be in cis-cis, cis-trans or trans-trans conformation relative to each other. In particular, at least one of these substituents may be placed in an equatorial position on the cyclohexane nucleus; for example all the substituents Z are placed in an equatorial position.

It is also possible to use, as compound of formula (XV), a mixture of cis-cis, cis-trans and/or trans-trans compounds.

Among the compounds of formula (XV) which may be used as organogelling agent, alone or in the form of a mixture, in the composition of the invention, the following compounds may be mentioned:
- cis-1,3,5-tris(dodecylaminocarbonyl)cyclohexane,
- cis-1,3,5-tris(octadecylaminocarbonyl)cyclohexane,
- cis-1,3,5-tris[N-(3,7-dimethyloctyl)aminocarbonyl]-cyclohexane,
- trans-1,3,5-trimethyl-1,3,5-tris(dodecylaminocarbonyl)cyclohexane, and
- trans-1,3,5-trimethyl-1,3,5-tris(octadecylaminocarbonyl)cyclohexane.

The compounds of formula (XV) are well known to persons skilled in the art and may be prepared by conventional methods.

According to the invention, among the organogelling agents described above, those which can be carried in silicone oils and considered as gelling agents for these media when they are used alone, without the polymer of the invention, are preferred. They are the following compounds:
a) 12-hydroxystearic acid, its salts and its ester or amide derivatives which are described in the documents US-A-5,480,637 [30], EP-A-616 842 [31] and EP-A-665 007 [32] as gelling agents for silicone oils.
b) amides of tricarboxylic acids, for example their diamides as described in US-A-5,776,494 [33].
c) esters and amides of N-acylamino acids described in WO-A-93/23008 [15] and US-A-5, 429, 816 [34], for example N-acylglutamides where the acyl group is a C₈ to C₂₂, and the dibutylamide of N-laurylglutamic acid from Ajinomoto.
d) diureas of N-acylamino acids.such as the methyl or ethyl esters of N^{ε}-lauroyl-N^{α}stearylamino-carbonyl-L-lysine and of N^{ε}-lauroyl-N^{α}-n-butylamino-L-lysine of formulae: 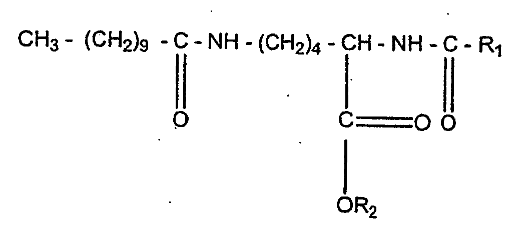
with R₁ = -NH-CH₂)₁₇-CH₃
or

-NH-(CH₂)₃-CH₃

and R₂ = -CH₃ or -C₂H₅,

These derivatives are described by K. Hanabusa, Chemistry Letters, 2000, pp. 1070-1071 [20].
e) Urethane amides of certain dipeptides such as N-benzyloxycarbonyl-L-valyl-L-valine n-octa-decylamide of formula: 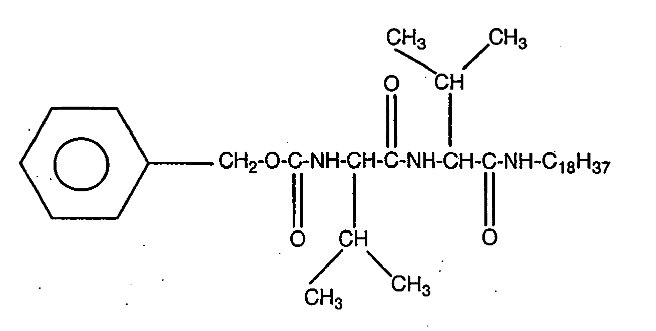 described by K. Hanabusa, J. Chem. Soc., Chem. Commun., 1993, pages 390-92 [35].
f) Dibenzylidenesorbitol and its derivatives.
g) Sterol derivatives such as:
   - the lanosterol described in EP-A-1 064 925 [36],
   - dihydrolanosterol,
   - cholesterol esters such as cholesterylphenyl acetate, cholesteryl laureate, cholesteryl cinnamate and cholesteryl 4-(2-anthryloxy)butanoate, and cholesterol esters with azobenzene groups such as: 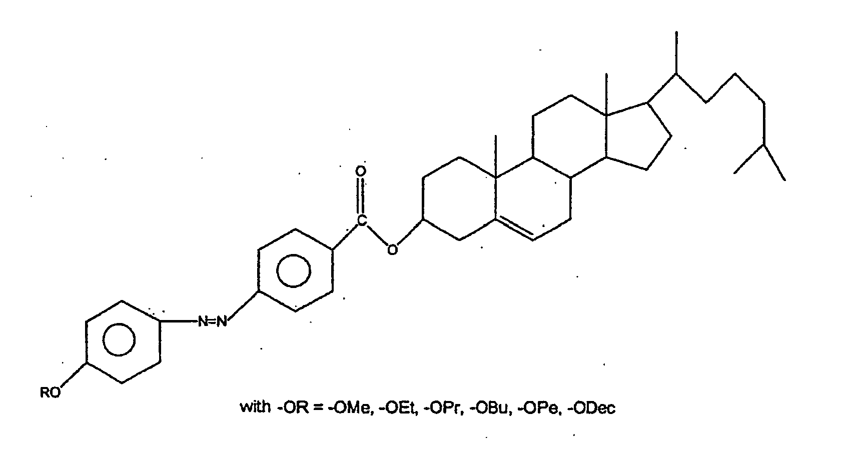 described by P. Terech, Chem. Rev., 97, 3133-59, 1997 [37].
h) Certains cyclodipeptides cited in the article by K. Hanabusa, J. Colloid and Int. Sci., 224, pp. 231-44, 2000 [38], in particular: cyclo(glycyl-L-alanyl), cyclo(glycyl-L-valyl), cyclo(glycyl-L-leucyl), cyclo(glycyl-L-phenylalanyl), cyclo(L-valyl-L-leucyl), cyclo(L-leucyl-L-leucyl), cyclo(L-phenylalanyl-L-leucyl), cyclo(L-phenylalanyl-L-phenylalanyl), cyclo(L-valyl-L-γ-3,7-dimethyloctylglutamyl), cyclo(L-valyl-L-γ-2-ethylhexylglutamyl), cyclo(L-leucyl-L-γ-ethylglutamyl), cyclo(L-leucyl-L-γ-dodecylglutamyl), cyclo(L-leucyl-L-γ-3,7-dimethyloctylglutamyl), cyclo(L-leucyl-L-γ-benzylglutamyl), cyclo(L-β-butylasparaginyl-L-phenylalanyl), cyclo(L-γ-dodecylasparaginyl-L-phenylalanyl), cyclo(L-β-3,7-dimethyloctylasparaginyl-L-phenylalanyl), cyclo(L-β-2-ethylhexylasparaginyl-L-phenylalanyl), cyclo(L-β-3,5,5-trimethylhexylasparaginyl-L-phenylalanyl) and cyclo(L-β-2-ethylbutylasparaginyl-L-phenylalanyl).
i) the trans-(1R,2R)-bis(undecylcarbonylamino)cyclohexane derivative of formula: 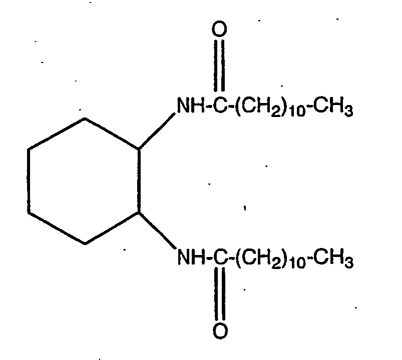
j) Fluorinated ethers such as those defined in US-A-6, 002, 048 [39].
k) The organogelling agents defined in WO 01/07007 [16], of general formula (XVII) described above.
l) The bolaamphiphilic amide organogelling agents derived from amino acids mentioned above.
m) The 2-alkyl-2-ammoniumisobutyl acetate p-toluenesulfonate salts of formula (XXII) cited above, in particular that for which R³⁷ is derived from L-leucine and R³⁸ represents - (CH₂)₁₁CH₃.
n) The diamide derivatives of benzenedicarboxylic acid and of valine cited above.
o) The non-polymeric organogelling agents of formula (XXV) or (XXVI) described above.

Among the preferred organogelling agents, the ones which will be even more preferred are those which are compatible with silicone oils and which in addition possess groups which can give hydrogen interactions with the polyorganosiloxane polymers used in the invention, that is amide, urea, urethane, ester, sulfonamide, carbamate, thiocarbamate, thiourea, oxamido, guanidino and biguanidino groups, and combinations thereof.

In a particularly preferred manner, there will be used in accordance with the invention:
- 12-hydroxystearic acid amide derivatives,
- amides of tricarboxylic acids,
- esters or amides of N-acylamino acids,
- the diureas cited above,
- urethane amides of certain dipeptides,
- the cyclodipeptides cited above,
- the derivatives of formula (XIV) and more particularly trans-(1R,2R)-bis(undecylcarbonylamino)-cyclohexane,
- bolaamphipilic amide organogelling agents derived from amino acids,
- diamide derivatives of benzenedicarboxylic acid and of valine, and
- organogelling agents which are soluble in silicone oils and which have a non-polymeric structure, defined by the formulae XXV and XXVI given above.

According to the invention, the polymer may be combined with at least one amphiphilic compound which is liquid at room temperature, having a hydrophilic/lipophilic balance (HLB) value of less than 12, in particular ranging from 1 to 7, preferably from 1 to 5, and even better from 3 to 5. According to the invention, it is possible to use one or more amphiphilic compounds. The aim of these amphiphilic compounds is to reinforce the structuring properties of the polymer, to facilitate the use of the polymer and to enhance the capacity of the stick to form a deposit.

According to the invention, the composition preferably has a hardness ranging from 20 to 2 000 gf and better still from 20 to 900 gf, particularly from 20 to 600 gf, and for example from 150 to 450 gf. This hardness may be measured according to a method of penetration of a probe into the said composition and in particular with the aid of a texture analyser (for example TA-TXT2i from Rheo) equipped with an ebonite cylinder 25 mm in height and 8 mm in diameter. The hardness measurement is carried out at 20°C at the centre of five samples of the said composition. The cylinder is introduced into each sample of composition at a pre-speed of 2 mm/s, then at a speed of 0.5 mm/s and finally at a post-speed of 2 mm/s, the total displacement being 1 mm. The recorded hardness value.is that of the maximum peak. The measurement error is ± 50 gf.

The hardness may also be measured by the "cheese wire" method, which consists in cutting a tube of lipstick 12.7 mm or 8.1 mm in diameter and in measuring the hardness at 20°C, using a DFGHS 2 tensile testing machine from the company Indelco-Chatillon, travelling at a speed of 100 mm/minute. It is expressed as the shear force (expressed in gram-force) required to cut a stick under these conditions. According to this method, the hardness of a composition in stick form according to the invention ranges from 30 to 300 gf, preferably from 30 to 250 gf, for a stick 12.7 mm in diameter and for example from 30 to 120 gf for a stick 8.1 mm in diameter.

The hardness of the composition according to the invention is such that the composition is self-supporting and can disintegrate easily to form a satisfactory deposit on the skin and the lips. In addition, with this hardness, the composition of the invention shows good impact strength.

According to the invention, the composition in stick form has the behaviour of a deformable and supple elastic solid, giving noteworthy elastic softness on application. The stick compositions of the prior art do not have this property of elasticity and suppleness.

The amphiphilic, silicone and non-silicone compound(s) which can be used in the composition of the invention comprise a lipophilic part linked to a polar part, the lipophilic part containing a carbon chain having at least 8 carbon atoms, in particular from 18 to 32 carbon atoms and even better from 18 to 28 carbon atoms. Preferably, the polar part of this or these amphiphilic compound(s) is the residue of a compound chosen from alcohols and polyols having from 1 to 12 hydroxyl groups, polyoxyalkylenes containing at least two oxyalkylenated moieties and having from 0 to 20 oxypropylenated moieties and/or from 0 to 20 oxyethylenated moieties. In particular, the amphiphilic compound is an ester chosen from hydroxystearates oleates and isostearates of glycerol, sorbitan or methylglucose, or branched C₁₂ to C₂₆ fatty alcohols such as octyldodecanol and mixtures thereof. Among these esters, monoesters and mixtures of mono- and diesters are preferred.

The respective amounts of lipophilic non-polymeric organogelling agent and of structuring silicone polymer and optionally of amphiphilic compound are chosen-according to the desired gel hardness and depending on the particular application envisaged. The respective quantities of polymer, organogelling agent and optionally of amphiphilic compound should be such that they allow the production of a self-supported composition, for example in the form of a disintegrable stick. In practice, the quantity of polymer (as active material) represents from 0.5 to 80% of the total weight of the composition, and even better from 5 to 40%. The quantity of amphiphilic compound represents in practice from 0.1% to 35% of the total weight of the composition, for example from 1% to 20% and even better from 2% to 15%. The quantity of organogelling agent represents in practice from 0.1 to 80%, preferably from 0.5 to 60%, and even better from 1 to 40% and better still from 1 to 15% of the total weight of the composition.

According to the invention, it is in fact preferable for the quantity of organogelling agent to be smaller than the quantity of structuring silicone polymer.

Generally, the silicone polymer/non-polymeric organogelling agent mass ratio is in the range from 20 to 0.15, preferably from 15 to 1.5.

### Other additive

The composition of the invention may also comprise any ingredient usually used in the field under consideration, and especially those chosen from dyes that are soluble in polyols or in the fatty phase, antioxidants, essential oils, preserving agents, perfumes, liposoluble polymers, especially hydrocarbon-based liposoluble polymers such as polyalkylenes or polyvinyl laurate, liquid-fatty-phase gelling agents, waxes, gums, resins, surfactants, for instance trioleyl phosphate, additional cosmetic or dermatological active agents such as, for example, water, emollients, moisturizers, vitamins, liquid lanolin, essential fatty acids, lipophilic sunscreens or sunscreens that are soluble in polyols, and mixtures thereof. The composition according to the invention may also contain lipid vesicles of ionic and/or non-ionic type. These ingredients, besides the water, may be present in the composition in the usual manner in a proportion of from 0% to 20% of the total weight of the composition and better still from 0.1% to 10%.

In the case where the composition contains an aqueous phase, which is the case for a simple or multiple emulsion, this aqueous phase can represent 0.1% to 70% of the total weight of the composition, especially from 0.5% to 40% and better still from 1% to 20%. This aqueous phase can contain any water-miscible compound such as polyols and may be optionally gelled with a suitable gelling agent.

Needless to say, the person skilled in the art will take care to select the optional additional ingredients and/or the amount thereof such that the advantageous properties of the composition according to the invention are not, or are not substantially, adversely affected by the envisaged addition.

The compositions of the invention may in particular contain one or more waxes, for example polyethylene wax, but the use of wax is avoided if it is desired to obtain glossy, or even transparent products. Generally, the amount of wax does not exceed 20% and preferably 10%; it represents, for example, from 3% to 5% of the total weight of the composition.

The composition according to the invention may be in the form of an optionally tinted dermatological or care composition for keratinous materials such as the skin, the lips and/or the superficial body growths, in the form of a sun protection or care composition, especially in the form of a make-up-removing product in stick form. It can especially be used as a care base for the skin, the superficial body growths or the lips (lip balms, for protecting the lips against the cold and/or sunlight and/or the wind, or a care cream for the skin, the nails or the hair).

The composition of the invention may be provided in particular in the form of a rigid gel, in particular in the form of a transparent anhydrous stick.

The composition of the invention may also be in the form of a coloured make-up product for the skin, in particular a foundation, optionally having care or treatment properties, a blusher, a face powder, an eyeshadow, a concealer product, an eyeliner or a make-up product for the body; a lip make-up, for instance a lipstick, optionally having care or treatment properties; a make-up for the superficial body growths, for instance the nails or the eyelashes, in particular in the form of a mascara cake, or for the eyebrows and the hair, especially in the form of a pencil. In particular, the composition of the invention may be a cosmetic product containing cosmetic and/or dermatological active agents, for instance essential oils, vitamins, moisturizers, sunscreens, cicatrizing agents and ceramides.

In the case of make-up compositions, hydrophobic or hydrophilic solid particles may constitute the pigment(s) for making up the skin, the lips and/or the superficial body growths.

Needless to say, the composition of the invention must be cosmetically or dermatologically acceptable, that is to say that it must contain a nontoxic physiologically acceptable medium that can be applied to the skin, the superficial body growths or the lips of human beings. For the purposes of the invention, the expression "cosmetically acceptable" means a composition of pleasant appearance, odour and feel.

Moreover, the make-up or care compositions in accordance with the invention must comprise at least 10% by mass of a non-volatile oil (silicone oil or non-silicone oil) and/or of a pasty or viscous product in order to obtain a product which is comfortable and which does not cause tightness.

The expression pasty product is understood to mean a viscous fatty substance containing a liquid fraction and a solid fraction. For the purposes of the invention, the expression "pasty fatty substances" means fatty substances with a melting point ranging from 20 to 55°C and preferably 25 to 45°C, and/or a viscosity at 40°C ranging from 0.1 to 40 Pa.s (1 to 400 poises) and preferably 0.5 to 25 Pa.s measured using a Contraves TV or Rheomat 180 viscometer, equipped with a spindle rotating at 240 min⁻¹ for a power supply at 60 Hz or at 200 min⁻¹ for a power supply at 50 Hz.

A person skilled in the art can select the spindle for measuring the viscosity, from the spindles MS-r3 and MS-r4, on the basis of his general knowledge, so as to be able to measure the viscosity of the tested pasty compound.

The melting point values correspond, according to the invention, to the melting peak measured by the "Differential Scanning Calorimetry" method with a temperature rise of 5 or 10°C/min.

By way of example of pasty products that may be used in the invention, mention may be made of lanolins and lanolin derivatives, for instance acetylated lanolins or oxypropylenated lanolins or isopropyl lanolate, with a viscosity of 18 to 21 Pa.s and preferably 19 to 20.5 Pa.s, and/or a melting point of 30 to 55°C, preferably 30 to 40°C, and mixtures thereof. Esters of fatty acids or of fatty alcohols may also be used, especially those containing 20 to 65 carbon atoms (melting point of about 20 to 35°C and/or viscosity at 40°C ranging from 0.1 to 40 Pa.s), for instance triisostearyl citrate or cetyl citrates arachidyl propionate; polyvinyl laurate; cholesterol esters, for instance triglycerides of plant origin such as hydrogenated plant oils, viscous polyesters, for instance poly(12-hydroxystearic acid) and mixtures thereof. Triglycerides of plant origin that may be used include hydrogenated castor oil derivatives, such as "THIXINR" from Rheox.

Mention may also be made of silicone-based pasty fatty substances such as polydimethylsiloxanes (PDMSs) containing pendent chains of the alkyl or alkoxy type containing from 8 to 24 carbon atoms, and having a melting point of 20-55°C, for example 20 to 40°C, for instance stearyl dimethicones, especially those sold by the company Dow Corning under the trade names DC2503 and.DC25514, and mixtures thereof.

The pasty fatty, substance (s) may be present in a proportion of from 0 to 60% by weight relative to the total weight of the composition, preferably in a proportion of 0.1-45% by weight and even more preferably in a proportion of 2-30% by weight.

According to the invention, the composition may furthermore contain colouring matter which may be a pigment or a pearlescent agent as defined above, or a soluble compound chosen from lipophilic dyes, hydrophilic dyes, and mixtures thereof.

The liposoluble dyes are, for example, Sudan red, DC Red 17, DC Green 6, β-carotene, soybean oil, Sudan brown, DC Yellow 11, DC Violet 2, DC Orange 5, quinoline yellow and annatto. The hydrophilic dyes are for example beet juice and methylene blue. Each type of dye can represent from 0% to 20% of the weight of the composition and better still from 0.1% to 6% (if present).

The composition according to the invention may be manufactured by known methods, generally used in the cosmetic or dermatological field. It may be manufactured by the method which consists in heating the polymer at least to its softening point, adding thereto the pasty compounds and/or the optional waxes, the oil(s), the organogelling agent, if necessary the amphiphilic compound(s), the colouring matter and/or the solid particles, and the additives, and then in mixing the whole until a transparent, clear solution is obtained. The homogeneous mixture obtained can then be cast in a suitable mould such as a lipstick mould, or directly into the packaging articles (especially a case or dish).

The subject of the invention according to a preferred embodiment of claim 1 is also a make-up structured solid composition for the skin, the lips and/or the superficial body growths, containing at least one pigment in a sufficient quantity for applying make-up to the skin, the lips and/or the superficial body growths and a liquid continuous fatty phase comprising at least one silicone oil structured with at least one polymer (homopolymer or copolymer) having a weight-average molecular mass ranging from 500 to 500 000, containing at least one moiety comprising:
- at least one polyorganosiloxane group, consisting of 1 to 1 000 organosiloxane units in the chain of the moiety or in the form of a graft, and
- at least two groups capable of establishing hydrogen interactions chosen from amide,
the polymer being solid at room temperature and soluble in the liquid fatty phase at a temperature of 25 to 250°C,
the liquid fatty phase consisting partially or totally of silicone oil(s) and containing a non-polymeric organogelling agent,
the said composition being provided in the form of a solid, and the pigment, the liquid fatty phase, the organogelling agent and the polymer forming a physiologically acceptable medium.

This make-up composition is preferably self-supporting.

The subject of the invention according to a preferred embodiment of claim 1 is also a lipstick structured composition, containing at least one pigment in a sufficient quantity for applying make-up to the lips and a liquid continuous fatty phase comprising at least one silicone oil structured with at least one polymer (homopolymer or copolymer) having a. weight-average molecular mass ranging from 500 to 500 000, containing at least one moiety comprising:
- at least one polyorganosiloxane group, consisting of 1 to 1 000 organosiloxane units in the chain of the moiety or in the form of a graft, and
- at least two groups capable of establishing hydrogen interactions chosen from amide,
the polymer being solid at room temperature and soluble in the liquid fatty phase at a temperature of 25 to 250°C,
the liquid fatty phase consisting partially or totally of silicone oil(s) and of an organogelling agent,
the said composition being provided in the form of a solid, and the pigment, the liquid fatty phase and the polymer forming a physiologically acceptable medium.

The composition of the invention may be provided in the form of a cake mascara, an eyeliner, a foundation, a lipstick, a blusher, a make-up-removing product, a make-up product for the body, an eyeliner or a face powder, or a concealer product.

The subject of the invention according to a preferred embodiment of claim 1 is also a make-up stick for the skin, the lips and/or the superficial body growths, and in particular for the lips, containing at least one pigment in a sufficient quantity for applying make-up to the skin, the lips and/or the superficial body growths and a liquid continuous fatty phase comprising at least one silicone oil structured with at least one polymer (homopolymer or copolymer) having a weight-average molecular mass ranging from 500 to 500 000, containing at least one moiety comprising:
- at least one polyorganosiloxane group, consisting of 1 to 1 000 organosiloxane units in the chain of the moiety or in the form of a graft, and
- at least two groups capable of establishing hydrogen interactions chosen from amide, the polymer being solid at room temperature and soluble in the liquid fatty phase at a temperature of 25 to 250°C,
   the liquid fatty phase consisting partially or totally of silicone oil(s) and of organogelling agent, the pigment, the fatty phase and the polymer forming a physiologically acceptable medium;

The invention relates to a cosmetic care, make-up or treatment method for the keratinous materials of human beings, comprising the application to the keratinous materials of a cosmetic composition in accordance with the invention.

The invention finally relates to a cosmetic method for limiting the migration of a cosmetic composition according to claim 1 containing a liquid fatty phase comprising at least one silicone oil, consisting in structuring the said fatty phase with a sufficient quantity of at least one polymer (homopolymer or copolymer) having a weight-average molecular mass ranging from 500 to 500 000, containing at least one moiety comprising:
- at least one polyorganosiloxane group, consisting of 1 to 1 000 organosiloxane units in the chain of the moiety or in the form of a graft, and
- at least two groups capable of establishing hydrogen interactions chosen from amide,
   the polymer being solid at room temperature and soluble in the liquid fatty phase at a temperature of 25 to 250°C,
the liquid fatty phase consisting partially silicone oil(s) and containing at least one organbgelling agent.

### REFERENCES

[1] EP-A-1 068 856
[2] WO-A-01/97758
[3] US-A-5 874 069
[4] US-A-5 919 441
[5] US-A-6 051 216
[6] WO-A-02/17870
[7] WO-A-02/17871
[8] EP-A-1 177 784
[9] US-A-5 412 004
[10] EP-A-1 048 686
[11] US-A-5 981 680
[12] Article by D. Robb, 1997, pp. 209-263, chapter 8 and by P. Terech "Specialist Surfactants"
[13] FR-A-2 796 276
[14] FR-A-2 811 552
[15] WO-93/23008
[16] WO-A-01/07007
[17] Article by R.J.H. Hafkamp, Chem. Commun., 1997, pages 545-546
[18] Article J. Org. Chem., vol. 64, No. 2, 412-26 (1999)
[19] Article by M. Jokic, J. Chem. Soc., Chem. Commun., pages 1723-24 (1995)
[20] Article by K. Hanabusa, Chemistry Letters, pp. 1070-71, 2000
[21] Article by K. Hanabusa, Chemistry Letters, pp. 767-8, 1999
[22] Article by X. Luo, Chem. Commun., pp. 2091-92, 2000
[23] Article by T. Shimizu, J. Am. Chem. Soc., 119, pp. 2812-18, 1997
[24] Article by K. Hanabusa, Adv. Mater., 9, No. 14, 1997, pp. 1095-1097
[25] Article by K. Hanabusa, Colloïd Polym. Sci., 276, pp. 252-59, 1998
[26] WO-A-00/61080
[27] WO-A-00/61081
[28] US-A-6 156 325
[29] Article by K. Hanabusa, Agnew. Chem., 108, 1997, 17, pp. 2086-2088
[30] US-A-5 480 637
[31] EP-A-616 842
[32] EP-A-665 007
[33] US-A-5 776 494
[34] US-A-5 429 816
[35] Article by K. Hanabusa, J. Chem. Soc., Chem. Commun., 1993, pp. 390-92
[36] EP-A-1 064 925
[37] Article by P. Terech, Chem. Rev., 97, 3133-59, 1997
[38] Article by K. Hanabusa, J. Colloid and Int. Sci., 224, pp. 231-44, 2000
[39] US-A-6 002 048
[40] WO-A-99/06473
[41] US-A-6 353 076

## Claims

1. Care and/or make-up cosmetic composition comprising a liquid fatty phase comprising at least one silicone oil, structured with a gelling system comprising:
1) at least one polymer (homopolymer or copolymer) having a weight-average molecular mass ranging from 500 to 500 000, containing at least one moiety comprising:
- at least one polyorganosiloxane group consisting of 1 to 1 000 organosiloxane units in the chain of the moiety or in the form of a graft, and
- at least two groups capable of establishing hydrogen interactions, said group being amide,
the polymer being solid at room temperature and soluble in the liquid fatty phase at a temperature of 25 to 250°C, and
2) at least one non-polymeric organogelling agent,
the liquid fatty phase and the gelling system forming a physiologically acceptable medium, and the liquid fatty phase comprising at least one volatile silicone oil and at least one volatile non-silicone oil, and further containing a non volatile non silicone oil,
in which the organogelling agent comprises at least one compound capable of gelling a silicone oil, chosen from:
a) 12-hydroxystearic acid, its salts and its ester or amide derivatives,
b) amides of tricarboxylic acids,
c) esters and amides of N-acylamino acids,
d) diureas of N-acylamino acids,
e) urethane amides of dipeptides,
f) dibenzylidenesorbitol and its derivatives,
g) sterol derivatives,
h) cyclodipeptides chosen from cyclo(glycyl-L-alanyl), cyclo(glycyl-L-valyl), cyclo(glycyl-L-leucyl), cyclo(glycyl-L-phenylalanyl), cyclo(L-valyl-L-leucyl), cyclo(L-leucyl-L-leucyl), cyclo(L-phenylalanyl-L-leucyl), cyclo(L-phenylalanyl-L-phenylalanyl), cyclo(L-valyl-L-γ-3,7-dimethyloctylglutamyl), cyclo(L-valyl-L-γ-2-ethylhexylglutamyl), cyclo(L-leucyl-L-y-ethylglutamyl), cyclo(L-leucyl-L-γ-dodecylglutamyl), cyclo(L-leucyl-L-γ-3,7-dimethyloctylglutamyl), cyclo(L-leucyl-L-y-benzylglutamyl), cyclo(L-β-butylasparaginyl-L-phenylalanyl), cyclo(L-γ-dodecylasparaginyl-L-phenylalanyl), cyclo(L-β-3,7-dimethyloctylasparaginyl-L-phenylalanyl), cyclo(L-β-2-ethy]-hexylasparaginyl-L-phenylalanyl), cyclo(L-β-3,5,5-trimethylhexylasparaginyl-L-phenylalanyl) and cyclo(L-β-2-ethylbutylasparaginyl-L-phenylalanyl),
i) trans-(1R,2R)-bis(undecylcarbonylamino)cyclohexane of formula: 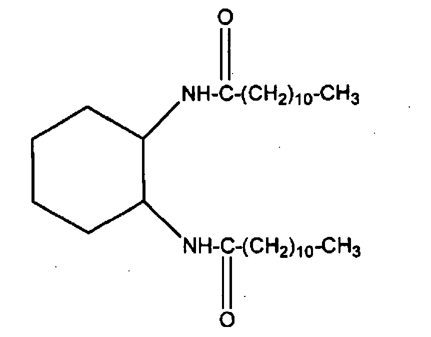
j) fluorinated ethers,
k) organogelling agents of formula (XVII):
Q-O-W-(CHOH)ₛ-W¹-O-Q¹ (XVII)
in which W and W¹, which may be identical or different, are chosen from -CH₂- and -CO-, and in which Q and Q¹, which may be identical or different, are a hydrocarbon chain chosen from saturated or unsaturated, linear or branched hydrocarbon chains containing at least 6 carbon atoms, and in which s is an integer from 2 to 4;
l) bolaamphiphilic amides derived from amino acids of formulae: 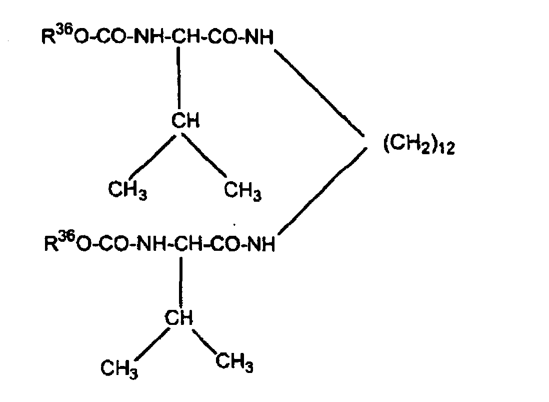 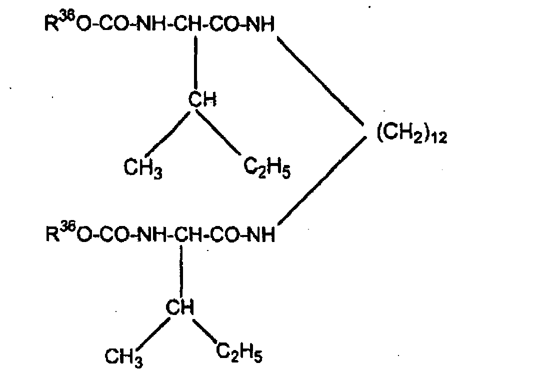 where R³⁶= -CH₂-C₆H₅ or -CH₂-CH₃, and 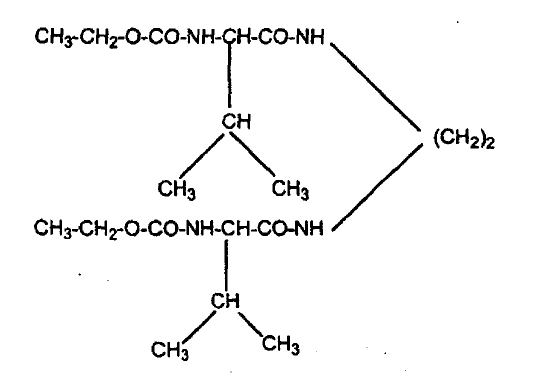
m) 2-alkyl-2-ammoniumisobutyl acetate p-toluenesulphonate salts of formula (XXII):
p-CH₃-C₆H₄-SO₃^{- +}H₃N-CH(R³⁷)-CO-OR³⁸ (XXII)
in which R³⁷ represents:
-CH₂-CH-(CH₃)₂ (leucine), -CH-(CH₃)₂ (L-valine), 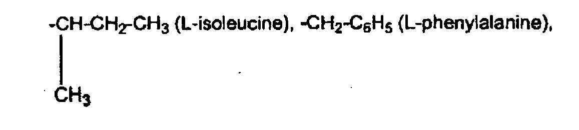 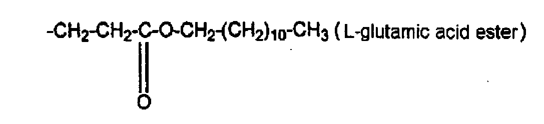
R³⁸ represents:
-CH₂-(CH₂)ₙ-CH₃ with n = 4 to 12, or 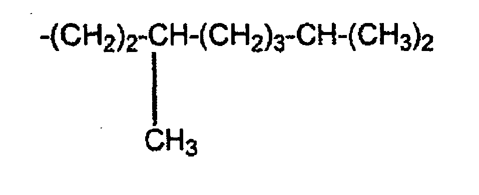
n) diamide derivatives of benzenedicarboxylic acid and of valine of formulae: 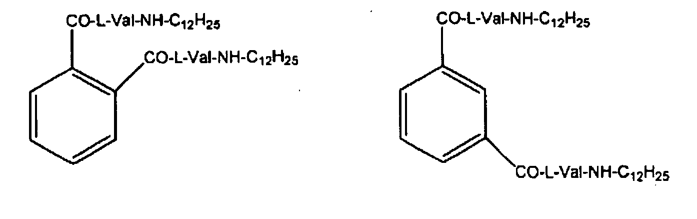 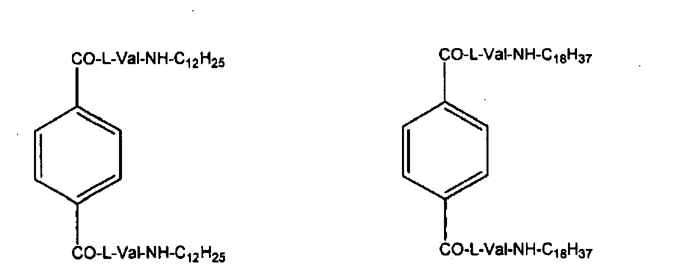 in which -L-Val- represents:
-NH-CH [CH(CH₃)₂)] -CO-;
o) diamides of formula (XXV) or (XXVI):
R⁴⁴-X-CO-NH-R⁴⁵-NH-CO-X-R⁴⁴ (XXV)
or
R⁴⁴-CO-NH-R⁴⁵-NH-CO-R⁴⁴ (XXVI)
in which the groups R⁴⁴, which may be identical or different, represent a saturated or unsaturated, linear or branched C₈-C₆₀ hydrocarbon chain, the group(s) R⁴⁴ optionally comprising a hydroxyl group or at least one heteroatom such as N, O, S or Si, R⁴⁵ is a hydrocarbon-based group chosen from linear, branched and cyclic C₁ to C₅₀ groups and C₅ to C₈ arylene groups optionally substituted with one or more C₁-C₄ alkyl groups, and X represents -O- or NH-.

2. Composition according to Claim 1, in which the volatile silicone oil has a flash point equal to or greater than 40°C and advantageously greater than the softening point of the gelling system.

3. Composition according to Claim 1 or 2, in which the volatile silicone oil is chosen from the group consisting of the following compounds: octyltrimethicone, hexyltrimethicone, octamethylcyclotetrasiloxane D4, dodecamethylcyclohexasiloxane D6, heptamethyloctyltrisiloxane, decamethyltetrasiloxane, dodecamethylpentasiloxane, polydimethylsiloxane of 1.5 cSt, polydimethylsiloxane of 2 cSt, polydimethylsiloxane of 3 cSt, polydimethylsiloxane of 5 cSt, and mixtures thereof.

4. Composition according to any one of the preceding Claim, in which the volatile oil has a flash point of 40 to 135°C.

5. Composition according to any one of the preceding Claims, in which the liquid fatty phase contains at least 30%, and even better at least 40% by weight of silicone oil.

6. Composition according to any one of the preceding Claims, in which the volatile oil represents from 3 to 89.4% of the total weight of the composition.

7. Composition according to any one of the preceding Claims, comprising, in addition, solid particles chosen from fillers, pigments and mixtures thereof.

8. Composition according to Claim 7, in which the solid particles are hydrophobic particles.

9. Composition according to the preceding Claim, in which the solid particles are hydrophilic particles, coated with a film of hydrophobic compound.

10. Composition according to Claim 7, in which the solid particles are hydrophilic particles and the composition further comprises an amphiphilic silicone.

11. Composition according to one of Claims 7 to 10, in which the particles are pigments chosen from zinc oxides, iron oxides, titanium oxides and mixtures thereof.

12. Composition according to any one of the preceding Claims, in which the polymer used in the gelling system comprises at least one moiety corresponding to the formula: 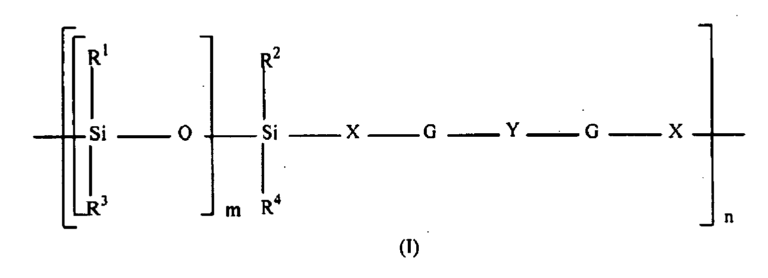 in which:
1) R¹, R², R³ and R⁴, which may be identical or different, represent a group chosen from:
- linear, branched or cyclic, saturated or unsaturated, C₁ to C₄₀ hydrocarbon-based groups, possibly containing in their chain one or more oxygen, sulphur and/or nitrogen atoms, and possibly being partially or totally substituted with fluorine atoms,
- C₆ to C₁₀ aryl groups, optionally substituted with one or more C₁ to C₄ alkyl groups,
- polyorganosiloxane chains possibly containing one or more oxygen, sulphur and/or nitrogen atoms;
2) the groups X, which may be identical or different, represent a linear or branched C₁ to C₃₀ alkylenediyl group, possibly containing in its chain one or more oxygen and/or nitrogen atoms;
3) Y is a saturated or unsaturated, C₁ to C₅₀ linear or branched divalent alkylene, arylene, cycloalkylene, alkylarylene or arylalkylene group, possibly comprising one or more oxygen, sulphur and/or nitrogen atoms, and/or bearing as substituent one of the following atoms or groups of atoms:
fluorine, hydroxyl, C₃ to C₈ cycloalkyl, C₁ to C₄₀ alkyl, C₅ to C₁₀ aryl, phenyl optionally substituted with 1 to 3 C₁ to C₃ alkyl groups, C₁ to C₃ hydroxyalkyl and C₁ to C₆ aminoalkyl, or
4) Y represents a group corresponding to the formula: 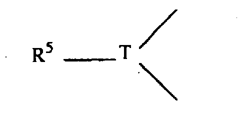 in which
- T represents a linear or branched, saturated or unsaturated, C₃ to C₂₄ trivalent or tetravalent hydrocarbon-based group optionally substituted with a polyorganosiloxane chain, and possibly containing one or more atoms chosen from O, N and S, or T represents a trivalent atom chosen from N, P and Al, and
- R⁵ represents a linear or branched C₁ to C₅₀ alkyl group or a polyorganosiloxane chain, possibly comprising one or more ester, amide, urethane, thiocarbamate, urea, thiourea and/or sulphonamide groups, which may possibly be linked to another chain of the polymer;
5) the groups G, which may be identical or different, represent divalent groups of formula: 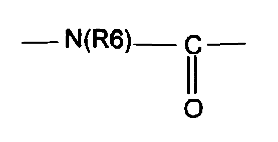 in which R⁶ represents a hydrogen atom or a linear or branched C₁ to C₂₀ alkyl group, on condition that at least 50% of the groups R⁶ of the polymer represent a hydrogen atom and that at least two of the groups G of the polymer are a group other than: 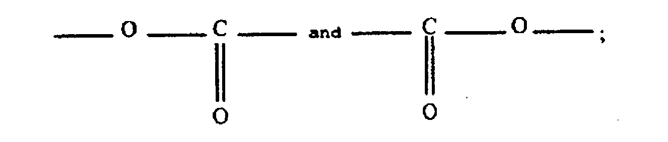
6) n is an integer ranging from 2 to 500 and preferably from 2 to 200, and m is an integer ranging from 1 to 1 000, preferably from 1 to 700 and better still from 6 to 200.

13. Composition according to the preceding Claim, in which Y represents a group chosen from:
a) linear C₁ to C₂₀ and preferably C₁ to C₁₀ alkylene groups,
b) C₃₀ to C₅₆ branched alkylene groups possibly comprising rings and unconjugated unsaturations,
c) C₅-C₆ cycloalkylene groups,
d) phenylene groups optionally substituted with one or more C₁ to C₄₀ alkyl groups,
e) C₁ to C₂₀ alkylene groups comprising from 1 to 5 amide groups,
f) C₁ to C₂₀ alkylene groups comprising one or more substituents chosen from hydroxyl, C₃ to C₈ cycloalkane, C₁ to C₃ hydroxyalkyl and C₁ to C₆ alkylamine groups,
g) polyorganosiloxane chains of formula: 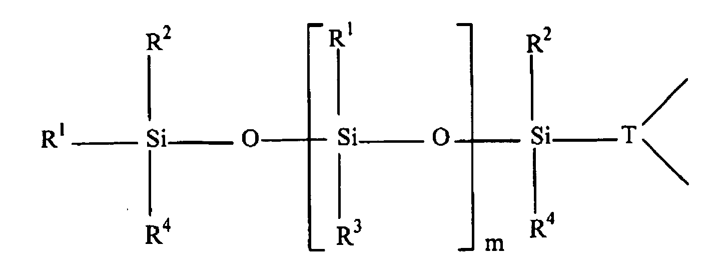 in which R¹, R², R³, R⁴, T and m are as defined above.

14. Composition according to any one of Claims 1 to 11, in which the polymer used in the gelling system comprises at least one moiety corresponding to formula (II): 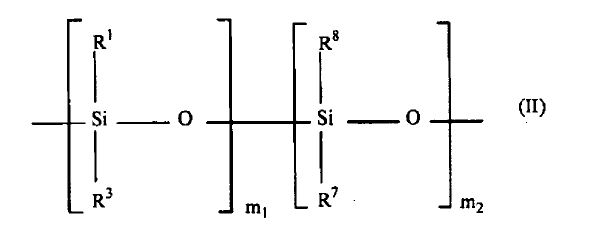 in which
- R¹ and R³, which may be identical or different, are as defined above for formula (I) in Claim 12,
- R⁷ represents a group as defined above for R¹ and R³, or represents the group of formula -X-G-R⁹ in which X and G are as defined above for formula (I) in Claim 12 and R⁹ represents a hydrogen atom or a linear, branched or cyclic, saturated or unsaturated, C₁ to C₅₀ hydrocarbon-based group optionally comprising in its chain one or more atoms chosen from O, S and N, optionally substituted with one or more fluorine atoms and/or one or more hydroxyl groups, or a phenyl group optionally substituted with one or more C₁ to C₄ alkyl groups,
- R⁸ represents the group of formula -X-G-R⁹ in which X, G and R⁹ are as defined above,
- m₁ is an integer ranging from 1 to 998, and
- m₂ is an integer ranging from 2 to 500.

15. Composition according to Claim 12, in which the polymer comprises at least one moiety of formula (III) or (IV): 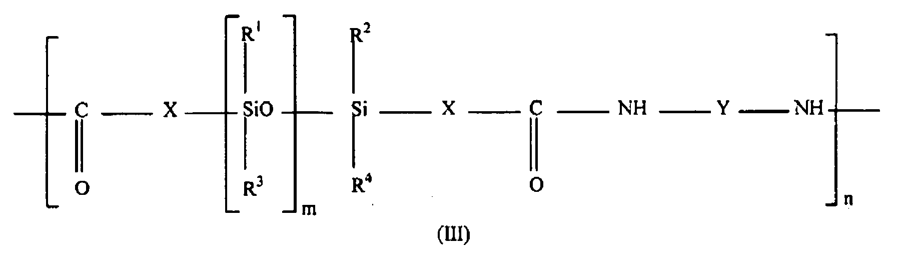 or 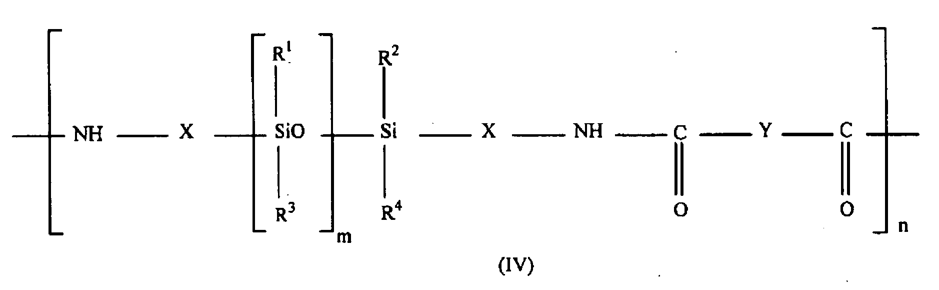 in which R¹, R², R³, R⁴, X, Y, m and n are as defined in Claim 12.

16. Composition according to either of Claims 12 and 15, in which X and/or Y represent an alkylene group containing in its alkylene portion at least one of the following elements:
1) 1 to 5 amide, urea or carbamate groups,
2) a C₅ or C₆ cycloalkyl group, and
3) a phenylene group optionally substituted with 1 to 3 identical or different C₁ to C₃ alkyl groups,
it being possible for the alkyl groups X or Y to also be substituted with at least one element chosen from the group consisting of:
- a hydroxyl group,
- a C₃ to C₈ cycloalkyl group,
- one to three C₁ to C₄₀ alkyl groups,
- a phenyl group optionally substituted with one to three C₁ to C₃ alkyl groups,
- a C₁ to C₃ hydroxyalkyl group, and
- a C₁ to C₆ aminoalkyl group.

17. Composition according to any one of Claims 12 to 15, in which Y represents: 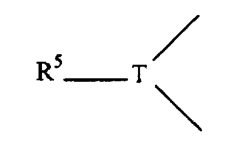 in which R⁵ represents a polyorganosiloxane chain and T represents a group of formula: 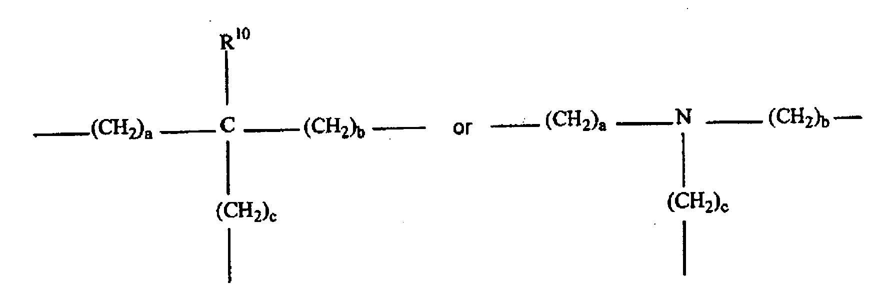 in which a, b and c are, independently, integers ranging from 1 to 10, and R¹⁰ is a hydrogen atom or a group such as those defined for R¹, R², R³ and R⁴, in Claim 12.

18. Composition according to any one of Claims 12 to 15, in which R¹, R², R³ and R⁴ represent, independently, a linear or branched C₁ to C₄₀ alkyl group, preferably a CH₃, C₂H₅, n-C₃H₇ or isopropyl group, a polyorganosiloxane chain or a phenyl group optionally substituted with one to three methyl or ethyl groups.

19. Composition according to any one of Claims 1 to 11, in which the polymer used in the gelling system comprises at least one moiety of formula: 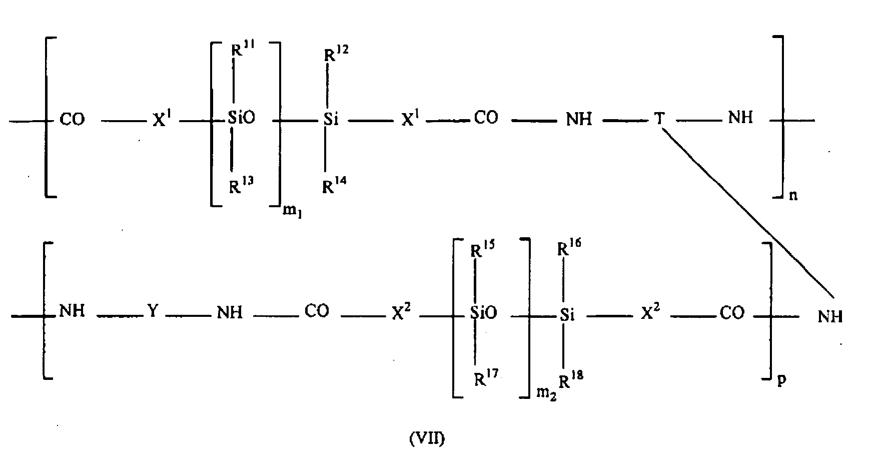 in which X¹ and X², which are identical or different, have the meaning given for X in Claim 12 n, Y and T are as defined in Claim 12 R¹¹ to R¹⁸ are groups chosen from the same group as R¹ to R⁴ in Claim 12 m₁ and m₂ are numbers in the range from 1 to 1 000, and p is an integer ranging from 2 to 500.

20. Composition according to the preceding Claim, in which:
- p is in the range from 1 to 25 and better still from 1 to 7,
- R¹¹ to R¹⁸ are methyl groups,
- T corresponds to one of the following formulae: 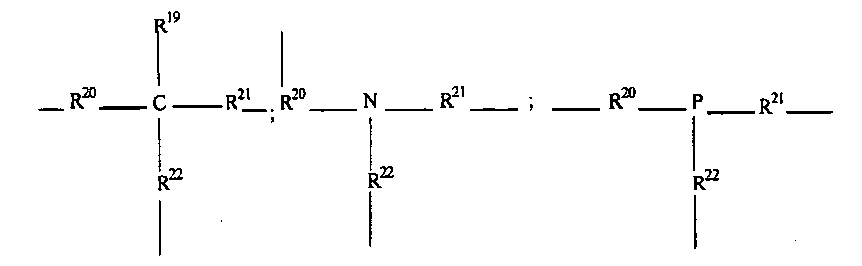 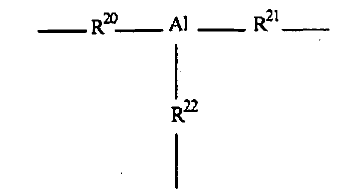 in which R¹⁹ is a hydrogen atom or a group chosen from the groups defined for R¹ to R⁴, and R²⁰, R²¹ and R²² are, independently, linear or branched alkylene groups, and more preferably corresponds to the formula: 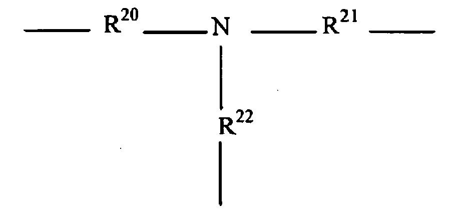 in particular with R²⁰, R²¹ and R²² representing -CH₂-CH₂-,
- m₁ and m₂ are in the range from 15 to 500 and better still from 15 to 45,
- X¹ and X² represent -(CH₂)₁₀-, and
- Y represents -CH₂-.

21. Composition according to any one of Claims 12 to 20, in which the polymer used in the gelling system further comprises a hydrocarbon-based moiety comprising two groups capable of establishing hydrogen interactions, chosen from ester, amide, sulphonamide, carbamate, thiocarbamate, urea, thiourea, oxamido, guanidino and biguanidino groups, and combinations thereof.

22. Composition according to the preceding Claim in which the copolymer is a block copolymer or a graft copolymer.

23. Composition according to any one of the preceding claims, in which the polymer represents from 0.5 to 80% of the total weight of the composition, preferably from 2 to 60% and even better from 5 to 40% of the total weight of the composition.

24. Composition according to any one of the preceding claims, in which the liquid fatty phase represents from 5 to 99% of the total weight of the composition and even better from 20 to 75% of the total weight of the composition.

25. Composition according to any one of the preceding Claims, in which the said organogelling agent is chosen from non-polymeric organic compounds whose molecules are capable of establishing, with each other, at least one physical interaction leading to self-aggregation of the said molecules with formation of a three-dimensional macromolecular network.

26. Composition according to the preceding Claim, in which the physical interaction(s) are chosen from self-complementary hydrogen interactions, interactions between unsaturated nuclei, dipolar interactions and coordination bonds with organometallic derivatives.

27. Composition according to any one of the preceding Claims, in which the organogelling agent is chosen from compounds whose molecules comprise at least one entity chosen from at least one group capable of establishing a hydrogen bond, at least one aromatic nucleus, at least one bond comprising an ethylenic unsaturation and at least one asymmetric carbon.

28. Composition according to any one of the preceding Claims, in which the organogelling agent is a compound whose molecules comprise at least two groups capable of establishing a hydrogen bond.

29. Composition according to the preceding Claim, in which the group capable of establishing a hydrogen bond is chosen from the hydroxyl, carbonyl, amine, carboxylic acid, amide, benzyl, sulphonamide, carbamate, thiocarbamate, urea, thiourea, oxamido, guanidino and biguanidino groups.

30. Composition according to Claim 1, in which the organogelling agent comprises at least one compound capable of gelling a silicone oil and possessing at least one group capable of establishing hydrogen interactions with the polymer of the gelling system, chosen from:
a) 12-hydroxystearic acid amide derivatives,
b) amides of tricarboxylic acids,
c) esters and amides of N-acylamino acids,
d) diureas of N-acylamino acids,
e) urethane amides of dipeptides,
f) cyclodipeptides chosen from cyclo(glycyl-L-alanyl), cyclo(glycyl-L-valyl), cyclo(glycyl-L-leucyl), cyclo(glycyl-L-phenylalanyl), cyclo(L-valyl-L-leucyl), cyclo(L-leucyl-L-leucyl), cyclo(L-phenylalanyl-L-leucyl), cyclo(L-phenylalanyl-L-phenylalanyl), cyclo(L-valyl-L-γ-3,7-ditnethyloctylglutamyl), cyclo(L-valyl-L-γ-2-ethylhexylglutamyl), cyclo(L-leucyl-L-γ-ethylglutamyl), cyclo(L-leucyl-L-γ-dodecylglutamyl), cyclo(L-leucyl-L-γ-3,7-dimethyloctylglutamyl), cyclo(L-leucyl-L-γ-benzylglutamyl), cyclo(L-β-butylasparaginyl-L-phenylalanyl), cyclo(L-γ-dodecylasparaginyl-L-phenylalanyl), cyclo(L-β-3,7-dimethyloctylasparaginyl-L-phenylalanyl), cyclo(L-β-2-ethylhexylasparaginyl-L-phenylalanyl), cyclo(L-β-3,5,5-trimethylhexylasparaginyl-L-phenylalanyl) and cyclo(L-(3-2-ethylbutylasparaginyl-L-phenylalanyl),
g) trans-(1R,2R)-bis(undecylcarbonylamino)cyclohexane of formula: 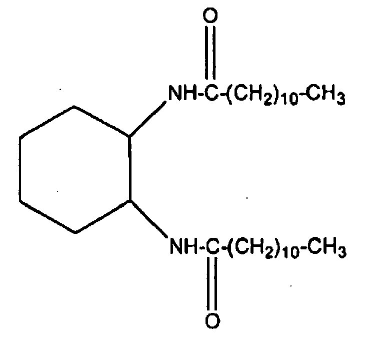
h) bolaamphiphilic amides derived from amino acids of formulae: 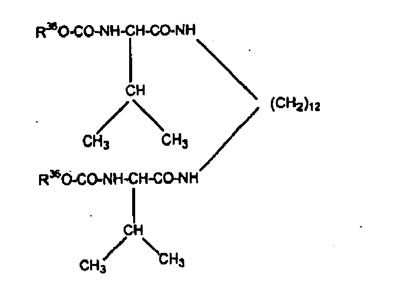 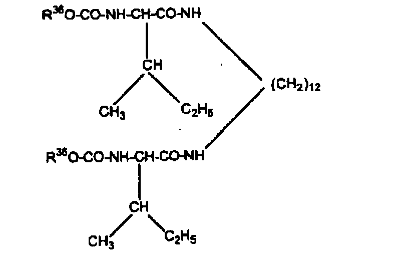 where R³⁶ = -CH₂-C₆H₅ or -CH₂-CH₃, and 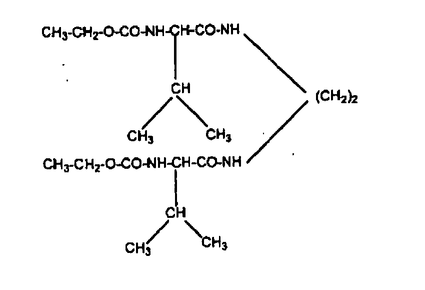
i) diamide derivatives of benzenedicarboxylic acid and of valine of formulae: 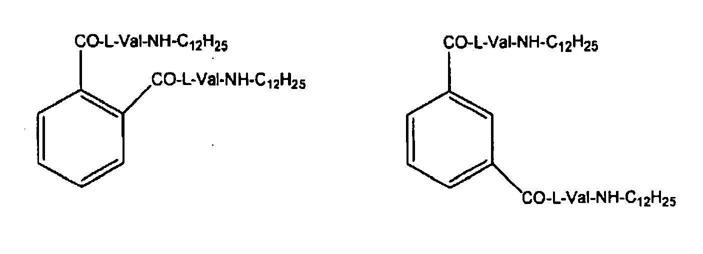 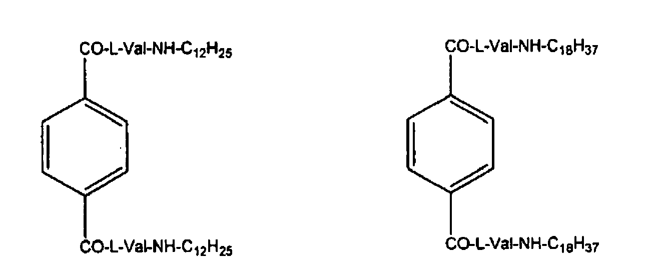 in which -L-Val- represents:
-NH-CH [CH(CH₃)₂)] -CO- ;
j) diamides of formula (XXV) or (XXVI):
R⁴⁴-X-CO-NH-R⁴⁵-NH-CO-X-R⁴⁴ (XXV)
or
R⁴⁴-CO-NH-R⁴⁵-NH-CO-R⁴⁴ (XXVI)
in which the groups R⁴⁴, which may be identical or different, represent a saturated or unsaturated, linear or branched C₆-C₆₀ hydrocarbon chain, the group(s) R⁴⁴ optionally comprising a hydroxyl group or at least one heteroatom such as N, O, S or Si, R⁴⁵ is a hydrocarbon-based group chosen from linear, branched and cyclic C₁ to C₅₀ groups and C₅ to C₈ arylene groups optionally substituted with one or more C₁-C₄ alkyl groups, and X represents -0- or NH-.

31. Composition according to any one of the preceding Claims in which the organogelling agent comprises at least one compound chosen from:
- the cis-trans mixture of N,N'-bis(dodecanoyl)- 1,2-diaminocyclohexane of formula: 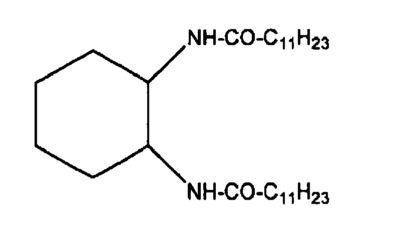
- the dibutylamide of N-laurylglutamic acid.

32. Composition according to any one of the preceding Claims in which the said organogelling agent is present in a quantity ranging from 0.1% to 80% by weight relative to the total weight of the composition, in particular from 0.5% to 60%, preferably from 1 to 40% and even better from 1 to 15% by weight relative to the total weight of the composition.

33. Composition according to any one of the preceding claims, in which the silicone polymer/non-polymeric organogelling agent mass ratio is in the range from 20 to 0.15, preferably from 15 to 1.5.

34. Composition according to any one of the preceding claims, **characterized in that** it comprises, in addition, at least one cosmetic or dermatological active agent.

35. Composition according to the preceding claim, **characterized in that** the active agent is chosen from essential oils, vitamins, moisturizers, sunscreens, cicatrizing agents and ceramides.

36. Composition according to any one of the preceding claims, **characterized in that** it comprises at least one additive chosen from dyes that are soluble in polyols or in the fatty phase, antioxidants, essential oils, preserving agents, perfumes, liposoluble polymers, especially hydrocarbon-based liposoluble polymers such as polyalkylenes or polyvinyl laurate, liquid-fatty-phase gelling agents, waxes, gums, resins, surfactants, for instance trioleyl phosphate, additional cosmetic or dermatological active agents chosen, for example, from the group consisting of water, emollients, moisturizers, vitamins, liquid lanolin, essential fatty acids, lipophilic sunscreens or sunscreens that are soluble in polyols, lipid vesicles, and mixtures thereof.

37. Composition according to any one of the preceding claims, **characterized in that** it contains, in addition, an amphiphilic compound which is liquid at room temperature, having a hydrophilic/lipophilic balance value of less than 12.

38. Composition according to any one of the preceding claims, **characterized in that** it additionally comprises colouring matter.

39. Composition according to any one of the preceding claims, **characterized in that** it is provided in the form of a rigid gel, and in particular of a transparent anhydrous stick.

40. A composition according to anyone of the preceding claims, **characterized in that** it has the form of a coloured make-up product for the skin, in particular a foundation, optionally having care or treatment properties, a blusher, a face powder, an eyeshadow, a concealer product, an eyeliner or a make-up product for the body; a lip make-up, for instance a lipstick, optionally having care or treatment properties ; a make-up for the superficial body growths, for instance the nails or the eyelashes, in particular in the form of a mascara cake, or for the eyebrows and the hair, especially in the form of a pencil.

41. Composition according to any one of Claims 1 to 39 **characterized in that** it is provided in the form of a cake mascara, an eyeliner, a foundation, a lipstick, a blusher, a make-up-removing, a make-up product for the body, an eyeshadow or a face powder, or a concealer product.

42. Cosmetic care, make-up or treatment method for the keratinous materials of human beings, comprising the application to the keratinous materials of a cosmetic composition in accordance with one of the preceding claims.

43. Cosmetic method for limiting the migration of a cosmetic composition containing a liquid fatty phase comprising at least one silicone oil, consisting in structuring the said fatty phase with a sufficient quantity of at least one polymer (homopolymer or copolymer) having a weight-average molecular mass ranging from 500 to 500 000, containing at least one moiety comprising:
- at least one polyorganosiloxane group, consisting of 1 to 1 000 organosiloxane units in the chain of the moiety or in the form of a graft, and
- at least two groups capable of establishing hydrogen interactions, said group being amide,
the polymer being solid at room temperature and soluble in the liquid fatty phase at a temperature of 25 to 250°C,
the liquid fatty phase comprising at least one volatile silicone oil and at least one volatile non silicone oil and further containing a non volatile non silicone oil and containing at least one organogelling agent, the organogelling agent being such as defined in claim 1, 30 or 31.

## Patentansprüche

1. Kosmetische Pflege- und/oder Make-up-Zusammensetzung, umfassend eine flüssige Fettphase, umfassend mindestens ein Silikonöl, strukturiert mit einem Geliersystem, umfassend:
1) mindestens ein Polymer (Homopolymer oder Copolymer) mit einer gewichtsmittleren Molekularmasse im Bereich von 500 bis 500.000, enthaltend mindestens einen Anteil, umfassend:
- mindestens einen Polyorganosiloxanrest, bestehend aus 1 bis 1.000 Organosiloxaneinheiten in der Kette des Anteils oder in der Form einer Pfropfung, und
- mindestens zwei Gruppen, welche zur Etablierung von Wasserstoffwechselwirkungen in der Lage sind, wobei die Gruppe Amid ist,
wobei das Polymer bei Raumtemperatur fest ist und in der flüssigen Fettphase bei einer Temperatur von 25 bis 250°C löslich ist, und
2) mindestens ein nicht-polymeres Organo-Geliermittel,
wobei die flüssige Fettphase und das Geliersystem ein physiologisch verträgliches Medium bilden und die flüssige Fettphase mindestens ein flüchtiges Silikonöl und mindestens ein flüchtiges Nichtsilikonöl umfasst und ferner ein nicht flüchtiges Nichtsilikonöl enthält,
wobei das Organo-Geliermittel mindestens eine Verbindung umfasst, welche zum Gelieren eines Silikonöls in der Lage ist, ausgewählt aus:
a) 12-Hydroxystearinsäure, ihren Salzen und ihren Ester- oder Amidderivaten,
b) Amiden von Tricarbonsäuren,
c) Estern und Amiden von N-Acylaminosäuren,
d) Diharnstoffen von N-Acylaminosäuren,
e) Urethanamiden von Dipeptiden,
f) Dibenzylidensorbitol und seinen Derivaten,
g) Sterolderivaten,
h) Cyclodipeptiden, ausgewählt aus Cyclo(glycyl-L-alanyl), Cyclo(glycyl-L-valyl), Cyclo(glycyl-L-leucyl), Cyclo(glycyl-L-phenylalanyl), Cyclo(L-valyl-L-leucyl), Cyclo(L-leucyl-L-leucyl), Cyclo(L-phenylalanyl-L-leucyl), Cyclo(L-phenylalanyl-L-phenylalanyl), Cyclo(L-valyl-L-γ-3,7-dimethyloctylglutamyl), Cyclo(L-valyl-L-γ-2-ethylhexylglutamyl), Cyclo(L-leucyl-L-γ-ethylglutamyl), Cyclo(L-leucyl-L-γ-dodecylglutamyl), Cyclo(L-leucyl-L-γ-3,7-dimethyloctyiglutamyl), Cyclo(L-leucyl-L-y-benzylglutamyl), Cyclo(L-β-butylasparaginyl-L-phenylalanyl), Cyclo(L-γ-dodecylasparaginyl-L-phenylalanyl), Cyclo(L-β-3,7-dimethyloctylasparaginyl-L-phenylalanyl), Cyclo(L-β-2-ethylhexylasparaginyl-L-phenylalanyl), Cyclo(L-β-3,5,5-trimethylhexylasparaginyl-L-phenylalanyl) und Cyclo(L-β-2-ethylbutylasparaginyl-L-phenylalanyl),
i) trans-(1R,2R)-Bis(undecylcarbonylamino)cyclohexan der Formel: 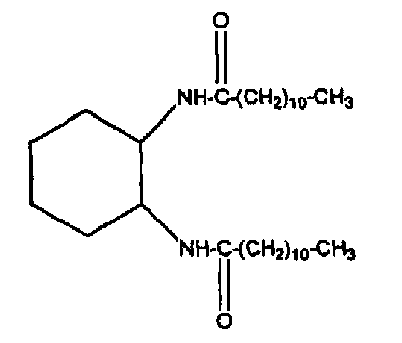
j) fluorierten Ethern,
k) Organo-Geliermitteln der Formel (XVII):
Q-O-W-(CHOH)ₛ-W¹-O-Q¹ (XVII)
wobei W und W¹, welche identisch oder unterschiedlich sein können, aus -CH₂-und -CO- ausgewählt sind und wobei Q und Q¹, welche identisch oder unterschiedlich sein können, eine Kohlenwasserstoffkette, welche aus gesättigten oder ungesättigten, linearen oder verzweigten Kohlenwasserstoffketten, die mindestens 6 Kohlenstoffatome enthalten, ausgewählt sind, sind und wobei s eine ganze Zahl von 2 bis 4 ist;
l) bolaamphiphilen Amiden, welche abgeleitet sind von Aminosäuren der Formeln: 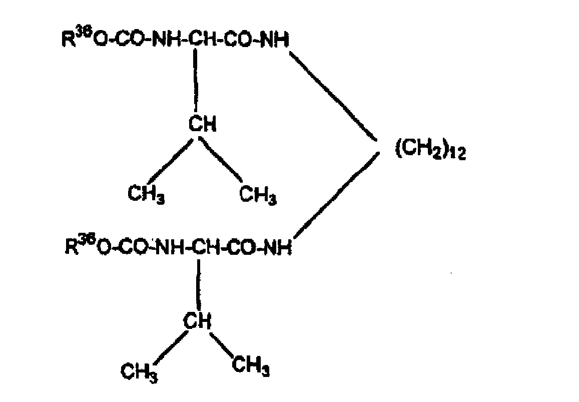 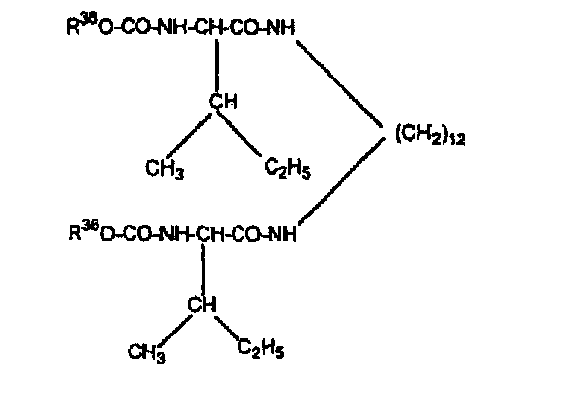 wobei R³⁶ = -CH₂-C₆H₅ oder -CH₂-CH₃ ist, und 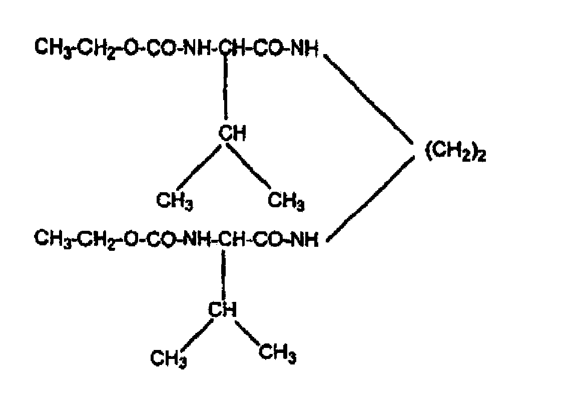
m) 2-Alkyl-2-ammoniumisobutylacetat-p-toluolsulfonatsalzen der Formel (XXII):
p-CH₃-C₆H₄-SO₃^{- +}H₃N-CH(R³⁷)-CO-OR³⁸ (XXII)
wobei R³⁷ darstellt:
-CH₂-CH-(CH₃)₂ (Leucin), -CH-(CH₃)₂ (L-Valin), 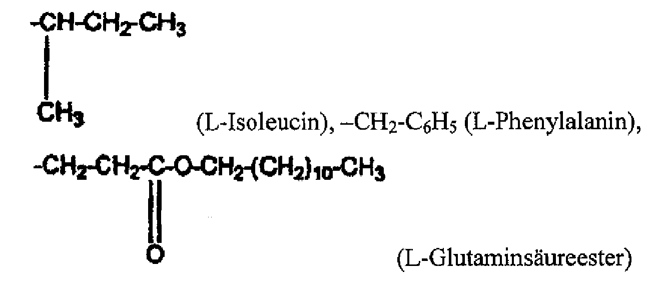
R³⁸ darstellt:
-CH₂-(CH₂)ₙ-CH₃ mit n = 4 bis 12 oder 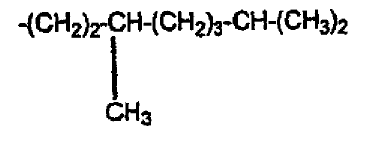
n) Diamidderivaten von Benzoldicarbonsäure und Valin der Formeln: 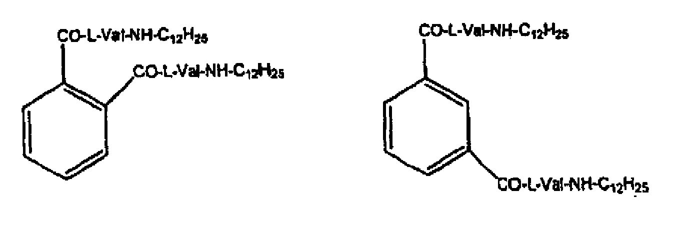 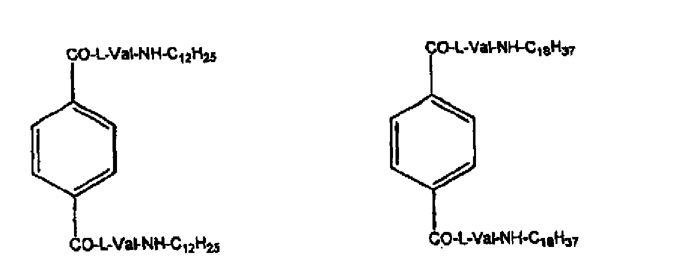 wobei -L-Val- darstellt:
-NH-CH [CH(CH₃)₂] -CO- ;
o) Diamiden der Formel (XXV) oder (XXVI):
R⁴⁴-X-CO-NH-R⁴⁵-NH-CO-X-R⁴⁴ (XXV)
oder
R⁴⁴-CO-NH-R⁴⁵-NH-CO-R⁴⁴ (XXVI)
wobei die Reste R⁴⁴, welche identisch oder unterschiedlich sein können, eine gesättigte oder ungesättigte, lineare oder verzweigte C₈-C₆₀-Kohlenwasserstoffkette darstellen, wobei der bzw. die Rest(e) R⁴⁴ gegebenenfalls eine Hydroxylgruppe oder mindestens ein Heteroatom wie N, O, S oder Si umfasst bzw. umfassen, R^{4s} ein Rest auf Kohlenwasserstoff-Basis, ausgewählt aus linearen, verzweigten und cyclischen C₁- bis C₅₀-Resten und C₅- bis C₈-Arylenresten, gegebenenfalls substituiert mit einem oder mehr C₁-C₄-Alkylresten, ist, und X -0- oder -NH- darstellt.

2. Zusammensetzung gemäß Anspruch 1, wobei das flüchtige Silikonöl einen Flammpunkt von gleich oder höher als 40°C und vorteilhafterweise höher als der Erweichungspunkt des Geliersystems aufweist.

3. Zusammensetzung gemäß Anspruch 1 oder 2, wobei das flüchtige Silikonöl aus der Gruppe ausgewählt ist, welche aus den folgenden Verbindungen besteht: Octyltrimethicon, Hexyltrimethicon, Octamethylcyclotetrasiloxan D4, Dodecamethylcyclohexasiloxan D6, Heptamethyloctyltrisiloxan, Decamethyltetrasiloxan, Dodecamethylpentasiloxan, Polydimethylsiloxan mit 1,5 cSt, Polydimethylsiloxan mit 2 cSt, Polydimethylsiloxan mit 3 cSt, Polydimethylsiloxan mit 5 cSt und Gemischen davon.

4. Zusammensetzung gemäß einem der vorhergehenden Ansprüche, wobei das flüchtige Öl einen Flammpunkt von 40 bis 135°C aufweist.

5. Zusammensetzung gemäß einem der vorhergehenden Ansprüche, wobei die flüssige Fettphase mindestens 30 Gew.-% und noch besser mindestens 40 Gew.-% Silikonöl enthält.

6. Zusammensetzung gemäß einem der vorhergehenden Ansprüche, wobei das flüchtige Öl3 bis 89,4 % des Gesamtgewichts der Zusammensetzung darstellt.

7. Zusammensetzung gemäß einem der vorhergehenden Ansprüche, umfassend zusätzlich feste Teilchen, welche aus Füllstoffe, Pigmenten und Gemischen davon ausgewählt sind.

8. Zusammensetzung gemäß Anspruch 7, wobei die festen Teilchen hydrophobe Teilchen sind.

9. Zusammensetzung gemäß dem vorhergehenden Anspruch, wobei die festen Teilchen hydrophile Teilchen sind, welche mit einem Film einer hydrophoben Verbindung überzogen.

10. Zusammensetzung gemäß Anspruch 7, wobei die festen Teilchen hydrophile Teilchen sind und die Zusammensetzung ferner ein amphiphiles Silikon umfasst.

11. Zusammensetzung gemäß einem der Ansprüche 7 bis 10, wobei die Teilchen Pigmente, ausgewählt aus Zinkoxiden, Eisenoxiden, Titanoxiden und Gemischen davon, sind.

12. Zusammensetzung gemäß einem der vorhergehenden Ansprüche, wobei das Polymer, welches in dem Geliersystem verwendet wird, mindestens einen Anteil, der der folgenden Formel entspricht, umfasst: 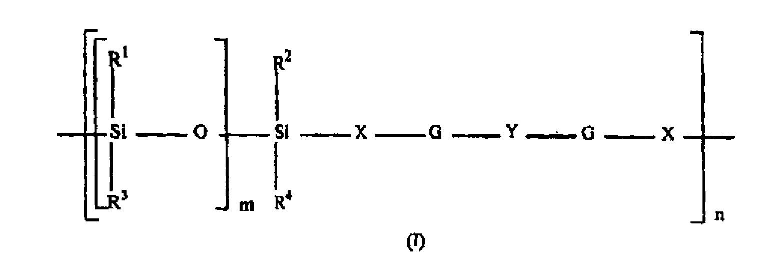 wobei:
1) R¹, R², R³ und R⁴, welche identisch oder unterschiedlich sein können, einen Rest darstellen, welcher ausgewählt ist aus:
- linearen, verzweigten oder cyclischen, gesättigten oder ungesättigten C₁- bis C₄₀-Resten auf Kohlenwasserstoffbasis, welche möglicherweise in ihrer Kette ein oder mehr Sauerstoff-, Schwefel- und/oder Stickstoffatome enthalten und möglicherweise teilweise oder vollständig substituiert sind mit Fluoratomen,
- C₆- bis C₁₀-Arylresten, welche gegebenenfalls substituiert sind mit einem oder mehreren C₁- bis C₄-Alkylresten,
- Polyorganosiloxanketten, welche möglicherweise ein oder mehrere Sauerstoff-, Schwefel- und/oder Stickstoffatome enthalten;
2) die Reste X, welche identisch oder unterschiedlich sein können, einen linearen oder verzweigten C₁- bis C₃₀-Alkylendiylrest, welcher möglicherweise in seiner Kette ein oder mehrere Sauerstoff-, und/oder Stickstoffatome enthält, darstellen;
3) Y ein gesättigter oder ungesättigter C₁- bis C₅₀-linearer oder verzweigter zweiwertiger Alkylen-, Arylen-, Cycloalkylen-, Alkylarylen- oder Arylalkylenrest ist, möglicherweise umfassend ein oder mehrere Sauerstoff-, Schwefel- und/oder Stickstoffatome und/oder tragend als einen Substituenten ein bzw. eine der folgenden Atome oder Atomgruppen:
Fluor, Hydroxyl, C₃- bis C₈-Cycloalkyl, C₁- bis C₄₀-Alkyl, C₅- bis C₁₀-Aryl, Phenyl, gegebenenfalls substituiert mit 1 bis 3 C₁- bis C₃-Alkylresten, C₁- bis C₃-Hydroxyalkyl und C₁- bis C₆-Aminoalkyl, oder
4) Y einen Rest, welcher der folgenden Formel entspricht, darstellt: 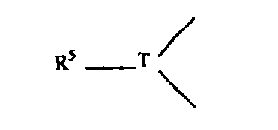 wobei
- T einen linearen oder verzweigten, gesättigten oder ungesättigten C₃- bis C₂₄-dreiwertigen oder vierwertigen Rest auf Kohlenwasserstoffbasis, gegebenenfalls substituiert mit einer Polyorganosiloxankette und möglicherweise enthaltend ein oder mehrere Atome, welche aus O, N und S ausgewählt sind, darstellt oder T ein dreiwertiges Atom, welches aus N, P und Al ausgewählt ist, darstellt, und
- R⁵ einen linearen oder verzweigten C₁- bis C₅₀-Alkylrest oder eine Polyorganosiloxankette, möglicherweise umfassend einen oder mehrere Ester-, Amid-, Urethan-, Thiocarbamat-, Harnstoff-, Thioharnstoff und/oder Sulfonamidreste, welche möglicherweise mit einer anderen Kette des Polymers verbunden sein können, darstellt;
5) die Reste G, welche identisch oder unterschiedlich sein können, zweiwertige Reste der folgenden Formel darstellen: 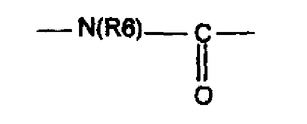 wobei R⁶ ein Wasserstoffatom oder einen linearen oder verzweigten C₁- bis C₂₀-Alkylrest darstellt, mit der Bedingung, dass mindestens 50 % der Reste R⁶ des Polymers ein Wasserstoffatom darstellen und dass mindestens zwei der Reste G des Polymers ein Rest sind, welcher verschieden ist von: 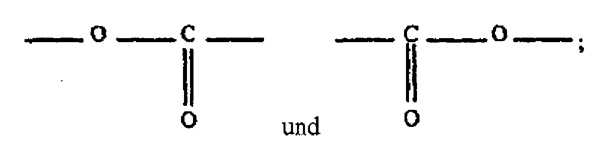
6) n eine ganze Zahl im Bereich von 2 bis 500 und bevorzugt von 2 bis 200 ist und m eine ganze Zahl im Bereich von 1 bis 1.000, bevorzugt von 1 bis 700 und noch besser von 6 bis 200 ist.

13. Zusammensetzung gemäß dem vorhergehenden Anspruch, wobei Y einen Rest darstellt, welcher ausgewählt ist aus:
a) linearen C₁- bis C₂₀- und bevorzugt C₁- bis C₁₀-Alkylenresten,
b) C₃₀- bis C₅₆- verzweigten Alkylenresten, welche möglicherweise Ringe und nicht konjugierte Ungesättigtheiten umfassen,
c) C₅-C₆-Cycloalkylentesten,
d) Phenylenresten, welche gegebenenfalls substituiert sind mit einem oder mehreren C₁- bis C₄₀-Alkylresten,
e) C₁- bis C₂₀-Alkylenresten, welche 1 bis 5 Amidgruppen umfassen,
f) C₁- bis C₂₀-Alkylenresten, welche einen oder mehrere Substituenten, ausgewählt aus Hydroxyl-, C₃- bis C₈-Cycloalkan-, C₁- bis C₃-Hydroxyalkyl- und C₁- bis C₆-Alkylaminresten, umfassen,
g) Polyorganosiloxanketten der Formel: 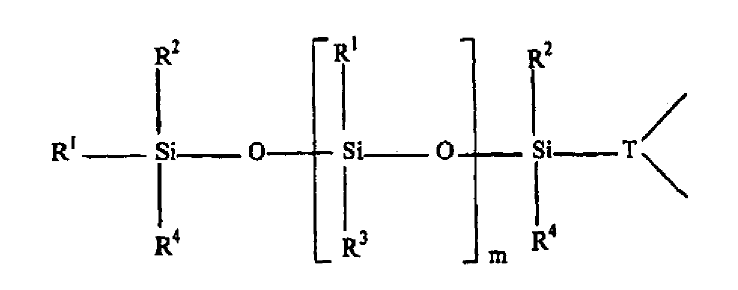 wobei R¹, R², R³, R⁴, T und m wie vorstehend definiert sind.

14. Zusammensetzung gemäß einem der Ansprüche 1 bis 11, wobei das Polymer, welches in dem Geliersystem verwendet wird, mindestens einen Anteil, welcher der folgenden Formel (II) entspricht, umfasst: 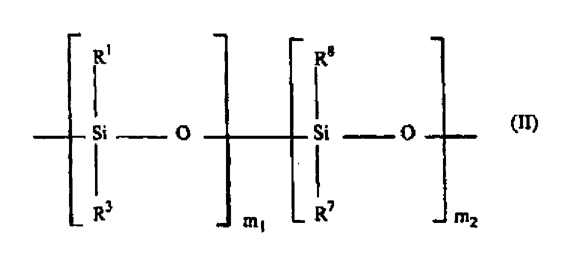 wobei
- R¹ und R³, welche identisch oder unterschiedlich sein können, wie vorstehend für Formel (I) in Anspruch 12 definiert sein können,
- R⁷ einen Rest, wie vorstehend für R¹ und R³ definiert, darstellt oder den Rest der Formel -X-G-R⁹ darstellt, wobei X und G wie vorstehend für Formel (I) in Anspruch 12 definiert sind und R⁹ ein Wasserstoffatom oder einen linearen, verzweigten oder cyclischen, gesättigten oder ungesättigten C₁- bis C₅₀-Rest auf Kohlenwasserstoffbasis, gegebenenfalls umfassend in seiner Kette ein oder mehrere Atome, welche ausgewählt sind aus O, S und N, gegebenenfalls substituiert mit einem oder mehreren Fluoratomen und/oder einer oder mehreren Hydroxylgruppen, oder ein Phenylrest, welcher gegebenenfalls mit einem oder mehreren C₁- bis C₄-Alkylresten substituiert ist, darstellt,
- R⁸ den Rest der Formel -X-G-R⁹ darstellt, wobei X, G und R⁹ wie vorstehend definiert sind,
- m₁ eine ganze Zahl im Bereich von 1 bis 998 ist, und
- m₂ eine ganze Zahl im Bereich von 2 bis 500 ist.

15. Zusammensetzung gemäß Anspruch 12, wobei das Polymer mindestens einen Anteil der Formel (III) oder (IV) umfasst: 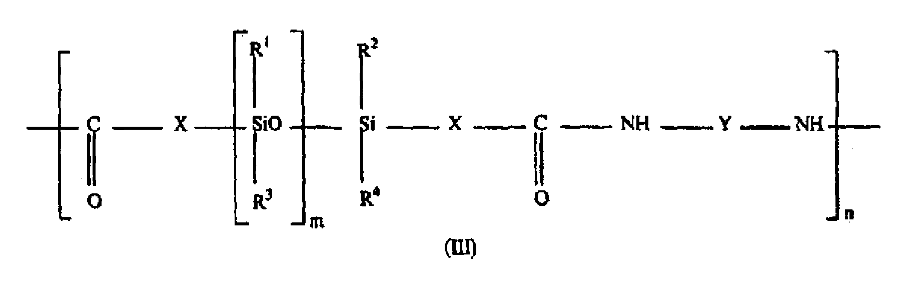 oder 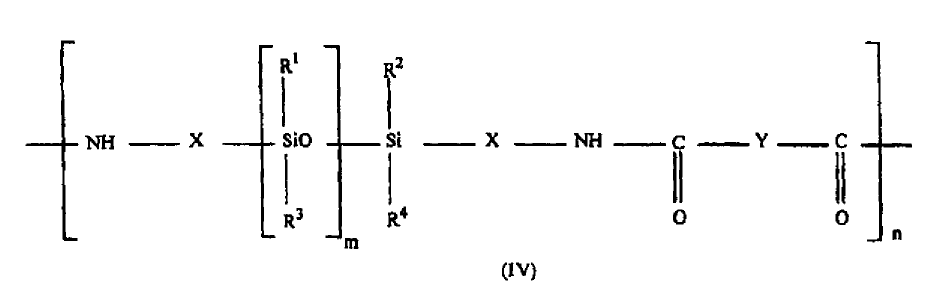 wobei R¹, R², R³, R⁴, X, Y, m und n wie in Anspruch 12 definiert sind.

16. Zusammensetzung gemäß einem der Ansprüche 12 und 15, wobei X und/oder Y einen Alkylenrest darstellen, welcher in seinem Alkylenteil mindestens eines der folgenden Elemente enthält:
1) 1 bis 5 Amid-, Harnstoff oder Carbamatreste,
2) einen C₅- oder C₆-Cycloalkylrest, und
3) einen Phenylenrest, welcher gegebenenfalls mit 1 bis 3 identischen oder unterschiedlichen C₁- bis C₃-Alkylresten substituiert ist,
wobei es möglich ist, dass die Alkylreste X oder Y auch mit mindestens einem Element substituiert sind, welches aus der Gruppe ausgewählt ist, die besteht aus:
- einer Hydroxylgruppe,
- einem C₃- bis C₈-Cycloalkylrest,
- einem bis drei C₁- bis C₄₀-Alkylresten,
- einer Phenylgruppe, welche gegebenenfalls mit einem bis drei C₁- bis C₃-Alkylresten substituiert ist,
- einem C₁- bis C₃-Hydroxyalkylrest, und
- einem C₁- bis C₆-Ammoalkylrest.

17. Zusammensetzung gemäß einem der Ansprüche 12 bis 15, wobei Y darstellt: 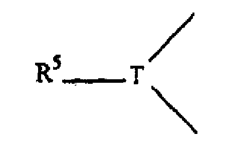 wobei R⁵ eine Polyorganosiloxankette darstellt und T einen Rest der folgenden Formel darstellt: 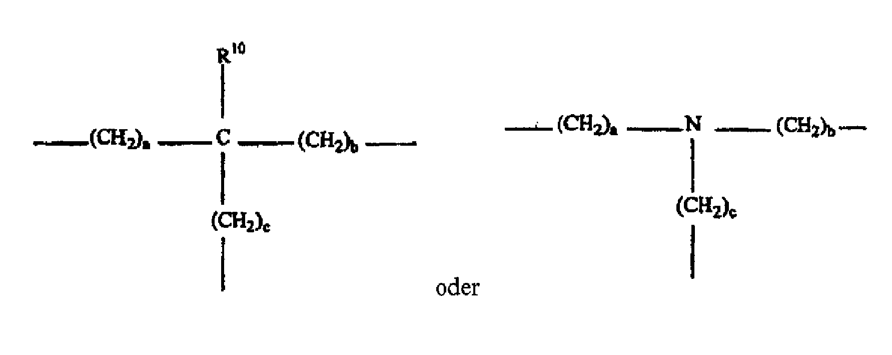 wobei a, b und c unabhängig voneinander ganze Zahlen im Bereich von 1 bis 10 sind und R¹⁰ ein Wasserstoffatom oder ein Rest wie jene, welche für R¹, R², R³ und R⁴ in Anspruch 12 definiert sind, ist.

18. Zusammensetzung gemäß einem der Ansprüche 12 bis 15, wobei R¹, R², R³ und R⁴ unabhängig voneinander einen linearen oder verzweigten C₁- bis C₄₀-Alkylrest, bevorzugt eine CH₃-, C₂H₅-, n-C₃H₇- oder Isopropylgruppe, eine Polyorganosiloxankette oder eine Phenylgruppe, gegebenenfalls substituiert mit ein bis drei Methyl- oder E-thylgruppen, darstellen.

19. Zusammensetzung gemäß einem der Ansprüche 1 bis 11, wobei das Polymer, welches in dem Geliersystem verwendet wird, mindestens einen Anteil der folgenden Formel umfasst: 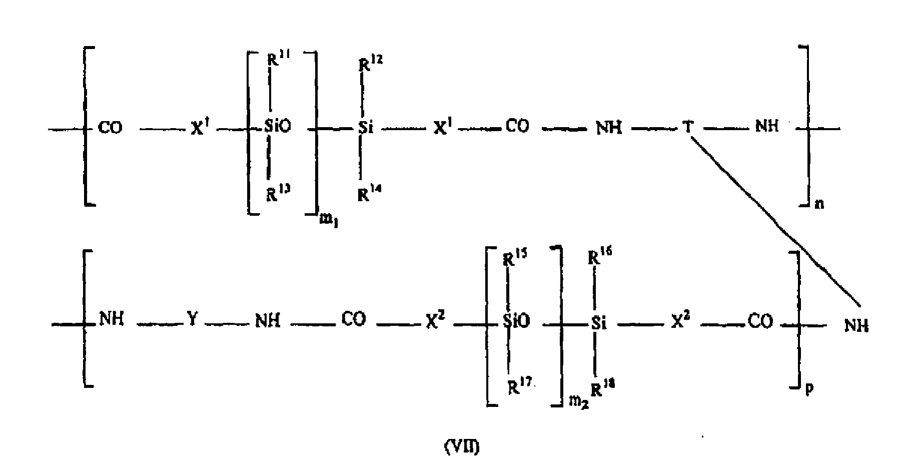 wobei X¹ und X², welche identisch oder unterschiedlich sind, die Bedeutung haben, welche für X in Anspruch 12 gegeben ist, n, Y und T wie in Anspruch 12 definiert sind, R¹¹ bis R¹⁸ Reste sind, welche aus der gleichen Gruppe wie R¹ bis R⁴ in Anspruch 12 ausgewählt sind, m₁ und m₂ Zahlen im Bereich von 1 bis 1.000 sind und p eine ganze Zahl im Bereich von 2 bis 500 ist.

20. Zusammensetzung gemäß dem vorhergehenden Anspruch, wobei:
- p im Bereich von 1 bis 25 und noch besser von 1 bis 7 liegt,
- R¹¹ bis R¹⁸ Methylgruppen sind,
- T einer der folgenden Formeln entspricht: 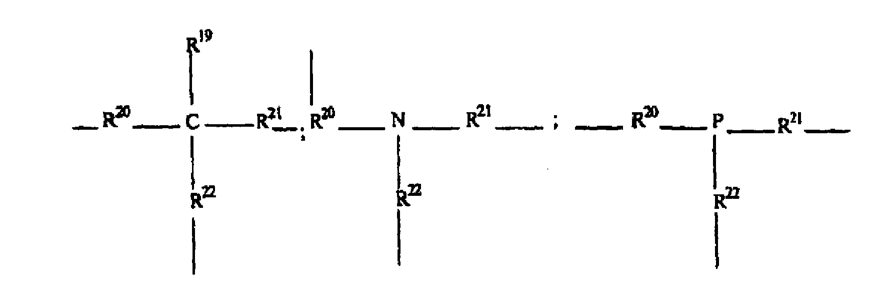 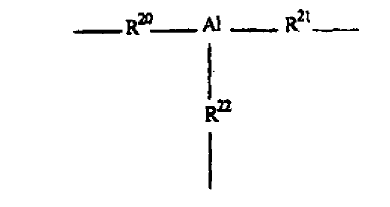 wobei R¹⁹ ein Wasserstoffatom oder ein Rest, welcher aus den Resten ausgewählt ist, die für R¹ bis R⁴ definiert sind, ist und R²⁰, R²¹ und R²² unabhängig voneinander lineare oder verzweigte Alkylenreste sind oder stärker bevorzugt der folgenden Formel entsprechen: 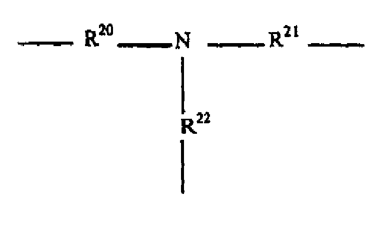 wobei R²⁰, R²¹ und R²² insbesondere -CH₂-CH₂- darstellen,
- m₁ und m₂ im Bereich von 15 bis 500 und noch besser von 15 bis 45 liegen,
- X¹ und X² -(CH₂)₁₀- darstellen und
- Y -CH₂- darstellt.

21. Zusammensetzung gemäß einem der Ansprüche 12 bis 20, wobei das Polymer, welches in dem Geliersystem verwendet wird, ferner einen Anteil auf Kohlenwasserstoff basis, umfassend zwei Gruppen, welche zur Etablierung von Wasserstoffwechselwirkungen in der Lage sind, ausgewählt aus Ester-, Amid-, Sulfonamid-, Carbamat-, Thiocarbamat-, Harnstoff-, Thioharnstoff-, Oxamido-, Guanidino- und Biguanidinogruppen und Kombinationen davon, umfasst.

22. Zusammensetzung gemäß dem vorhergehenden Anspruch, wobei das Copolymer ein Blockcopolymer oder ein Pfropfcopolymer ist.

23. Zusammensetzung gemäß einem der vorhergehenden Ansprüche, wobei das Polymer 0,5 bis 80 % des Gesamtgewichts der Zusammensetzung, bevorzugt 2 bis 60 % und noch besser 5 bis 40 % des Gesamtgewichts der Zusammensetzung darstellt.

24. Zusammensetzung gemäß einem der vorhergehenden Ansprüche, wobei die flüssige Fettphase 5 bis 99 % des Gesamtgewichts der Zusammensetzung und noch besser 20 bis 75 % des Gesamtgewichts der Zusammensetzung darstellt.

25. Zusammensetzung gemäß einem der vorhergehenden Ansprüche, wobei das Organo-Geliermittel ausgewählt ist aus nicht-polymeren organischen Verbindungen, deren Moleküle in der Lage sind, miteinander mindestens eine physikalische Wechselwirkung zu etablieren, welche zu Selbstaggregation der Moleküle führt, mit der Bildung eines dreidimensionalen makromolekularen Netzwerks.

26. Zusammensetzung gemäß dem vorhergehenden Anspruch, wobei die physikalische(n) Wechselwirkung(en) ausgewählt ist bzw. sind aus selbstkomplementären Wasserstoffwechselwirkungen, Wechselwirkungen zwischen ungesättigten Kernen, dipolaren Wechselwirkungen und Koordinationsbindungen mit organometallischen Derivaten.

27. Zusammensetzung gemäß einem der vorhergehenden Ansprüche, wobei das Organo-Geliermittel ausgewählt ist aus Verbindungen, deren Moleküle mindestens eine Einheit umfassen, welche ausgewählt ist aus mindestens einer Gruppe, welche zur Etablierung einer Wasserstoffbrückenbindung in der Lage ist, mindestens einem aromatischen Kern, mindestens einer Bindung, umfassend eine ethylenische Ungesättigtheit und mindestens einen asymmetrischen Kohlenstoff.

28. Zusammensetzung gemäß einem der vorhergehenden Ansprüche, wobei das Organo-Geliermittel eine Verbindung ist, deren Moleküle mindestens zwei Gruppen umfassen, welche zur Etablierung einer Wasserstoffbrückenbindung in der Lage sind.

29. Zusammensetzung gemäß dem vorhergehenden Anspruch, wobei die Gruppe, welche zur Etablierung einer Wasserstoffbrückenbindung in der Lage ist, aus den Hydroxyl-, Carbonyl-, Amin-, Carbonsäure-, Amid-, Benzyl-, Sulfonamid-, Carbamat-, Thiocarbamat-, Harnstoff-, Thioharnstoff-, Oxamido-, Guanidino- und Biguanidinoresten ausgewählt ist.

30. Zusammensetzung gemäß Anspruch 1, wobei das Organo-Geliermittel mindestens eine Verbindung umfasst, welche zum Gelieren eines Silikonöls in der Lage ist und welche mindestens eine Gruppe aufweist, welche zur Etablierung von Wasserstoffwechselwirkungen mit dem Polymer des Geliersystems in der Lage ist, ausgewählt aus:
a) 12-Hydroxystearinsäureamidderivaten,
b) Amiden von Tricarbonsäuren,
c) Estern und Amiden von N-Acylaminosäuren,
d) Diharnstoffen von N-Acylaminosäuren,
e) Urethanamiden von Dipeptiden,
f) Cyclodipeptiden, ausgewählt aus Cyclo(glycyl-L-alanyl), Cyclo(glycyl-L-valyl), Cyclo(glycyl-L-leucyl), Cyclo(glycyl-L-phenylalanyl), Cyclo(L-valyl-L-leucyl), Cyclo(L-leucyl-L-leucyl), Cyclo(L-phenylalanyl-L-leucyl), Cyclo(L-phenylalanyl-L-phenylalanyl), Cyclo(L-valyl-L-γ-3,7-dimethyloctylglutamyl), Cyclo(L-valyl-L-γ-2-ethylhexylglutamyl), Cyclo(L-leucyl-L-γ-ethylglutamyl), Cyclo(L-leucyl-L-γ-dodecylglutamyl), Cyclo(L-leucyl-L-γ-3,7-dimethyloctylglutamyl), Cyclo(L-leucyl-L-γ-benzylglutamyl), Cyclo(L-β-butylasparaginyl-L-phenylalanyl), Cyclo(L-γ-dodecylasparaginyl-L-phenylalanyl), Cyclo(L-β-3,7-dimethyloctylasparaginyl-L-phenylalanyl), Cyclo(L-β-2-ethylhexylasparaginyl-L-phenylalanyl), Cyclo(L-β-3,5,5-trimethylhexylasparaginyl-L-phenylalanyl) und Cyclo(L-β-2-ethylbutylasparaginyl-L-phenylalanyl),
g) trans-(1R,2R)-Bis(undecylcarbonylamino)cyclohexan der Formel: 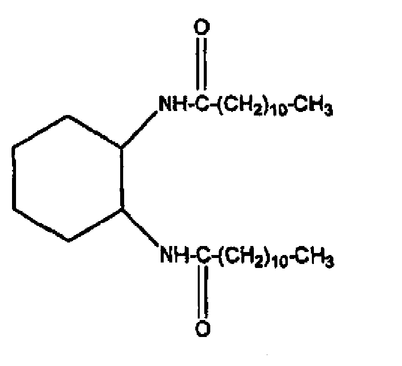
h) bolaamphiphilen Amiden, welche abgeleitet sind von Aminosäuren der Formeln: 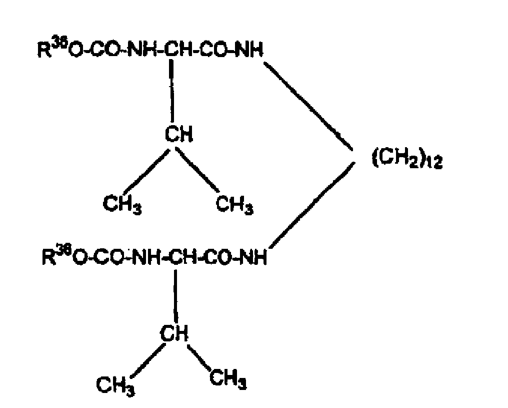 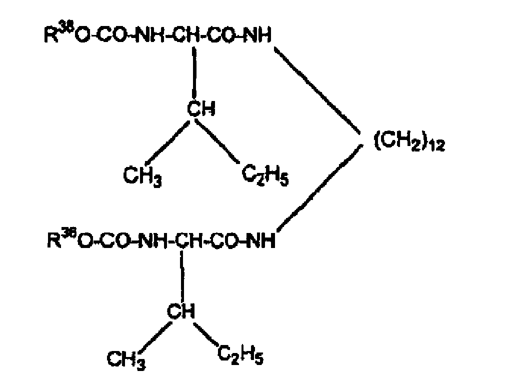 wobei R³⁶ = -CH₂-C₆H₅ oder -CH₂-CH₃ ist, und 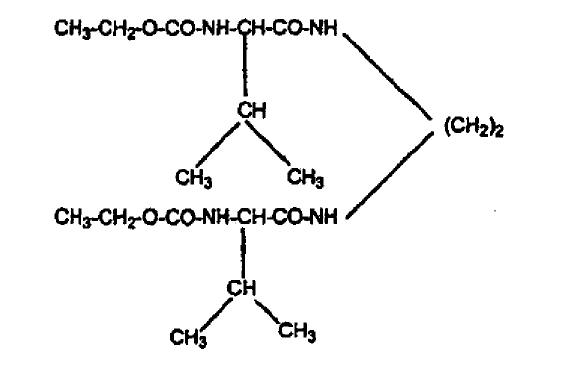
i) Diamidderivaten von Benzoldicarbonsäure und Valin der Formeln: 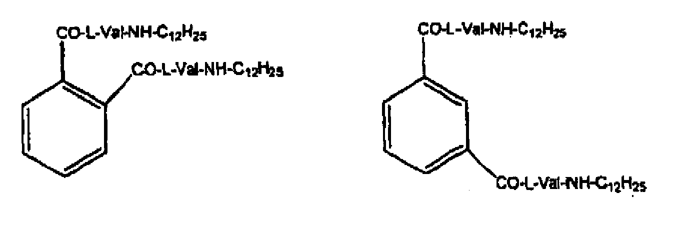 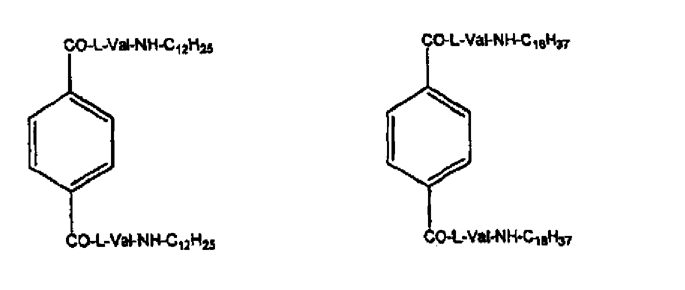 wobei -L-Val- darstellt:
-NH-CH [CH(CH₃)₂] -CO-;
j) Diamiden der Formel (XXV) oder (XXVI):
R⁴⁴-X-CO-NH-R⁴⁵-NH-CO-X-R⁴⁴ (XXV)
oder
R⁴⁴-CO-NH-R⁴⁵-NH-CO-R⁴⁴ (XXVI)
wobei die Reste R⁴⁴, welche identisch oder unterschiedlich sein können, eine gesättigte oder ungesättigte, lineare oder verzweigte C₆-C₆₀-Kohlenwasserstoffkette darstellen, wobei der bzw. die Rest(e) R⁴⁴ gegebenenfalls eine Hydroxylgruppe oder mindestens ein Heteroatom wie N, O, S oder Si umfasst bzw. umfassen, R⁴⁵ ein Rest auf Kohlenwasserstoff-Basis, ausgewählt aus linearen, verzweigten und cyclischen C₁- bis C₅₀-Resten und C₅- bis C₈-Arylenresten, gegebenenfalls substituiert mit einem oder mehr C₁-C₄-Alkylresten, ist, und X -0- oder NH- darstellt.

31. Zusammensetzung gemäß einem der vorhergehenden Ansprüche, wobei das Organo-Geliermittel mindestens eine Verbindung umfasst, welche ausgewählt ist aus:
- dem cis-trans-Gemisch von N,N'-Bis(dodecanoyl)-1,2-diaminocyclohexan der Formel: 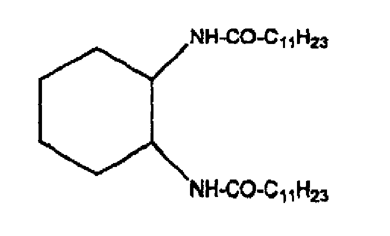
- dem Dibutylamid von N-Laurylglutaminsäure.

32. Zusammensetzung gemäß einem der vorhergehenden Ansprüche, wobei das Organo-Geliermittel in einer Menge im Bereich von 0,1 Gew.-% bis 80 Gew.-%, relativ zum Gesamtgewicht der Zusammensetzung, insbesondere von 0,5 Gew.-% bis 60 Gew.-%, bevorzugt von 1 bis 40 Gew.-% und noch besser von 1 bis 15 Gew.-%, relativ zum Gesamtgewicht der Zusammensetzung, vorhanden ist.

33. Zusammensetzung gemäß einem der vorhergehenden Ansprüche, wobei das Silikonpolymer/nicht-polymeres Organo-Geliermittel-Massenverhältnis im Bereich von 20 bis 0,15, bevorzugt von 15 bis 1,5 liegt.

34. Zusammensetzung gemäß einem der vorhergehenden Ansprüche, **dadurch gekennzeichnet, dass** sie zusätzlich mindestens einen kosmetischen oder dermatologischen Wirkstoff umfasst.

35. Zusammensetzung gemäß dem vorhergehenden Anspruch, **dadurch gekennzeichnet, dass** der Wirkstoff aus ätherischen Ölen, Vitaminen, Feuchthaltemitteln, Sonnenschutzmitteln, Narbenmitteln und Ceramiden ausgewählt ist.

36. Zusammensetzung gemäß einem der vorhergehenden Ansprüche, **dadurch gekennzeichnet, dass** sie mindestens ein Additiv umfasst, welches ausgewählt ist aus Farbstoffen, die in Polyolen oder in der Fettphase löslich sind, Antioxidantien, ätherischen Ölen, Konservierungsstoffen, Geruchsstoffen, fettlöslichen Polymeren, insbesondere fettlöslichen Polymeren auf Kohlenwasserstoffbasis wie Polyalkylene oder Polyvinyllaurat, Mitteln zum Gelieren einer flüssigen Fettphase, Wachsen, Gumme, Harzen, grenzflächenaktiven Mitteln, zum Beispiel Trioleylphosphat, zusätzlichen kosmetischen oder dermatologischen Wirkstoffen, welche zum Beispiel ausgewählt sind aus der Gruppe, welche aus Wasser, Emolliensen, Feuchthaltemitteln, Vitaminen, flüssigem Lanolin, essentiellen Fettsäuren, lipophilen Sonnenschutzmitteln oder Sonnenschutzmitteln, welche in Polyolen, Lipidvesikeln und Gemischen davon löslich sind, besteht.

37. Zusammensetzung gemäß einem der vorhergehenden Ansprüche, **dadurch gekennzeichnet, dass** sie zusätzlich eine amphiphile Verbindung, welche bei Raumtemperatur flüssig ist, mit einem Hydrophilie/Lipophilie-Gleichgewichtswert von niedriger als 12 enthält.

38. Zusammensetzung gemäß einem der vorhergehenden Ansprüche, **dadurch gekennzeichnet, dass** sie zusätzlich farbgebendes Material umfasst.

39. Zusammensetzung gemäß einem der vorhergehenden Ansprüche, **dadurch gekennzeichnet, dass** sie in der Form eines steifen Gels und insbesondere eines transparenten wasserfreien Stifts bereitgestellt wird.

40. Zusammensetzung gemäß einem der vorhergehenden Ansprüche, **dadurch gekennzeichnet, dass** sie die Form eines gefärbten Make-up-Produkts für die Haut, insbesondere einer Grundlage, gegebenenfalls mit Pflege- oder Behandlungseigenschaften, eines Rouge, eines Gesichtspuders, eines Lidschattens, eines Abdeckprodukts, eines Eyeliners oder eines Make-up-Produkts für den Körper; eines Lippen-Make-up, zum Beispiel eines Lippenstifts, gegebenenfalls mit Pflege- oder Behandlungseigenschaften; eines Make-up für das oberflächliche Körperwachstum, zum Beispiel der Nägel oder der Wimpern, insbesondere in der Form eines Maskarastücks, oder für die Augenbrauen und das Haar, insbesondere in der Form eines Stifts, aufweist.

41. Zusammensetzung gemäß einem der Ansprüche 1 bis 39, **dadurch gekennzeichnet, dass** es in der Form eines Stücks Maskara, eines Eyeliners, einer Grundlage, eines Lippenstifts, eines Rouge, eines Make-up-Entfernungsmittels, eines Make-up-Produkts für den Körper, eines Lidschattens oder eines Gesichtspuders oder eines Abdeckprodukts bereitgestellt wird.

42. Kosmetisches Pflege-, Make-up- oder Behandlungsverfahren für die Hornmaterialien eines Menschen, umfassend die Aufbringung einer kosmetischen Zusammensetzung gemäß einem der vorhergehenden Ansprüche auf die Hornmaterialien.

43. Kosmetisches Verfahren zum Einschränken der Migration einer kosmetischen Zusammensetzung, welche eine flüssige Fettphase enthält, die mindestens ein Silikonöl umfasst, welches besteht aus Strukturieren der Fettphase mit einer ausreichenden Menge von mindestens einem Polymer (Homopolymer oder Copolymer) mit einer gewichtsmittleren Molekularmasse im Bereich von 500 bis 500.000, enthaltend mindestens einen Anteil, welcher umfasst:
- mindestens einen Polyorganosiloxanrest, bestehend aus 1 bis 1.000 Organosiloxaneinheiten in der Kette des Anteils oder in der Form einer Pfropfung, und
- mindestens zwei Gruppen, welche zur Etablierung von Wasserstoffwechselwirkungen in der Lage sind, wobei die Gruppe Amid ist,
wobei das Polymer bei Raumtemperatur fest ist und in der flüssigen Fettphase bei einer Temperatur von 25 bis 250°C löslich ist,
wobei die flüssige Fettphase mindestens ein flüchtiges Silikonöl und mindestens ein flüchtiges Nichtsilikonöl umfasst und ferner ein nicht flüchtiges Nichtsilikonöl enthält und mindestens ein Organo-Geliermittel enthält, wobei das Organo-Geliermittel so wie in Anspruch 1, 30 oder 31 definiert ist.

## Revendications

1. Composition cosmétique de soin et/ou de maquillage comprenant une phase grasse liquide comprenant au moins une huile de silicone, structurée avec un système gélifiant comprenant :
1) au moins un polymère (homopolymère ou copolymère) ayant une masse moléculaire moyenne en poids allant de 500 à 500 000, qui contient au moins une fraction comprenant :
- au moins un groupe polyorganosiloxane constitué de 1 à 1 000 unités organosiloxane dans la chaîne de la structure ou sous la forme d'un greffon, et
- au moins deux groupes capables d'établir des interactions hydrogène, ledit groupe étant un amide,
le polymère étant solide à température ambiante et soluble dans la phase grasse liquide à une température de 25 à 250°C, et
2) au moins un agent organogélifiant non polymérique, la phase grasse liquide et le système gélifiant formant un milieu physiologiquement acceptable et la phase grasse liquide comprenant au moins une huile de silicone volatile et au moins une huile non-silicone volatile, et comprenant en outre une huile non-silicone non volatile,
dans lequel l'agent organogélifiant comprend au moins un composé capable de gélifier une huile de silicone, choisi parmi :
a) l'acide 12-hydroxystéarique, ses sels et ses dérivés ester ou amide,
b) des amides d'acides tricarboxyliques,
c) des esters et amides d'acides N-acylaminés,
d) des diurées d'acides N-acylaminés,
e) des uréthane-amides de dipeptides,
f) le dibenzylidènesorbitol et ses dérivés,
g) des dérivés de stérol,
h) des cyclodipeptides choisis parmi le cyclo(glycyl-L-alanyl), cyclo(glycyl-L-valyl), cyclo(glycyl-L-leucyl), cyclo(glycyl-L-phénylalanyl), cyclo(L-valyl-L-leucyl), cyclo(L-leucyl-L-leucyl), cylco(L-phénylalanyl-L-leucyl), cyclo(L-phénylalanyl-L-phénylalanyl), cyclo(L-valyl-L-γ-3,7-diméthyloctylglutamyl), cyclo(L-valyl-L-γ-2-éthylhexylglutamyl), cyclo(L-leucyl-L-γ-éthylglutamyl), cyclo(L-leucyl-L-γ-dodécylglutamyl), cyclo(L-leucyl-L-γ-3,7-diméthyloctylglutamyl), cyclo(L-leucyl-L-γ-benzylglutamyl), cyclo(L-β-butylasparaginyl-L-phénylalanyl), cyclo(L-γ-dodécylasparaginyl-L-phénylalanyl), cyclo(L-β-3,7-diméthyloctylasparaginyl-L-phénylalanyl), cyclo(L-β-2-éthylhexylasparaginyl-L-phénylalanyl), cyclo(L-β-3,5,5-triméthylhexylasparaginyl-L-phénylalanyl) et cyclo(L-β-2-éthylbutyl-asparaginyl-L-phénylalanyl),
i) un trans-(1R,2R)-bis(undécylcarbonylamino)cyclohexane de formule 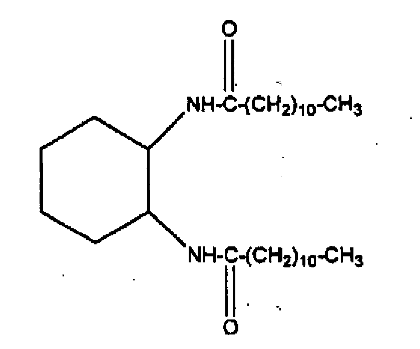
j) des éthers fluorés,
k) des agents organogélifiants de formule (XVII) :
Q-O-W-(CHOH)ₛ-W¹-O-Q¹ (XVII)
dans laquelle W et W¹, qui peuvent être identiques ou différents, sont choisis parmi -CH₂- et -CO-, et dans laquelle Q et Q¹, qui peuvent être identiques ou différents, sont une chaîne hydrocarbonée choisie parmi des chaînes hydrocarbonées saturées ou insaturées, linéaires ou ramifiées contenant au moins 6 atomes de carbone, et dans laquelle s est un nombre entier de 2 à 4 ;
l) des amides bolaamphiphiles dérivés des acides aminés de formules : 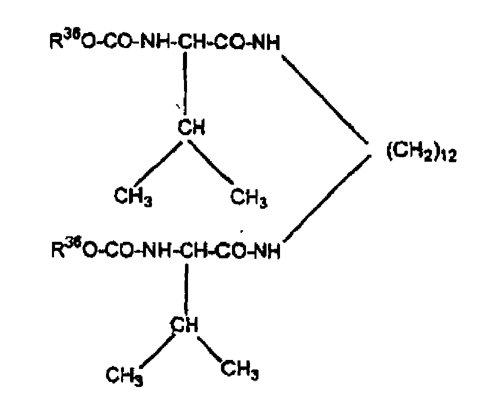 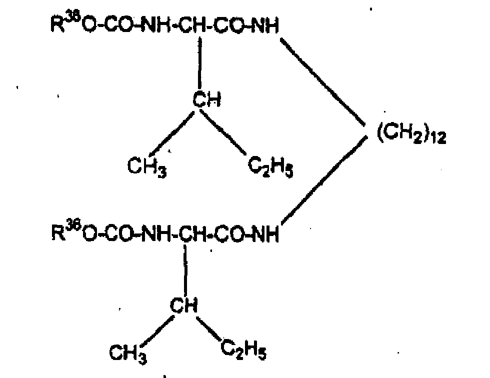 dans lesquelles R³⁶=**-**CH₂-C₆H₅ or -CH₂-CH₃, et 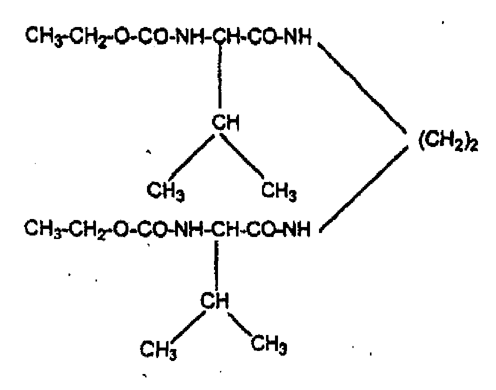
m) des sels de 2-alkyl--2-ammoniumisobutyl acétate p-toluènesulfonate de formule (XXII) : p-CH₃-C₆H₄-SO₃^{- +}H₃N-CH (R³⁷) -CO-OR³⁸ (XXII) dans laquelle R³⁷ représente :
**-CH₂-CH-(CH₃)₂ (leucine), -CH-(CH_{3)z} (L-valine),** 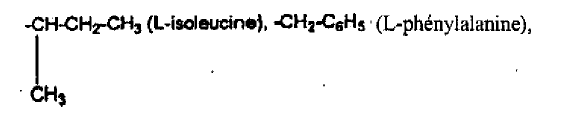 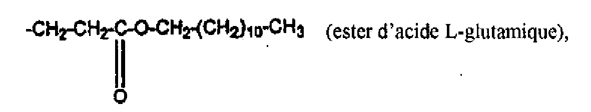
R³⁸ représente :
**-CH₂-(CH₂)ₙ-CH₃** avec n= 4 à 12, ou 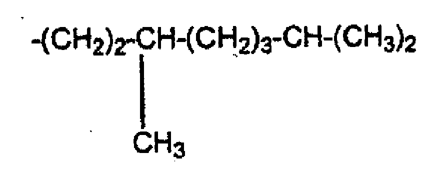
n) des dérivés diamide d'acide benzènedicarboxylique et valine de formules : 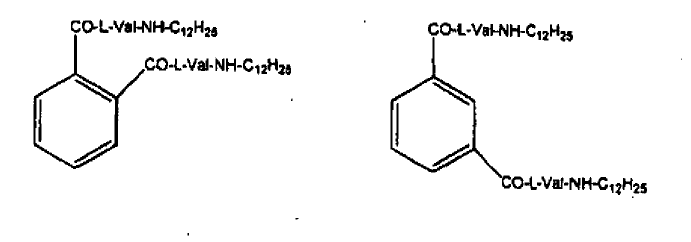 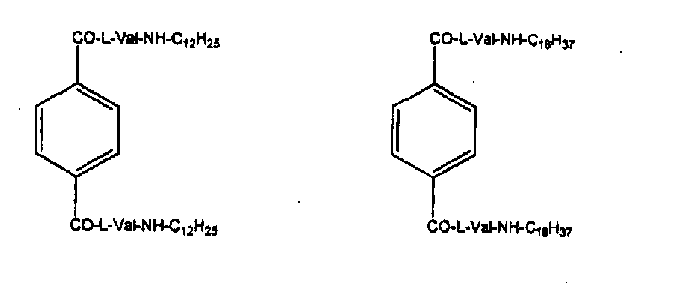 dans lesquelles -L-Val- représente :
-NH-CH [CH (CH₃) ₂] -CO- ;
o) des diamides de formule (XXV) ou (XXVI) :
R^{H} -X - CO- NH - R⁴⁵ - NH - CO - X -R⁴⁴ (XXV)
ou
R⁴⁴ - CO-NH-R^{4s}-NH-CO-R⁴⁴ (XXVI)
dans laquelle les groupes R⁴⁴,qui peuvent être identiques ou différents, représentent une chaîne hydrocarbonée en C₈-C₆₀ saturée ou insaturée, linéaire ou ramifiée, le(s) groupe(s) R⁴⁴ comprenant éventuellement un groupe hydroxyle ou au moins un hétéroatome tel que N, O, S ou Si, R⁴⁵ est un groupe à base hydrocarbonée choisi parmi des groupes linéaires, ramifiés et cycliques en C₁ à C₅₀ et des groupes arylène en C₅ à C₈ éventuellement substitués par un ou plusieurs groupes alkyle en C₁-C₄, et X représente -0- ou -NH- .

2. Composition selon la revendication 1, dans laquelle l'huile de silicone volatile a un point d'éclair égal ou supérieur à 40°C et avantageusement supérieur au point de ramollissement du système gélifiant.

3. Composition selon la revendication 1 ou 2, dans laquelle l'huile de silicone volatile est choisie dans le groupe constitué des composés suivants : octyltriméthicone, hexyltriméthicone, octaméthylcyclotétrasiloxane D4, dodécaméthylcyclotétrasiloxane D6, heptaméthyloctyltrisiloxane, décaméthyltétrasiloxane, dodécaméthylpentasiloxane, polydiméthylsiloxane de 1,5 cSt, polydiméthylsiloxane de 2cst, polydiméthylsiloxane de 3 cSt, polydiméthylsiloxane de 5 cSt, et des mélanges de ceux-ci.

4. Composition selon l'une quelconque des revendications précédentes, dans laquelle l'huile volatile a un point éclair de 40 à 135°C.

5. Composition selon l'une quelconque des revendications précédentes, dans laquelle la phase grasse liquide contient au moins 30 %, et même mieux au moins 40 % en poids d'huile de silicone.

6. Composition selon l'une quelconque des revendications précédentes, dans laquelle l'huile volatile représente de 3 à 89,4 % du poids total de la composition.

7. Composition selon l'une quelconque des revendications précédentes, comprenant, en outre, des particules solides choisies parmi des charges, des pigments et des mélanges de ceux-ci.

8. Composition selon la revendication 7, dans laquelle les particules solides sont des particules hydrophobes.

9. Composition selon la revendication précédente, dans laquelle les particules solides sont des particules hydrophiles, enrobées d'un film de composé hydrophobe.

10. Composition selon la revendication 7, dans laquelle les particules solides sont des particules hydrophiles et la composition comprend en outre un silicone amphiphile.

11. Composition selon l'une des revendications 7 à 10, dans laquelle les particules sont des pigments choisis parmi des oxydes de zinc, des oxydes de fer, des oxydes de titane et des mélanges de ceux-ci.

12. Composition selon l'une quelconque des revendications précédentes, dans laquelle le polymère utilisé dans le système gélifiant comprend au moins une structure correspondant à la formule : 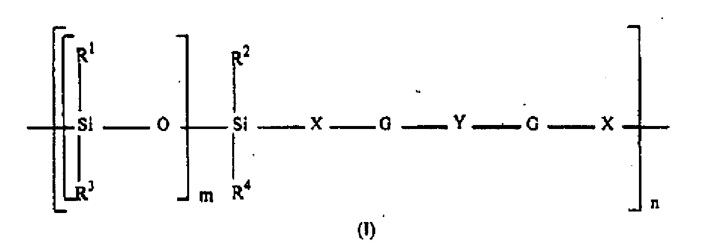 dans laquelle :
1) R¹, R², R³ et R⁴, qui peuvent être identiques ou différents, représentent un groupe choisi parmi :
- des groupes linéaires, ramifiés ou cycliques, saturés ou insaturés, à base hydrocarbonée en C₁ à C₁₀, contenant éventuellement dans leur chaîne un ou plusieurs atomes d'oxygène, de soufre et/ou d'azote, et étant possiblement partiellement ou totalement substitués par des atomes de fluor,
- des groupes aryle en C₆ à C₁₀, éventuellement substitués par un ou plusieurs groupes alkyle en C₁ à C₄,
- des chaînes polyorganosiloxane contenant possiblement un ou plusieurs atomes d'oxygène, de soufre et/ou d'azote ;
2) les groupes X, qui peuvent être identiques ou différents, représentent un groupe alkylènediyle en C₁ à C₃₀ linéaire ou ramifié, contenant possiblement dans sa chaîne un ou plusieurs atomes d'oxygène et/ou d'azote ;
3) Y est un groupe alkylène, arylène, cycloalkylène, alkylarylène ou arylalkylène divalent saturé ou insaturé, linéaire ou ramifié en C₁ à C₅₀, comprenant possiblement un ou plusieurs atomes d'oxygène, de soufre et/ou d'azote, et/ou portant comme substituant un des atomes ou groupes d'atomes suivants :
fluor, hydroxyle, cycloalkyle en C₃ à C₈, alkyle en C₁ à C₄₀, aryle en C₅ à C₁₀, phényle éventuellement substitué par 1 à 3 groupes alkyle en C₁ à C₃, hydroxyalkyle en C₁ à C₃ et aminoalkyle en C₁ à C₆, ou
4) Y représente un groupe correspondant à la formule : 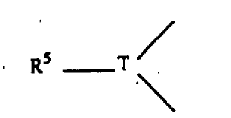 dans laquelle
- T représente un groupe à base hydrocarbonée trivalent ou tétravalent en C₃ à C₂₄, linéaire ou ramifié, saturé ou insaturé, éventuellement substitué par une chaîne polyorganosiloxane et contenant possiblement un ou plusieurs atomes choisis parmi O, N et S, ou bien T représente un atome trivalent choisi parmi N, P et Al, et
- R⁵ représente un groupe alkyle en C₁ à C₅₀ linéaire ou ramifié ou une chaîne de polyorganosiloxane, comprenant possiblement un ou plusieurs groupes ester, amide, uréthane, thiocarbamate, urée, thioruée et/ou sulfonamide, qui peuvent être liés à une autre chaîne du polymère ;
5) les groupes G, qui peuvent être identiques ou différents, représentent des groupes divalents de formule : 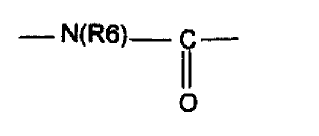 dans laquelle R⁶ représente un atome d'hydrogène ou un groupe alkyle en C₁ à C₂₀ linéaire ou ramifié, à condition qu'au moins 50 % des groupes R⁶ du polymère représente un atome d'hydrogène et qu'au moins deux des groupes G du polymère soient un groupe autre que : 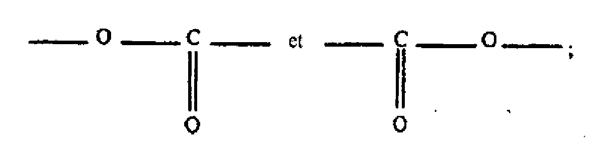
6) n est un nombre entier allant de 2 à 500 et de préférence de 2 à 200, et m est un nombre entier allant de 1 à 1 000, de préférence de 1 à 700 et mieux encore, de 6 à 200.

13. Composition selon la revendication précédente, dans laquelle Y représente un groupe choisi parmi :
a) des groupes alkylène linéaires en C₁ à C₂₀ et de préférence en C₁ à C₁₀,
b) des groupes alkylène ramifiés en C₃₀ à C₅₆ comprenant possiblement des cycles et des insaturations non conjuguées,
c) des groupes cycloalkylène en C₅-C₆,
d) des groupes phénylène éventuellement substitués par un ou plusieurs groupes alkyle en C₁ à C₄₀,
e) des groupes alkylène en C₁ à C₂₀ comprenant de 1 à 5 groupes amide,
f) des groupes alkylène en C₁ à C₂₀ comprenant un ou plusieurs substituants choisis parmi des groupes hydroxyle, cycloalkane en C₃ à C₈, hydroxyalkyle en C₁ à C₃ et alkylamine en C₁ à C₆,
g) des chaînes polyorganosiloxane de formule : 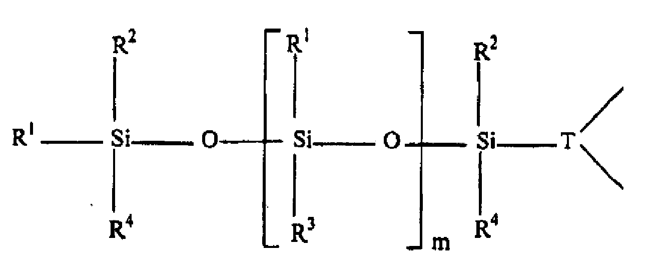 dans laquelle R¹, R², R³, R⁴, T et m sont tels que définis ci-dessus.

14. Composition selon l'une quelconque des revendications 1 à 11, dans laquelle le polymère utilisé dans le système gélifiant comprend au moins une fraction correspondant à la formule (II) : 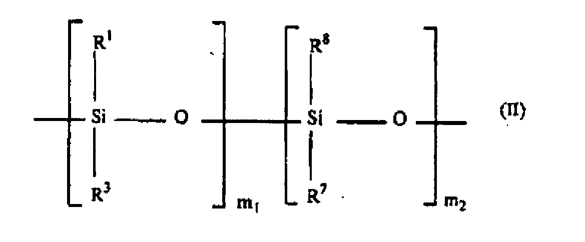 dans laquelle
- R¹ et R³, qui peuvent être identiques ou différents sont tels que définis ci-dessus pour la formule (I) dans la revendication 12,
- R⁷ représente un groupe tel que défini ci-dessus pour R¹ et R², ou représente le groupe de formule -X-G-R⁹ dans laquelle X et G sont tels que définis ci-dessus pour la formule (I) de la revendication 12 et R⁹ représente un atome d'hydrogène ou un groupe à base hydrocarbonée en C₁ à C₅₀ linéaire, ramifié ou cyclique, saturé ou insaturé, comprenant éventuellement dans sa chaîne un ou plusieurs atomes choisis parmi O, S et N, éventuellement substitués par un ou plusieurs atomes de fluor et/ou un ou plusieurs groupes hydroxyle, ou un groupe phényle éventuellement substitué par un ou plusieurs groupes alkyle en C₁ à C₄,
- R⁸ représente le groupe de formule -X-G-R⁹ dans laquelle X, G et R⁹ sont tels que définis ci-dessus,
- m₁ est un nombre entier allant de 1 à 998, et
- m₂ est un nombre entier allant de 2 à 500.

15. Composition selon la revendication 12, dans laquelle le polymère comprend au moins une fraction de formule (III) ou (IV) : 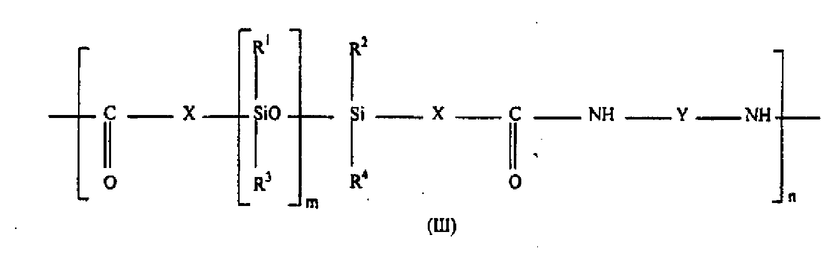 ou 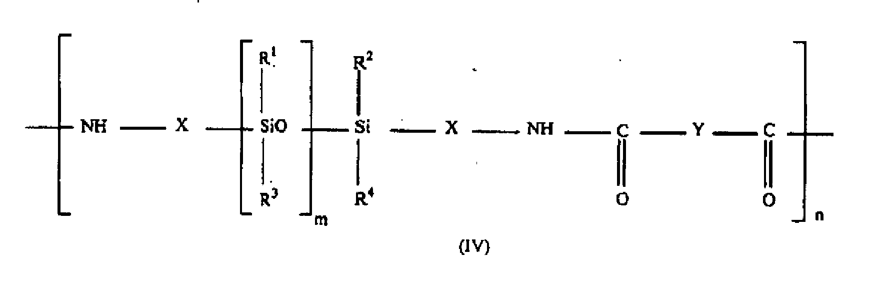 dans laquelle R¹, R², R³, R⁴, X, Y, m et n sont tels que définis à la revendication 12.

16. Composition selon l'une ou l'autre des revendications 12 et 15, dans laquelle X et/ou Y représentent un groupe alkylène contenant dans sa portion alkylène au moins un des éléments suivants :
1) 1 à 5 groupes amide, urée ou carbamate,
2) un groupe cycloalkyle en C₅ ou C₆, et
3) un groupe phénylène éventuellement substitué par 1 à 3 groupes alkyle en C₁ à C₃ identiques ou différents, la possibilité existant pour les groupes alkyle X ou Y d'être également substitués par au moins un élément choisi parmi le groupe constitué de :
- un groupe hydroxyle,
- un groupe cycloalkyle en C₃ à C₈,
- un à trois groupes alkyle en C₁ à C₄₀,
- un groupe phényle éventuellement substitué par un ou trois groupes alkyle en C₁ à C₃,
- un groupe hydroxyalkyle en C₁ à C₃, et
- un groupe aminoalkyle en C₁ à C₆.

17. Composition selon l'une quelconque des revendications 12 à 15, dans laquelle Y représente : 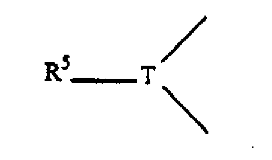 dans laquelle R⁵ représente une chaîne polyorganosiloxane et T représente un groupe de formule : 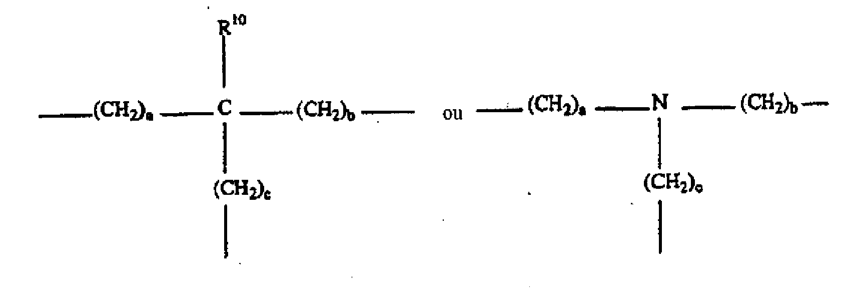 dans laquelle a, b et c sont, indépendamment, des nombres entiers allant de 1 à 10, et R¹⁰ est un atome d'hydrogène ou un groupe tel que ceux définis pour R¹, R², R³ et R⁴, à la revendication 12.

18. Composition selon l'une quelconque des revendications 12 à 15, dans laquelle R¹, R², R³ et R⁴ représentent, indépendamment, un groupe alkyle en C₁ à C₄₀ linéaire ou ramifié, de préférence un groupe CH₃, C₂H₅, n-C₃H₇ ou isopropyle, une chaîne polyorganosiloxane ou un groupe phényle éventuellement substitué par un à trois groupes méthyle ou éthyle.

19. Composition selon l'une quelconque des revendications 1 à 11, dans laquelle le polymère utilisé dans le système gélifiant comprend au moins une structure de formule : 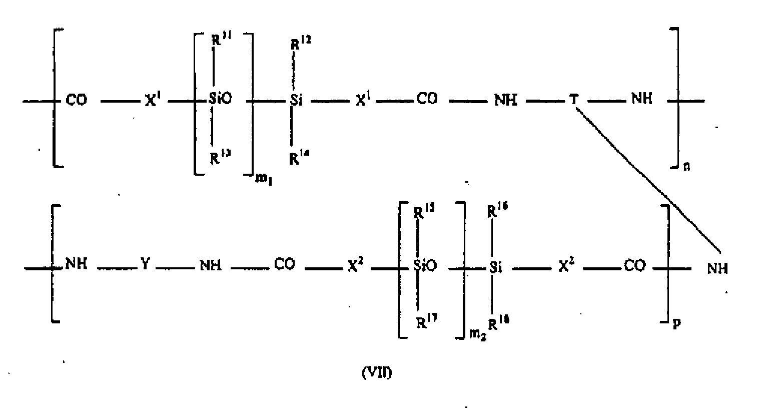 dans laquelle X¹ et X², qui sont identiques ou différents, ont la signification donnée pour X à la revendication 12, n, Y et T sont tels que définis à la revendication 12, R¹¹ à R¹⁸ sont des groupes choisis parmi le même groupe que R¹ à R⁴ dans la revendication 12, m₁ et m₂ sont des nombres allant de 1 à 1 000, et p est un nombre entier allant de 2 à 500.

20. Composition selon la revendication précédente, dans laquelle :
- p va de 1 à 25 et mieux encore de 1 à 7,
- R¹¹ à R¹⁸ sont des groupes méthyle,
- T correspond à une des formules suivantes : 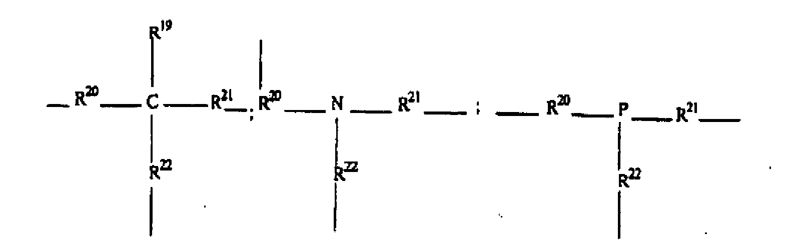 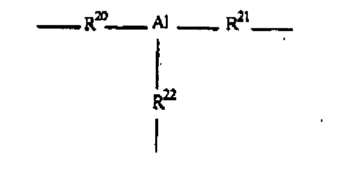 dans lesquelles R¹⁹ est un atome d'hydrogène ou un groupe choisi parmi les groupes définis pour R¹ à R⁴, et R²⁰, R²¹ et R²² sont, indépendamment, des groupes alkylène linéaires ou ramifiés, et plus avantageusement correspond à la formule : 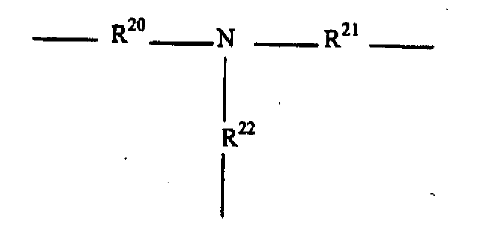 en particulier, avec R²⁰, R²¹ et R²² représentant -CH₂-CH₂-,
- m₁ et m₂ sont allant de 15 à 500 et mieux encore entre 15 et 45,
- X¹ et X² représentent - (CH₂)₁₀-, et
- Y représente -CH₂-.

21. Composition selon l'une quelconque des revendications 12 à 20, dans laquelle le polymère utilisé dans le système gélifiant comprend en outre une structure à base hydrocarbonée comprenant deux groupes capables d'établir des interactions hydrogène, choisies parmi des groupes ester, amide, sulfonamide, carbamate, thiocarbamate, urée, thiourée, oxamido, guanidino et biguanidino et des combinaisons de ceux-ci.

22. Composition selon la revendication précédente dans laquelle le copolymère est un copolymère à blocs ou un copolymère greffé.

23. Composition selon l'une quelconque des revendications précédentes, dans laquelle le polymère représente de 0,5 à 80 % du poids total de la composition, de préférence de 2 à 60 % et mieux encore de 5 à 40 % du poids total de la composition.

24. Composition selon l'une quelconque des revendications précédentes, dans laquelle la phase grasse liquide représente de 5 à 99 % du poids total de la composition et mieux encore de 20 à 75 % du poids total de la composition.

25. Composition selon l'une quelconque des revendications précédentes, dans laquelle ledit agent organogélifiant est choisi parmi des composés organiques non polymères dont les molécules sont capables d'établir, l'une avec l'autre, au moins une interaction physique entraînant une auto-agrégation desdites molécules avec formation d'un réseau macromoléculaire tridimensionnel.

26. Composition selon la revendication précédente, dans laquelle l'(les) interaction(s) physique(s) est (sont) choisie(s) parmi des interactions hydrogène auto-complémentaires, des interactions entre noyaux insaturés, des interactions dipolaires et des liens de coordination avec des dérivés organométalliques.

27. Composition selon l'une quelconque des revendications précédentes, dans laquelle l'agent organogélifiant est choisi parmi des composés dont les molécules comprennent au moins une entité choisie parmi au moins un groupe capable d'établir une liaison hydrogène, au moins un noyau aromatique, au moins une liaison comprenant une insaturation éthylénique et au moins un carbone asymétrique.

28. Composition selon l'une quelconque des revendications précédentes, dans laquelle l'agent organogélifiant est un composé dont les molécules comprennent au moins deux groupes capables d'établir une liaison hydrogène.

29. Composition selon la revendication précédente, dans laquelle le groupe capable d'établir une liaison hydrogène est choisi parmi les groupes hydroxyle, carbonyle, amine, acide carboxylique, amide, benzyle, sulfonamide, carbamate, thiocarbamate, urée, thiourée, oxamido, guanidino et biguanidino.

30. Composition selon la revendication 1, dans laquelle l'agent organogélifiant comprend au moins un composé capable de gélifier une huile de silicone et possédant au moins un groupe capable d'établir des interactions hydrogène avec le polymère du système gélifiant, choisi parmi :
a) des dérivés amide d'acide 12-hydroxystéarique,
b) des amides d'acides tricarboxyliques,
c) des esters et des amides d'acides N-acylaminés,
d) des diurées d'acides N-acylaminés,
e) des uréthanes-amides de dipeptides,
f) des cyclodipeptides choisis parmi le cyclo(glycyl-L-alanyl), cyclo(glycyl-L-valyl), cyclo(glycyl-L-leucyl), cyclo(glycyl-L-phénylalanyl), cyclo(L-valyl-L-leucyl), 1 cyclo(L-leucyl-L-leucyl), cyclo(L-phénylalanyl-L-leucyl), cyclo(L-phénylalanyl-L-phénylalanyl), cyclo(L-valyl-L-γ-3,7-diméthyloctylglutamyl), cyclo(L-valyl-L-γ-2-éthylhexylglutamyl), cyclo(L-leucyl-L-γ-éthylglytamyl), cyclo(L-leucyl-L-γ-dodêcylgiutamyl), cyclo(L-leucyl-L-γ-3,7-dimëthyloctylglutamyl), cyclo(L-leucyl-L-γ-benzylglutamyl), cyclo(L-β-butylasparaginyl-L-phénylalanyl), cyclo(L-γ-dodécylasparaginyl-L-phénylalanyl), cyclo(L-β-3,7-diméthyloctylasparaginyl-L-phénylalqnyl), cyclo(L-β-2-éthyl-hexylasparaginyl-L-phénylanyl), cyclo(L-β-3,5,5-triméthylhexylasparaginyl-L-phénylalanyl) et cyclo(L-β-2-éthylbutylasparaginyl-L-phénylalanyl),
g) un trans-(1R,2R)-bis(undécylcabonylamino)cyclohexane de formule : 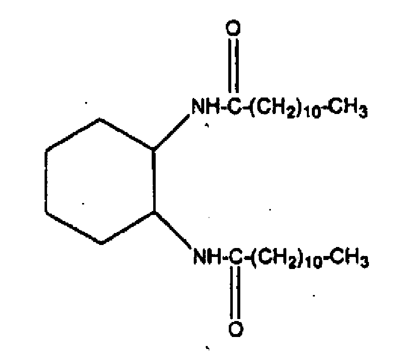
h) des amides bolaamphiphiles dérivés d'acides aminés de formules : 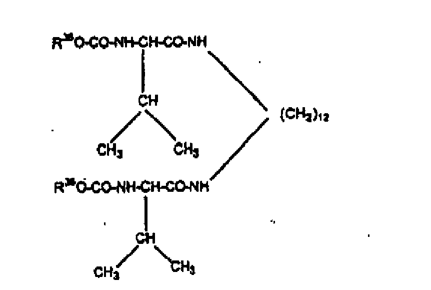 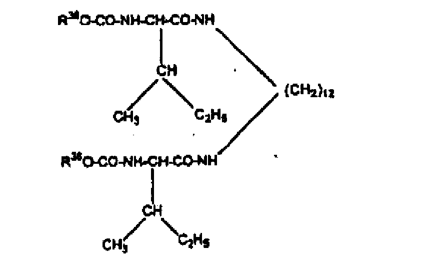 where R³⁶= -CH₂-C₆H₅ or -CH₂-CH₃, et 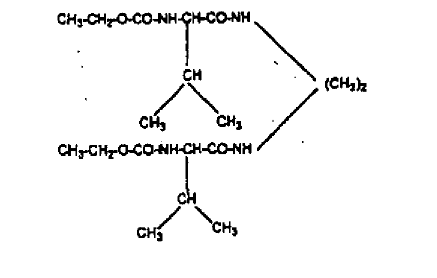
i) des dérivés diamide d'acide benzènedicarboxylique et de valine de formules : 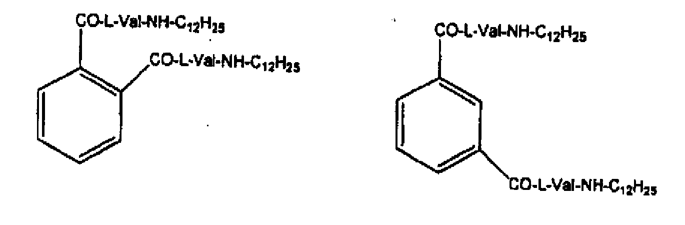 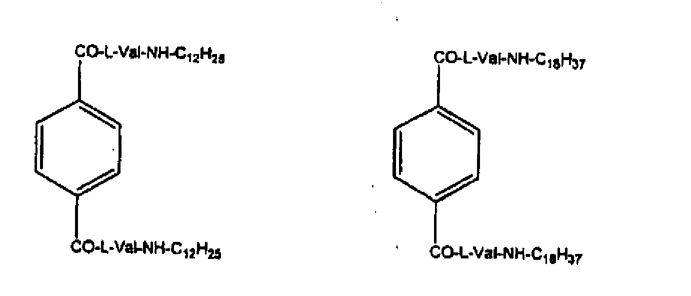 dans lesquelles -L-Val- représente :
-NH-CH[CH(CH₃)₂]-CO- ;
j) des diamides de formule (XXV) ou (XXVI) :
R⁴⁴-X-CO-NH-R⁴⁵-NH-CO-X-R⁴⁴ (XXV)
ou
R⁴⁴-CO-NH-R⁴⁵-NH-CO-R⁴⁴ (XXVI)
dans laquelle les groupes R⁴⁴, qui peuvent être identiques ou différents, représentent une chaîne hydrocarbonée en C₆-C₆₀ saturée ou insaturée, linéaire ou ramifiée, le(s) groupe(s) R⁴⁴ comprenant éventuellement un groupe hydroxyle ou au moins un hétéroatome tel que N, O, S ou Si, R⁴⁵ est un groupe à base hydrocarbonée choisi parmi des groupes en C₁, à C₅₀ linéaires, ramifiés et cycliques et des groupes arylène en C₅ à C₈ éventuellement substitués par un ou plusieurs groupes alkyle en C₁-C₄, et X représente -O- ou -NH- .

31. Composition selon l'une quelconque des revendications précédentes dans laquelle l'agent organogélifiant comprend au moins un composé choisi parmi :
- le mélange cis-trans de N,N'-bis(dodécanoyl)-1,2-diaminocyclohexane de formule : 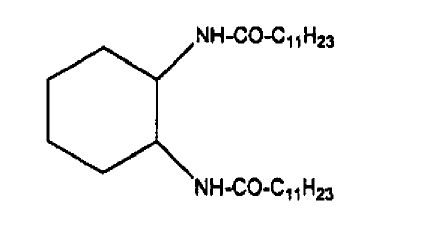
- le butylamide d'acide N-laurylglutamique.

32. Composition selon l'une quelconque des revendications précédentes dans laquelle ledit agent organogélifiant est présent en une quantité allant de 0,1 % à 80 % en poids par rapport au poids total de la composition, en particulier de 0,5 % à 60 %, de préférence de 1 à 40 % et mieux encore de 1 à 15 % en poids par rapport au poids total de la composition.

33. Composition selon l'une quelconque des revendications précédentes, dans laquelle le rapport massique de l'agent organogélifiant polymère/ non polymère de silicone va de 20 à 0,15, de préférence de 15 à 1,5.

34. Composition selon l'une quelconque des revendications précédentes, **caractérisée en ce qu'**elle comprend, en outre, au moins un principe actif cosmétique ou dermatologique.

35. Composition selon la revendication précédente, **caractérisée en ce que** le principe actif est choisi parmi des huiles essentielles, des vitamines, des humidifiants, des crèmes solaires, des agents cicatrisants et des céramides.

36. Composition selon l'une quelconque des revendications précédentes, **caractérisée en ce qu'**elle comprend au moins un additif choisi parmi des colorants qui sont solubles dans des polyols ou dans la phase grasse, des antioxydants, des huiles essentielles, des agents de conservation, des parfums, des polymères liposolubles, en particulier des polymères liposolubles à base hydrocarbonée, tels que des polyalkylènes ou du laurate de polyvinyle, des agents gélifiants de phase grasse liquide, des cires, des gommes, des résines, des tensioactifs, par exemple le phosphate de trioléyle, des principes actifs cosmétiques ou dermatologiques supplémentaires choisis, par exemple, parmi le groupe constitué de l'eau, des émollients, des humidifiants, des vitamines, de la lanoline liquide, des acides gras essentiels, des crèmes solaires lipophiles ou des crèmes solaires qui sont solubles dans des polyols, des vésicules lipidiques, et des mélanges de ceux-ci.

37. Composition selon l'une quelconque des revendications précédentes, **caractérisée en ce qu'**elle contient, en outre, un composé amphiphile qui est liquide à température ambiante, ayant une valeur HLB (équilibre hydrophile/lipophile) inférieure à 12.

38. Composition selon l'une quelconque des revendications précédentes, **caractérisée en ce qu'**elle comprend en outre une matière colorée.

39. Composition selon l'une quelconque des revendications précédentes, **caractérisée en ce qu'**elle est fournie sous la forme d'un gel rigide, et en particulier d'un stick anhydre transparent.

40. Composition selon l'une quelconque des revendications précédentes **caractérisée en ce qu'**elle a la forme d'un produit de maquillage coloré pour la peau, en particulier un fond de teint, éventuellement ayant des propriétés de soin ou de traitement, un blush, un fard à joues, un fard à paupières, un produit anticerne, un eye-liner ou un produit de maquillage du corps ; un produit de maquillage des lèvres, par exemple un stick de rouge à lèvres, présentant éventuellement des propriétés de soin ou de traitement ; un maquillage des phanères, par exemple les ongles ou les cils, en particulier sous la forme d'un mascara pain, ou les sourcils et les cheveux, en particulier sous la forme d'un crayon.

41. Composition selon l'une quelconque des revendications 1 à 39 **caractérisée en ce qu'**elle est fournie sous la forme d'un mascara pain, d'un eye-liner, d'un fond de teint, d'un stick de rouge à lèvres, d'un blush, d'un démaquillant, d'un produit de maquillage du corps, d'un fard à paupières ou d'un fard à joues, ou d'un produit anticerne.

42. Composition cosmétique de soin, maquillage ou méthode de traitement de matières kératiniques des êtres humains, comprenant l'application aux matières kératiniques d'une composition cosmétique selon l'une quelconque des revendications précédentes.

43. Méthode cosmétique pour limiter la migration d'une composition cosmétique contenant une phase grasse comprenant au moins une huile de silicone, consistant en la structuration de ladite phase grasse liquide avec une quantité suffisante d'au moins un polymère (homopolymère ou copolymère) ayant une masse moléculaire moyenne en poids allant de 500 à 500 000, qui contient au moins une fraction comprenant :
- au moins un groupe polyorganosiloxane, consistant en 1 à 1 000 unités organosiloxane dans la chaîne de la fraction ou sous la forme d'une greffe,
et
- au moins deux groupes capables d'établir des interactions hydrogène, ledit groupe étant un amide,
le polymère étant solide à température ambiante et soluble dans la phase grasse liquide à une température de 25 à 250°C,
la phase grasse liquide comprenant au moins une huile de silicone volatile et au moins une huile non silicone volatile et contenant en outre une huile non silicone non volatile et contenant au moins un agent organogélifiant, l'agent organogélifiant étant tel que défini à la revendication 1, 30 ou 31.
